(19) Europäisches Patentamt
European Patent Office
Office européen des brevets

(11) EP 2 061 767 B1

(12) **EUROPÄISCHE PATENTSCHRIFT**

(45) Veröffentlichungstag und Bekanntmachung des Hinweises auf die Patenterteilung:
**17.12.2014 Patentblatt 2014/51**

(21) Anmeldenummer: **07786319.9**

(22) Anmeldetag: **25.07.2007**

(51) Int Cl.:
***C07D 233/72*** *(2006.01)* ***A61K 31/4166*** *(2006.01)*
***A61K 31/4178*** *(2006.01)*

(86) Internationale Anmeldenummer:
**PCT/EP2007/006594**

(87) Internationale Veröffentlichungsnummer:
**WO 2008/017381 (14.02.2008 Gazette 2008/07)**

(54) **Arylaminoaryl-alkyl-substituierte Imidazolidin-2,4-dione, Verfahren zu ihrer Herstellung, diese Verbindungen enthaltende Arzneimittel und ihre Verwendung**

Arylaminoaryl-alkyl-substituted imidazolidine-2,4-diones, processes for preparing them, medicaments comprising these compounds, and their use

Imidazolidin-2,4-diones arylaminoaryl-alkyl-substituées, procédé de fabrication, médicaments les contenant et leur utilisation

(84) Benannte Vertragsstaaten:
**AT BE BG CH CY CZ DE DK EE ES FI FR GB GR HU IE IS IT LI LT LU LV MC MT NL PL PT RO SE SI SK TR**
Benannte Erstreckungsstaaten:
**AL BA HR MK RS**

(30) Priorität: **08.08.2006 DE 102006036930**
**30.08.2006 DE 102006040592**

(43) Veröffentlichungstag der Anmeldung:
**27.05.2009 Patentblatt 2009/22**

(73) Patentinhaber: **SANOFI**
**75008 Paris (FR)**

(72) Erfinder:
- **JAEHNE, Gerhard**
**65926 Frankfurt am Main (DE)**
- **STENGELIN, Siegfird**
**65926 Frankfurt Am Main (DE)**
- **GOSSEL, Matthias**
**65926 Frankfurt am Main (DE)**
- **KLABUNDE, Thomas**
**65926 Frankfurt am Main (DE)**
- **WINKLER, Irvin**
**65926 Frankfurt am Main (DE)**

(74) Vertreter: **Fischer, Hans-Jürgen**
**Sanofi-Aventis Deutschland GmbH**
**Patent- und Lizenzabteilung**
**Industriepark Höchst**
**Gebaüde K 801**
**65926 Frankfurt am Main (DE)**

(56) Entgegenhaltungen:
**WO-A-01/09090** **WO-A-2004/070050**
**WO-A-2005/049580** **WO-A-2007/083017**
**US-A- 5 411 981**

- **KING F D ED - KING F D (ED): "BIOISOSTERES, CONFORMATIONAL RESTRICTION, AND PRO-DRUGS - CASE HISTORY: AN EXAMPLE OF CONFORMATIONAL RESTRICTION APPROACH" MEDICINAL CHEMISTRY : PRINCIPLES AND PRACTICE, CAMBRIDGE, RSC, GB, 1994, Seiten 206-225, XP000995047**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 061 767 B1

## Beschreibung

Die Erfindung betrifft Imidazolidin-2,4-dione, die mit einem Arylaminoaryl-alkylrest substituiert sind sowie ihre physiologisch verträglichen Salze.

Es sind bereits strukturähnliche Imidazolin-2,4-dione beschrieben (siehe US 5,411,981) WO 2004/070050 A offenbart strukturähnliche, cyclische Harnstoffderivate, die als Medikamente und insbesondere als Kinaseinhibitoren geeignet sind.

WO2005/049580 A offenbart monocyclische N-Aryl Hydantoin Modulatoren des Androgenrezeptors.

WO2007/083017 A offenbart strukturähnliche, cyclische Harnstoffderivate, die als Medikamente und insbesondere als Kinaseinhibitoren geeignet sind.

Der Erfindung lag die Aufgabe zugrunde, Verbindungen zur Verfügung zu stellen, die eine therapeutisch verwertbare Wirkung entfalten. Insbesondere bestand die Aufgabe darin, neue Verbindungen zu finden, die zur Behandlung des metabolischen Syndroms, des Diabetes Typ II und der Adipositas geeignet sind.

Die Erfindung betrifft daher Verbindungen der Formel I,

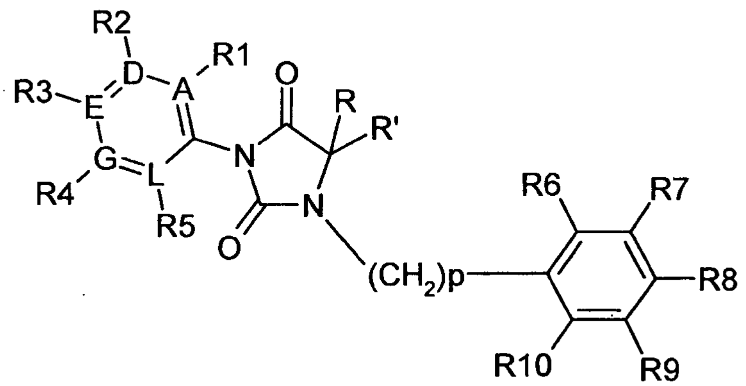

I

worin bedeuten

R, R' Methyl;

oder R und R' bilden gemeinsam einen Cyclohexylring;

| | |
|---|---|
| n | 0, 1, 2; |
| p | 1; |
| A, D, E, G, L | unabhängig voneinander C oder N, wobei bei der Bedeutung N der entsprechende Substituent R1, R2, R3, R4, R5 entfällt; |
| R1, R2, R5 | unabhängig voneinander H, F, Cl, Br, J, CN, $CF_3$, $(C_1-C_4)$-Alkyl, O-$(C_1-C_4)$-Alkyl, Phenyl, -O-Phenyl, $SF_5$, wobei die Alkylreste mit Fluoratomen substituiert sein können und wobei die Phenylreste mit F, Cl, Br, J substituiert sein können; |
| R3 | F, CN; |
| R4 | $CF_3$, $(C_1-C_4)$-Alkyl, O-$(C_1-C_4)$-Alkyl; |
| R6, R7, R8, R9, R10 | unabhängig voneinander H, F, Cl, Br, J, $(C_1-C_4)$-Alkyl, O-$(C_1-C_4)$-Alkyl, wobei die Alkylreste mit Fluoratomen substituiert sein können, NR17-Aryl, wobei der Arylrest substituiert sein kann mit F, Cl, Br, J, $(CH_2)_n$-CO-$NH_2$, $NH_2$, -$SO_2$- $NH_2$, COOH, $(CH_2)_n$-P(O)(OH)[O-$(C_1-C_4)$-Alkyl], $(CH_2)_n$-P(O)$(OH)_2$; |

wobei immer mindestens einer der Reste R6, R7, R8, R9 und R10 die Bedeutung NR17-Aryl besitzt;

R17 H, $(C_1-C_4)$-Alkyl;
sowie deren physiologisch verträgliche Salze.

Weiterhin ganz besonders bevorzugt sind Verbindungen der Formel I, worin R3 gleich F oder CN ist und R4

gleich $CF_3$ ist.

Weiterhin ganz besonders bevorzugt sind Verbindungen der Formel I, worin R3 gleich CN ist und R4 gleich $CF_3$ ist.

Weiterhin ganz besonders bevorzugt sind Verbindungen der Formel I, worin A, D, E, G und L gleich C sind.

Weiterhin ganz besonders bevorzugt sind Verbindungen der Formel I, worin p gleich 1 ist.

Können Reste oder Substituenten (wie z.B. R12) mehrfach in den Verbindungen der Formel I auftreten, so können sie alle unabhängig voneinander die angegebenen Bedeutungen haben und gleich oder verschieden sein.

Gegenstand der Erfindung sind weiterhin sowohl Stereoisomerengemische der Formel I als auch die reinen Stereoisomere der Formel I, sowie Diastereoisomerengemische der Formel I als auch die reinen Diastereoisomere. Die Trennung der Gemische erfolgt z. B. auf chromatographischem Weg.

Die Erfindung bezieht sich auf Verbindungen der Formel I, in Form ihrer Tautomere, Racemate, racemischen Mischungen, Stereoisomerengemische, reinen Stereoisomere, Diastereoisomerengemische, reinen Diastereoisomere. Die Trennung der Gemische erfolgt z. B. auf chromatographischem Weg.

Die Alkylreste in den Substituenten R1 bis R22 und R und R' können sowohl geradkettig wie verzweigt sein.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch- , Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate wie hierin beschrieben.

Unter einem Alkylrest wird eine geradkettige oder verzweigte Kohlenwasserstoffkette mit einem bis acht Kohlenstoffen verstanden, wie z.B. Methyl, Ethyl, iso-Propyl, tert.-Butyl, Hexyl, Heptyl, Octyl. Die Alkylreste können einfach oder mehrfach wie oben beschrieben substituiert sein.

Unter einem Cycloalkylrest wird ein einen oder mehre Ringe enthaltendes Ringssystem, welches gesättigt oder partiell ungesättigt (mit einer oder zwei Doppelbindungen) vorliegt, verstanden, das ausschließlich aus Kohlenstoffatomen aufgebaut ist, wie z.B. Cyclopropyl, Cyclopentyl, Cyclopentenyl, Cyclohexyl oder Adamantyl.

Die Cycloalkylreste können ein oder mehrfach mit geeigneten Gruppen wie oben beschrieben substituiert sein.

Unter einem Arylrest wird ein Phenyl, Naphthyl-, Biphenyl-, Tetrahydronaphthyl-, alpha- oder beta-Tetralon-, Indanyl- oder Indan-1-on-ylrest verstanden.

Die Arylreste können ein oder mehrfach mit geeigneten Gruppen wie oben beschrieben substituiert sein.

Unter Heteroarylrest werden aromatische Ringe und Ringsysteme verstanden, die außer Kohlenstoff noch Heteroatome, wie zum Beispiel Stickstoff, Sauerstoff oder Schwefel enthalten. Ferner gehören auch Ringsysteme zu dieser Definition, worin der Heteroarylrest mit Benzolkernen kondensiert ist. Ebenso fallen darunter Systeme, bei welchen eine oder mehrere CH-Gruppe(n) durch C=O oder C=S, vorzugsweise C=O, ersetzt ist (sind).

Geeignete Heteroarylreste sind z.B. Furyl, Imidazolyl, Benzimidazolyl, Indolyl, Indolinyl, Pyrimidinyl, Pyridyl, Pyrazinyl, Pyrrolyl, Thiazolyl, Oxazolyl, Thienyl, 1,2,3-Triazolyl, 1,2,4-Triazolyl, Tetrazolyl, Isoxazolyl, Pyridazinyl, 1,3,5-Triazinyl, 1,2,4-Triazinyl; das 2H-Pyridazin-3-on-, Dihydropyridazin-3,6-dion-, Imidazolidin-2-on-, 1,3-Dihydro-imidazol-2-on-, Imidazolidin-2,5-dion-, Chinolin-, Isochinolin-, Chinoxalin-, Chinazolin-System.

Die Verknüpfung mit den Heteroarylresten kann an jedem der dafür in Frage kommenden Atome erfolgen; so kann z. B. Pyridyl sowohl für 2-, 3- als auch 4-Pyridyl stehen; Thienyl sowohl für 2- als auch 3-Thienyl stehen; Furyl sowohl für 2- als auch 3-Furyl stehen.

Umfasst sind weiterhin die entsprechenden N-Oxide dieser Verbindungen, also z.B. 1-Oxy-2-, 3- oder 4-pyridyl.

Die Heteroarylreste können ein- oder mehrfach mit geeigneten Gruppen wie oben beschrieben substituiert sein.

Unter einem bicyclischen Heterocyclus werden aromatische und nicht aromatische bicyclische Ringsysteme mit 9 bis 12 Ringgliedern verstanden. Dabei können bis zu fünf CH- oder $CH_2$-Gruppen des Bicyclus unabhängig voneinander durch N, NR20, O, $S(O)_m$ oder C=O ersetzt sein.

Geeignete bicyclische Heterocyclen sind z. B. Benzimidazol, Benzotriazol, Indazol, Indol, 2,3-Dihydroindol,

1,3-Dihydro-benzimidazol-2-on, 3-Indazolinon, Oxindol, Isatin, Indolin-3-on1-Oxo-2-isoindolin, 2H-Benzo[1,2,4]thiadiazin-1,1-dioxid, 1,3-Dihydro-benzo[1,2,5]thiadiazol-2,2-dioxid, Benzo[1,3,2]dithiazol-1,1,3,3-tetraoxid, Saccharin, Benzothiazol, Benzoxazol.

Die Verknüpfung mit den bicyclischen Heterocyclen kann an jedem der dafür in Frage kommenden Atome erfolgen; so kann z. B. Benzimidazolyl sowohl für 2-, 4- als auch 5-Benzimidazolyl stehen; Indolyl sowohl für 2- als auch für 3-, 4-, 5-, 6- oder 7-Indolyl stehen.

Umfasst sind weiterhin die entsprechenden N-Oxide dieser Verbindungen.

Die bicyclischen Heterocyclen können ein- oder mehrfach mit geeigneten Gruppen wie oben beschrieben substituiert sein.

Unter Zucker werden Polyhydroxyverbindungen wie z.B. die D-Aldosen Erythrose, Threose (Tetrosen); Ribose, Arabinose, Xylose, Lyxose (Pentosen); Allose, Altrose, Glucose, Mannose, Gulose, Idose, Galactose oder Talose (Aldohexosen) jeweils in ihrer $\alpha$- oder $\beta$-Form, und wo möglich, in der Furanose- oder Pyranoseform verstanden; siehe Peter M. Collins, Robert J. Ferrier: Monosaccharides, John Wiley & Sons Ltd, Chichester, England; 1995, Seite 16 - 18 [ISBN 0 471 95342 3].

Weiterhin werden unter dem Begriff Zucker D-Ketosen (Ulosen) wie z. B. Erythrulose, Ribulose, Xylulose, Psicose, Fructose, Sorbose oder Tagatose jeweils in ihrer $\alpha$- oder $\beta$-Form, und wo möglich, in der Furanose- oder Pyranoseform verstanden; siehe Peter M. Collins, Robert J. Ferrier: Monosaccharides, John Wiley & Sons Ltd, Chichester, England; 1995, Seite 19 - 21 [ISBN 0 471 95342 3].

Ferner werden unter dem Begriff Zucker auch acyclische Polyhydroxyverbindungen wie z.B. Ethan-1,2-diol, Glycerin, 1,2,3,4-Tetrahydroxybutan (z.B. Threitol), 1,2,3,4,5-Pentahydroxypentan (z.B. Arabitol), 1,2,3,4,5,6-Hexahydroxyhexan (z.B. Sorbitol) oder D-Glucamin verstanden.

Die Bezeichnung $(CH_2)_n$-O-Zucker bedeutet, dass der Ether mit jeder der im Zuckerrest vorhandenen Hydroxyfunktion gebildet werden kann; erfolgt die Verknüpfung über die anomere Hydroxyfunktion des Zuckers liegt eine glycosidische Bindung vor.

Die Bezeichnung $(CH_2)_n$-O-Glucosid bedeutet, dass die Verknüpfung über die anomere Hydroxygruppe der Glucose erfolgt ist.

Die Bezeichnung $(CH_2)_n$-O-Galactosid bedeutet, dass die Verknüpfung über die anomere Hydroxygruppe der Galactose erfolgt ist.

Zuckersäure bezeichnet Aldonsäuren; als repräsentatives Beispiel sei die D-Gluconsäure genannt; diese Säuren können frei vorliegen oder zu Lactonen ringgeschlossen sein (siehe Peter M. Collins, Robert J. Ferrier: Monosaccharides, John Wiley & Sons Ltd, Chichester, England; 1995, Seite 126 - 129 [ISBN 0 471 95342 3]).

Der Begriff Zuckersäuren bezeichnet weiterhin Polyhydroxydicarbonsäuren welche von Zuckern abgeleitet sind; diese Säuren können u.U. auch ringgeschlossen als Lactone vorliegen. Beispiele solcher Aldarsäuren sind die D-Glucarsäure, die Galactarsäure (Schleimsäure) oder die Weinsäure (siehe Peter M. Collins, Robert J. Ferrier: Monosaccharides, John Wiley & Sons Ltd, Chichester, England; 1995, Seite 138 - 139 [ISBN 0 471 95342 3]).

Ferner werden vom Begriff Zuckersäuren solche Monosaccharidderivate umfasst, die eine Aldehydfunktion und eine Carbonsäurefunktion an den Enden der polyhydroxysubstituierten Kette enthalten (Uronsäuren). Als repräsentative Beispiele seien die D-Glucuronsäure, die D-Galacturonsäure und die D-Mannuronsäure genannt. Die Uronsäuren können ringoffen oder ringgeschlossen (als Lacton) vorliegen (siehe Peter M. Collins, Robert J. Ferrier: Monosaccharides, John Wiley & Sons Ltd, Chichester, England; 1995, Seite 313 - 314 [ISBN 0 471 95342 3]).

Unter den Begriff Zuckersäure fällt auch die L-Ascorbinsäure (Vitamin C).

Die Bezeichnung $(CH_2)_n$-O-Zuckersäure bedeutet, dass die Verknüpfung mit der Zuckersäure über eine Hydroxyfunktion als Ether oder glycosidisch über die anomere Hydroxyfunktion oder über die Hydroxyfunktion der Carbonsäure als Ester erfolgen kann.

Die Bezeichnung $(CH_2)_n$-O-Glucuronid bedeutet, dass die Verknüpfung mit der D-Glucuronsäure glycosidisch über die anomere Hydroxyfunktion der D-Glucuronsäure erfolgt.

Unter der Bezeichnung $(CH_2)_n$-O-Zuckersäure werden auch solche Pyranderivate verstanden, die eine Doppelbindung enthalten. Dazu gehören z.B. hydroxylierte 5,6-Dihydro-4H-pyran-2-carbonsäure oder ihre Ester. Ein Beispiel ist 4,5,6-Trihydroxy-5,6-dihydro-4H-pyran-2-carbonsäuremethylester.

Die Hydroxyfunktionen der Zucker und Zuckersäuren können unabhängig voneinander frei vorliegen, benzyliert, acyliert, besonders benzoyliert oder acetyliert, oder alkyliert, besonders methyliert, sein; ferner können zwei Hydroxygruppen mit Aceton zum Acetonid umgesetzt sein.

Die Erfindung umfasst auch Solvate oder Hydrate der Verbindungen der Formel I.

Die Verbindungen der Formel I stellen Cannabinoid 1 Rezeptor (CB1R) Modulatoren dar und sind als solche beim Menschen und bei Tieren zur Behandlung oder zur Verhütung von Krankheiten geeignet, die auf einer Störung des Endocannabinoid-systems beruhen.

Zum Beispiel, und nicht einschränkend, sind die Verbindungen der Formel I als psychotrope Medikamente nützlich, insbesondere zur Behandlung psychiatrischer Störungen, darunter Angstzustände, Depressionen, Gemütsstörungen,

Schlaflosigkeit, Delirien, Zwangsneurosen, generelle Psychosen, Schizophrenie, Defizit der Aufmerksamkeit und Hyperaktivität (ADHS) bei hyperkinetischen Kindern, sowie zur Behandlung von Störungen in Zusammenhang mit dem Gebrauch psychotroper Substanzen, insbesondere in dem Fall eines Missbrauchs einer Substanz und/oder einer Abhängigkeit von einer solchen Substanz, darunter Alkoholabhängigkeit und Nikotinabhängigkeit aber auch Abhängigkeit von Kokain, Methamphetamin und Heroin (siehe z.B. Behavioural Pharmacology 2005, 16:275-296). Übersichten über CB1R-vermittelte therapeutische Eingriffsmöglichkeiten finden sich z. B. in Ken Mackie: Annu. Rev. Pharmacol. Toxicol. 46, 101-122 (2006), S. C. Black: Curr. Opin. Investig. Drugs 5, 389-394 (2004), V. Di Marzio et al.: Nat. Rev. Drug Discov. 3, 771-784 (2004), B. Le Foll et al.: J. Pharmacol. Exp. Ther. 312, 875-883 (2005) oder L. Walter et al.: Br. J. Pharmacol. 141, 775-785 (2004).

Die erfindungsgemäßen Verbindungen der Formel I können als Medikamente zur Behandlung von Migräne, Stress, Krankheiten psychosomatischen Ursprungs, Panikattackenkrisen, Epilepsie, Bewegungsstörungen, insbesondere Dyskinesien oder Parkinsonsche Krankheit, Zittern und Dystonie verwendet werden.

Die erfindungsgemäßen Verbindungen der Formel I können weiterhin auch als Medikamente zur Behandlung von Gedächtnisstörungen, geistiger Defekte, insbesondere zur Behandlung der Altersdemenzen, der Alzheimer'schen Krankheit sowie zur Behandlung verminderter Aufmerksamkeit oder Wachsamkeit verwendet werden.

Ferner können die Verbindungen der Formel I als Neuroprotektoren, zur Behandlung von Ischämie, Schädelverletzungen und Behandlung neurodegenerativer Krankheiten, darunter Chorea, Chorea Huntington, Tourette-Syndrom, verwendet werden.

Die erfindungsgemäßen Verbindungen der Formel I können ferner als Medikamente bei der Schmerzbehandlung verwendet werden; dazu zählen neuropathische Schmerzen, akute periphere Schmerzen, chronische Schmerzen entzündlicher Herkunft.

Die erfindungsgemäßen Verbindungen der Formel I können weiterhin als Medikamente zur Behandlung von Essstörungen (z. B. zwanghafte Essanfälle (binge eating disorder), Anorexie und Bulimie), zur Behandlung der Sucht nach Süßigkeiten, Kohlenhydraten, Drogen, Alkohol oder anderen suchterzeugenden Substanzen dienen.

Die erfindungsgemäßen Verbindungen der Formel I sind besonders geeignet zur Behandlung der Adipositas oder der Bulimie sowie zur Behandlung von Diabetes Typ II wie auch zur Behandlung von Dyslipidämien und des metabolischen Syndroms. Die erfindungsgemäßen Verbindungen der Formel I sind daher zur Behandlung der Adipositas und der Gefahren in Zusammenhang mit Adipositas, insbesondere der kardiovaskulären Gefahren, nützlich. Ferner können die erfindungsgemäßen Verbindungen der Formel I als Medikamente zur Behandlung gastrointestinaler Störungen, zur Behandlung von Durchfällen, von Magen-Darmgeschwüren, von Erbrechen, von Blasenleiden und Störungen des Wasserlassens, von Störungen endokrinen Ursprungs, von kardiovaskulären Problemen, von niedrigem Blutdruck, des hämorrhagischen Schocks, des septischen Schocks, chronischer Leberzirrhose, Lebersteatose, der nicht alkoholischen Steatohepatitis, von Asthma, des Raynaudschen Syndroms, des Glaukoms, von Fruchtbarkeitsbeschwerden, Schwangerschaftsunterbrechung, Frühgeburt, Entzündungserscheinungen, Krankheiten des Immunsystems, insbesondere autoimmun- und neuroinflammatorische, wie zum Beispiel rheumatische Gelenkentzündung, reaktive Arthritis, von Krankheiten, die zu Demyelinisation führen, der multiplen Sklerose, von Infektionskrankheiten und viralen Erkrankungen, wie zum Beispiel von Enzephalitis, ischämischem Schlaganfall sowie als Medikamente zur Krebschemotherapie, zur Behandlung des Guillain-Barré-Syndroms und zur Behandlung der Osteoporose verwendet werden.

Die erfindungsgemäßen Verbindungen der Formel I können weiterhin auch als Medikamente zur Behandlung des Syndroms der polycystischen Ovarien (PCOS, polycystic ovary syndrome) Verwendung finden.

Gemäß der vorliegenden Erfindung sind die Verbindungen der Formel I besonders nützlich zur Behandlung psychotischer Beschwerden, insbesondere der Schizophrenie, verminderter Aufmerksamkeit und Hyperaktivität (ADHS) bei hyperkinetischen Kindern, zur Behandlung von Essstörungen und der Adipositas, zur Behandlung des Diabetes Typ II, zur Behandlung von Gedächtnisdefiziten und kognitiven Defiziten, zur Behandlung der Alkoholsucht, der Nikotinsucht, das heißt für die Alkohol- und Tabakentwöhnung.

Ganz besonders nützlich sind die erfindungsgemäßen Verbindungen der Formel I zur Behandlung und Verhütung von Essstörungen Appetitstörungen, metabolischen Störungen, gastrointestinalen Störungen, Entzündungserscheinungen, Erkrankungen des Immunsystems, psychotischen Störungen, der Alkoholsucht und der Nikotinsucht.

Gemäß einem ihrer Aspekte bezieht sich die Erfindung auf den Gebrauch einer Verbindung der Formel I, ihrer pharmazeutisch akzeptablen Salze und deren Solvate oder Hydrate zur Behandlung der oben angegebenen Störungen und Erkrankungen.

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für

diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel I selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwähnt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der

Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1% bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder Iontophorese freigesetzt werden.

Als weitere Wirkstoffe für die Kombinationspräparate sind geeignet:

Alle Antidiabetika, die in der Roten Liste 2005, Kapitel 12 genannt sind; alle Abmagerungsmittel/Appetitzügler, die in der Roten Liste 2005, Kapitel 1 genannt sind; alle Lipidsenker, die in der Roten Liste 2005, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus® (siehe www.lantus.com) oder HMR 1964 oder Levemir® (insulin detemir) oder solche, wie sie in WO2005005477 (Novo Nordisk) beschrieben sind, schnell wirkende Insuline (siehe US 6,221,633), inhalierbare Insuline, wie z. B. Exubera® oder orale Insuline, wie z. B. IN-105 (Nobex) oder Oral-lyn™ (Generex Biotechnology), GLP-1-Derivate und GLP-1 Agonisten wie z.B. Exenatide, Liraglutide oder diejenigen die in WO 98/08871, WO2005027978, WO2006037811, WO2006037810 von Novo Nordisk A/S, in WO 01/04156 von Zealand oder in WO 00/34331 von Beaufour-Ipsen offenbart wurden, Pramlintide Acetat (Symlin; Amylin Pharmaceuticals), BIM-51077, PC-DAC:Exendin-4 (ein Exendin-4 Analogon, welches kovalent an rekombinantes menschliches Albumin gebunden ist), Agonisten wie sie z.B. bei D. Chen et al., Proc. Natl. Acad. Sci. USA 104 (2007) 943 beschrieben sind, solche wie sie in WO2006124529 beschrieben sind, sowie oral wirksame hypoglykämische Wirkstoffe.

Antidiabetika umfassen auch Agonisten des Glukose-abhängigen insulinotropen Polypeptids (GIP) Rezeptors wie sie z.B. in WO2006121860 beschrieben sind.

Die oral wirksamen hypoglykämischen Wirkstoffe umfassen vorzugsweise Sulfonylharnstoffe,

Biguanidine,
Meglitinide,
Oxadiazolidindione,
Thiazolidindione,
Glukosidase-Inhibitoren,
Hemmstoffe der Glykogenphosphorylase,
Glukagon-Antagonisten,
Glukokinaseaktivatoren,
Inhibitoren der Fructose-1,6-bisphosphatase
Modulatoren des Glukosetransporters-4 (GLUT4),
Inhibitoren der Glutamin-Fructose-6-Phosphat-Amidotransferase (GFAT), GLP-1-Agonisten,
Kaliumkanalöffner, wie z.B. Pinacidil, Cromakalim, Diazoxid oder solche wie sie bei R. D. Carr et al., Diabetes 52, 2003, 2513.2518, bei J. B. Hansen et al, Current Medicinal Chemistry 11, 2004, 1595-1615, bei T. M. Tagmose et al., J. Med. Chem. 47, 2004, 3202-3211 oder bei M. J. Coghlan et al., J. Med. Chem. 44, 2001, 1627-1653 beschrieben sind, oder diejenigen, die in WO 97/26265 und WO 99/03861 von Novo Nordisk A/S offenbart wurden, Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption, Hemmstoffe der 11ß-HSD1,
Inhibitoren der Protein-Tyrosin-Phosphatase-1B (PTP1B),
Modulatoren des natrium-abhängigen Glukosetransporters 1 oder 2 (SGLT1, SGLT2),
den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindungen der Formel I in Kombination mit einem HMG-

CoA-Reduktase Inhibitor wie Simvastatin, Fluvastatin, Pravastatin, Lovastatin, Atorvastatin, Cerivastatin, Rosuvastatin, L-659699 verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, WO2005042692, WO2005005453), MD-0727 (Microbia Inc., WO2005021497, WO2005021495) oder mit Verbindungen, wie in WO2002066464, WO2005000353 (Kotobuki Pharmaceutical Co. Ltd.) oder WO2005044256 oder WO2005062824 (Merck & Co.) oder WO2005061451 und WO2005061452 (AstraZeneca AB) und WO2006017257 (Phenomix) oder WO2005033100 (Lipideon Biotechnology AG) oder wie in WO2004097655, WO2004000805, WO2004000804, WO2004000803, WO2002050068, WO2002050060, WO2005047248, WO2006086562, WO2006102674, WO2006116499, WO2006121861, WO2006122186, WO2006122216, WO2006127893, WO2006137794, WO2006137796, WO2006137782, WO2006137793, WO2006137797, WO2006137795, WO2006137792, WO2006138163 beschrieben, verabreicht.

Bei einer Ausführungsform der Erfindung wird die Verbindung der Formel I in Kombination mit Vytorin™, einer festen Kombination von Ezetimibe mit Simvastatin, verabreicht.

Bei einer Ausführungsform der Erfindung wird die Verbindung der Formel I in Kombination mit einer festen Kombination von Ezetimibe mit Atorvastatin, verabreicht.

Bei einer Ausführungsform der Erfindung wird die Verbindung der Formel I in Kombination mit einer festen Kombination von Ezetimibe mit Fenofibrat verabreicht.

Bei einer weiteren Ausführungsform der Erfindung wird die Verbindung der Formel I in Kombination mit einer festen Kombination von Fenofibrat mit Rosuvastatin verabreicht.

Bei einer weiteren Ausführungsform der Erfindung wird die Verbindung der Formel I in Kombination mit Synordia (R), einer festen Kombination von Fenofibrat mit Metformin, verabreicht.

Bei einer Ausführungsform der Erfindung wird die Verbindung der Formel I in Kombination mit ISIS-301012, einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, GI 262570, R-483, CS-011 (Rivoglitazon) verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.

Bei einer Ausführungsform der Erfindung wird die Verbindung der Formel I in Kombination mit Tandemact™, einer festen Kombination von Pioglitazon mit Glimeprid, verabreicht.

Bei einer weiteren Ausführungsform der Erfindung wird die Verbindung der Formel I in Kombination mit einer festen Kombination von Pioglitazon Hydrochlorid mit einem Angiotensin II Agonisten, wie z.B. TAK-536, verabreicht.

Bei einer Ausführungsform der Erfindung wird die Verbindung der Formel I in Kombination mit einem PPAR alpha Agonisten, wie z.B. GW9578, GW-590735, K-111, LY-674, KRP-101, DRF-10945, LY-518674 oder solchen wie sie in WO2001040207, WO2002096894, WO2005097076 beschrieben sind, verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, CKD-501 (Lobeglitazon Sulfat) oder wie in PCT/US 00/11833, PCT/US 00/11490, DE10142734.4 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 oder wie sie in WO2006059744, WO2006084176, WO2006029699, WO2007039172-WO2007039178 beschrieben sind, verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit einem MTP-Inhibitor, wie z.B. Implitapide , BMS-201038, R-103757, AS-1552133 oder solchen wie in WO2005085226, WO2005121091, WO2006010423 beschrieben, verabreicht.

Bei einer Ausführungsform der Erfindung wird die Verbindung der Formel I in Kombination mit einem CETP-Inhibitor, wie z.B. Torcetrapib oder JTT-705 oder solchen wie sie in WO2006002342, WO2006010422, WO2006012093, WO2006073973, WO2006072362, WO2006097169, WO2007041494 beschrieben sind, verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9, WO2007009655-56 beschrieben, verabreicht.

Bei einer Ausführungsform der Erfindung wird die Verbindung der Formel I in Kombination mit einem polymeren Gallensäureadsorber, wie z.B. Cholestyramin, Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in WO2005097738 beschrieben, verabreicht.

Bei einer Ausführungsform der Erfindung wird die Verbindung der Formel I in Kombination mit einem ABCA1 Expressionsverstäker, wie sie z.B. in WO2006072393 beschrieben, verabreicht.

Bei einer weiteren Ausführungsform der Erfindung wird die Verbindung der Formel I in Kombination mit einem RNAi Therapeutikum, welches gegen PCSK9 (Proprotein Convertase Subtilisin/Kexin Typ 9) gerichtet ist, verabreicht.

Bei einer Ausführungsform wird die Verbindung der Formel I in Kombination mit Omacor® (Omega-3-Fettsäuren; hochkonzentrierte Ethylester der Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe oder SMP-797, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, β-Caroten oder Selen verabreicht.

Bei einer Ausführungsform der Erfindung wird die Verbindung der Formel I in Kombination mit einem Vitamin, wie z. B. Vitamin B6 oder Vitamin B12 verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform der Erfindung wird die Verbindung der Formel I in Kombination mit einem Squalen Synthetase Inhibitor, wie z.B. BMS-188494, TAK-475 oder wie in WO2005077907, JP2007022943 beschrieben, verabreicht.

Bei einer Ausführungsform der Erfindung wird die Verbindung der Formel I in Kombination mit einem Lipoprotein(a) antagonist, wie z.B. Gemcabene (CI-1027) verabreicht.

Bei einer Ausführungsform der Erfindung wird die Verbindung der Formel I in Kombination mit einem Aqonisten des GPR109A (HM74A Rezeptor Agonisten; NAR-Agonisten (Nikotinsäurerezeptoragonisten)), wie z.B. Nicotinsäure oder "extended release niacin" in Verbindung mit MK-0524A oder solchen Verbindungen, wie sie in WO2006045565, WO2006045564, WO2006069242, WO2006124490, WO2006113150, WO2007017261, WO2007017262, WO2007017265, WO2007015744, WO2007027532 beschrieben sind, verabreicht.

Bei einer anderen Ausführungsform der Erfindung wird die Verbindung der Formel I in Kombination mit einem Aqonisten des GPR116, wie sie z.B. in WO2006067531, WO2006067532 beschrieben sind, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform wird die Verbindung der Formel I in Kombination mit einem Sulfonylharnstoff, wie z.B. Tolbutamid, Glibenclamid, Glipizid, Gliclazide oder Glimepirid verabreicht.

Bei einer Ausführungsform wird die Verbindung der Formel I in Kombination mit einer die Insulinsekretion verstärkende Substanz, wie z. B. KCP-265 (WO2003097064), oder solchen wie sie in WO2007026761 beschrieben sind, verabreicht.

Bei einer Ausführungsform wird die Verbindung der Formel I in Kombination mit Agonisten des glucose-abhängigen insulinotropischen Rezeptors (GDIR) wie z. B. APD-668 verabreicht

Bei einer Ausführungsform wird die Verbindung der Formel I in Kombination mit einem Biguanid, wie z.B. Metformin, verabreicht.

Bei wieder einer Ausführungsform wird die Verbindung der Formel I in Kombination mit einem Meglitinid, wie z.B. Repaglinide, Nateglinid oder Mitiglinide verabreicht.

Bei einer weiteren Ausführungsform wird die Verbindung der Formel I mit einer Kombination von Mitiglinide mit einem Glitazon, z.B. Pioglitazon Hydrochlorid, verabreicht.

Bei einer weiteren Ausführungsform wird die Verbindung der Formel I mit einer Kombination von Mitiglinide mit einem alpha-Glukosidaseinhibitor verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]phenyl]methyl]-2,4-thiazolidindion, verabreicht.

Bei einer Ausführungsform wird die Verbindung der Formel I in Kombination mit einem α-Glukosidase-Inhibitor, wie z.B. Miglitol oder Acarbose, verabreicht.

Bei einer Ausführungsform wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform wird die Verbindung der Formel I in Kombination mit mehr als einer der vorstehend

genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer Ausführungsform wird die Verbindung der Formel I in Kombination mit einem Hemmstoff der Glykogenphosphorylase, wie z.B. PSN-357 oder FR-258900 oder solchen wie in WO2003084922, WO2004007455, WO2005073229-31, WO2005067932 beschrieben, verabreicht.

Bei einer Ausführungsform wird die Verbindung der Formel I in Kombination mit Glukagon-Rezeptor-Antagonisten, wie z.B. A-770077 oder NNC-25-2504 oder wie in WO2004100875, WO2005065680 beschrieben, verabreicht.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in WO2004072031, WO2004072066, WO2005080360, WO2005044801, WO2006016194, WO2006058923, WO2006112549, WO2006125972, WO2007017549, WO2007017649, WO2007007910, WO2007007040-42, WO2007006760-61, WO2007006814, WO2007007886, WO2007028135, WO2007031739, WO2007041365, WO2007041366, WO2007037534, WO2007043638, WO2007053345, WO2007051846, WO2007051845, WO2007053765, WO2007051847 beschrieben sind, verabreicht.

Bei einer Ausführungsform wird die Verbindung der Formel I in Kombination mit einem Inhibitor der Glukoneogenese, wie z. B. FR-225654, verabreicht.

Bei einer Ausführungsform wird die Verbindung der Formel I in Kombination mit Inhibitoren der Fructose-1,6-bisphosphatase (FBPase) wie z.B. CS-917 (MB-06322) oder MB-07803 oder solchen wie sie in WO2006023515, WO2006104030, WO2007014619 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Sitagliptin Phosphat, Saxagliptin ((BMS-477118), GSK-823093, PSN-9301, SYR-322, SYR-619, TA-6666, TS-021, GRC-8200, GW-825964X, KRP-104, DP-893, ABT-341, ABT-279 oder ein anderes Salz davon oder solchen Verbindungen wie sie in WO2003074500, WO2003106456, WO2004037169, WO200450658, WO2005058901, WO2005012312, WO2005/012308, WO2006039325, WO2006058064, PCT/EP2005/007821, PCT/EP2005/008005, PCT/EP2005/008002, PCT/EP2005/008004, PCT/EP2005/008283, DE 10 2005 012874.2, DE 10 2005 012873.4, JP2006160733, WO2006071752, WO2006065826, WO2006078676, WO2006073167, WO2006068163, WO2006090915, WO2006104356, WO2006127530, WO2006111261, WO2007015767, WO2007024993, WO2007029086 beschrieben sind, verabreicht.

Bei einer Ausführungsform wird die Verbindung der Formel I in Kombination mit Janumet™, einer festen Kombination von Sitagliptin Phosphat mit Metformin Hydrochlorid, verabreicht.

Bei einer Ausführungsform wird die Verbindung der Formel I in Kombination mit Hemmstoffen der 11-beta-Hydroxysteroid-Dehydrogenase-1 (11ß-HSD1), wie z. B. BVT-2733, JNJ-25918646, INCB-13739 oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109, WO2006074244, WO2006078006, WO2006106423, WO2006132436, WO2006134481, WO2006134467, WO2006135795, WO2006136502, WO2006138695, WO2006133926, WO2007003521, WO2007007688, US2007066584, WO2007047625, WO2007051811, WO2007051810 beschrieben sind, verabreicht.

Bei einer Ausführungsform wird die Verbindung der Formel I in Kombination mit Inhibitoren der Protein-Tyrosin-Phosphatase-1B (PTP1B), wie sie z. B. in WO200119830-31, WO200117516, WO2004506446, WO2005012295, WO2005116003, PCT/EP2005/005311, PCT/EP2005/005321, PCT/EP2005/007151, DE 10 2004 060542.4, WO2007009911, WO2007028145 beschrieben sind, verabreicht.

Bei einer Ausführungsform wird die Verbindung der Formel I in Kombination mit Modulatoren des natriumabhängigen Glukosetransporters 1 oder 2 (SGLT1, SGLT2), wie z.B. KGA-2727, T-1095, SGL-0010, AVE 2268, SAR 7226 und Sergliflozin oder wie sie z. B. in WO2004007517, WO200452903, WO200452902, PCT/EP2005/005959, WO2005085237, JP2004359630, WO2005121161, WO2006018150, WO2006035796, WO2006062224, WO2006058597, WO2006073197, WO2006080577, WO2006087997, WO2006108842, WO2007000445, WO2007014895 oder von A. L. Handlon in Expert Opin. Ther. Patents (2005) 15(11), 1531-1540 beschrieben sind,

verabreicht.

Bei einer Ausführungsform wird die Verbindung der Formel I in Kombination mit Modulatoren des GPR40, wie sie z.B. in WO2007013689, WO2007033002 beschrieben sind, verabreicht.

Bei einer Ausführungsform wird die Verbindung der Formel I in Kombination mit Modulatoren des GPR119b, wie sie z. B. in WO2004041274, beschrieben sind, verabreicht. Bei einer Ausführungsform wird die Verbindung der Formel I in Kombination mit Modulatoren des GPR119, wie sie z. B. in WO2005061489 (PSN-632408), WO2004065380, WO2007003960-62 und WO2007003964 beschrieben sind, verabreicht.

Bei einer weiteren Ausführungsform wird die Verbindung der Formel I in Kombination mit Modulatoren des GPR120 verabreicht.

Bei einer Ausführungsform wird die Verbindung der Formel I in Kombination mit Inhibitoren der hormon-sensitiven Lipase (HSL) und/oder Phospholipasen, wie z. B. in WO2005073199, WO2006074957, WO2006087309, WO2006111321, WO2007042178 beschrieben, verabreicht.

Bei einer Ausführungsform wird die Verbindung der Formel I in Kombination mit Hemmstoffen der Acetyl-CoA Carboxylase (ACC) wie z. B. solchen wie in WO 199946262, WO200372197, WO2003072197, WO2005044814, WO2005108370, JP2006131559, WO2007011809, WO2007011811 WO2007013691 beschrieben, verabreicht.

Bei einer weiteren Ausführungsform wird die Verbindung der Formel I in Kombination mit Modulatoren der Xanthin-Oxidoreductase (XOR) verabreicht.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, PCT/EP2005/005346, WO2003078403, WO2004022544, WO2003106410, WO2005058908, US2005038023, WO2005009997, US2005026984, WO2005000836, WO2004106343, EP1460075, WO2004014910, WO2003076442, WO2005087727, WO2004046117 beschrieben.

Bei einer Ausführungsform wird die Verbindung der Formel I in Kombination mit einem Inhibitor der Serum/Glucocorticoid regulierten Kinase (SGK), wie z. B. in WO2006072354 beschrieben, verabreicht.

Bei einer Ausführungsform wird die Verbindung der Formel I in Kombination mit einem Agonisten des RUP3 Rezeptors, wie z. B. in WO2007035355 beschrieben, verabreicht.

Bei einer Ausführungsform wird die Verbindung der Formel I in Kombination mit einem Inhibitor der Protein Kinase C beta (PKC beta), wie z. B. Ruboxistaurin, verabreicht.

Bei einer anderen Ausführungsform wird die Verbindung der Formel I in Kombination mit einem Aktivator des Gens, welches für die Ataxia Telangiectasia Mutated (ATM) Proteinkinase kodiert, wie z. B. Chloroquin, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Inhibitoren der "I-kappaB kinase" (IKK Inhibitoren), wie sie z. B. in WO2001000610, WO2001030774, WO2004022553, WO2005097129 beschrieben sind, verabreicht.

Bei einer Ausführungsform wird die Verbindung der Formel I in Kombination mit Modulatoren des Glucocorticoidrezeptors (GR), wie sie z. B. in WO2005090336, WO2006071609, WO2006135826 beschrieben sind, verabreicht.

Bei einer weiteren Ausführungsform wird die Verbindung der Formel I in Kombination mit

CART-Modulatoren (siehe "Cocaine-amphetamine-regulated transcript influences energy metabolism, anxiety and gastric emptying in mice" Asakawa, A. et al.: Hormone and Metabolie Research (2001), 33(9), 554-558);

NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);

NPY-5 Rezeptorantagonisten wie L-152804 oder wie sie z. B. in WO2006001318 beschrieben sind;

NPY-4-Rezeptorantagonisten wie sie z. B. in WO2007038942 beschrieben sind; NPY-2-Rezeptorantagonisten wie sie z. B. in WO2007038943 beschrieben sind;

Peptid YY 3-36 (PYY3-36) oder analoge Verbindungen wie z. B. CJC-1682 (PYY3-36 konjugiert mit humanem Serum Albumin über Cys34) oder CJC-1643 (Derivat des PYY3-36, welches sich in vivo an Serum Albumin konjugiert) oder solche, wie sie in WO2005080424, WO2006095166 beschrieben sind;

Derivate des Peptids Obestatin wie sie WO2006096847 beschrieben sind;

CB1R (Cannabinoid Rezeptor 1) Antagonisten (wie z.B. Rimonabant, SR147778, SLV-319, AVE-1625, MK-0364 oder Salze davon oder solche Verbindungen wie sie in z. B. EP 0656354, WO 00/15609, WO2001/64632, WO2001/64633, WO2001/64634, WO 02/076949, WO2005080345, WO2005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120,

WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006047516, WO2006060461, WO2006067428, WO2006067443, WO2006087480, WO2006087476, WO2006100208, WO2006106054, WO2006111849, WO2006113704, WO2007009705, WO2007017124, WO2007017126, WO2007018459, WO2007016460, WO2007020502, WO2007026215, WO2007028849, WO2007031720, WO2007031721, WO2007036945, WO2007038045, WO2007039740, US20070015810, WO2007046548, WO2007047737 beschrieben sind);

Cannabinoid Rezeptor 1 / Cannabinoid Rezeptor 2 (CB1/CB2) modulierende Verbindungen wie sie z.B. in WO2007001939, WO2007044215, WO2007047737 beschrieben sind;

MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chloro-phenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950, WO2004087159, WO2004078717, WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, WO2005118573, EP1538159, WO2004072076, WO20040720777, WO2006021655-57, WO2007009894, WO2007015162, WO2007041061, WO2007041052 beschrieben sind;

Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224 beschrieben sind);

Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893, WO2006107661, WO2007003804, WO2007016496, WO2007020213 beschrieben sind);

Histamin H1 / Histamin H3 Modulatoren, wie z. B. Betahistin bzw. seinem Dihydrochlorid; CRF-Antagonisten (z.B. [2-Methyl-9-(2,4,6-trimethyl-phenyl)-9H-1,3,9-triaza-fluoren-4-yl]-dipropyl-amin (WO 00/66585));

CRF BP-Antagonisten (z.B. Urocortin);

Urocortin-Agonisten;

Agonisten des beta-3 Adrenoceptors wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553, WO2002038543, WO2007048840-843 beschrieben sind;

MSH (Melanocyt-stimulierendes Hormon)-Agonisten;

MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2005085200, WO2005019240, WO2004011438, WO2004012648, WO2003015769, WO2004072025, WO2005070898, WO2005070925, WO2004039780, WO2004092181, WO2003033476, WO2002006245, WO2002089729, WO2002002744, WO2003004027, FR2868780, WO2006010446, WO2006038680, WO2006044293, WO2006044174, JP2006176443, WO2006018280, WO2006018279, WO2006118320, WO2006130075, WO2007018248, WO2007012661, WO2007029847, WO2007024004, WO2007039462, WO2007042660, WO2007042668, WO2007042669, US2007093508, US2007093509, WO2007048802, JP2007091649 beschrieben sind);

CCK-A Agonisten (wie z.B. {2-[4-(4-Chloro-2,5-dimethoxy-phenyl)-5-(2-cyclohexyl-ethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180) oder solchen, wie sie in WO2005116034 beschrieben sind;

Serotonin-Wiederaufnahme-Inhibitoren (z.B. Dexfenfluramine);

gemischte Serotonin-/Dopamin-Wiederaufnahme-Inhibitoren (z.B. Bupropion) oder feste Kombinationen von Bupropion mit Naltrexon;

gemischte Sertonin- und noradrenerge Verbindungen (z.B. WO 00/71549);

5-HT-Rezeptor Agonisten z.B. 1-(3-Ethyl-benzofuran-7-yl)-piperazin Oxalsäuresalz (WO 01/09111);

gemischte Dopamin/Norepinephrin/Acetylcholin-Wiederaufnahme-Inhibitoren (z.B. Tesofensine);

5-HT2C Rezeptor Agonisten (wie z.B. Lorcaserin Hydrochlorid (APD-356) oder BVT-933 oder solche, wie sie in WO200077010, WO200077001-02, WO2005019180, WO2003064423, WO200242304, WO2005035533, WO2005082859, WO2006077025, WO2006103511 beschrieben sind);

5-HT6 Rezeptor Modulatoren, wie z.B. E-6837 oder BVT-74316 oder solche wie sie z.B. in WO2005058858, WO2007054257 beschrieben sind;

Bombesin-Rezeptor Agonisten (BRS-3 Agonisten;

Galanin-Rezeptor Antagonisten;
Wachstumshormon (z.B. humanes Wachstumshormon oder AOD-9604); Wachstumshormon freisetzende Verbindungen (6-Benzyloxy-1-(2-diisopropylamino-ethylcarbamoyl)-3,4-dihydro-1H-isochinolin-2-carbonsäuretertiärbutylester (WO 01/85695)); Growth Hormone Secretagogue Receptor Antagonisten (Ghrelin Antagonisten) wie z. B. A-778193 oder solchen, wie sie in WO2005030734 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin, Doprexin);
Lipase/Amylase-Inhibitoren (z.B. WO 00/40569);
Inhibitoren der Diacylglycerol O-Acyltransferasen (DGATs) wie z. B. BAY-74-4113 oder wie z. B. in US2004/0224997, WO2004094618, WO200058491, WO2005044250, WO2005072740, JP2005206492, WO2005013907, WO2006004200, WO2006019020, WO2006064189, WO2006082952, WO2006120125, WO2006113919, WO2006134317, WO2007016538 beschrieben;
Inhibitoren der Fettsäuresynthase (FAS) wie z.B. C75 oder solchen, wie in WO2004005277 beschrieben;
Inhibitoren der Stearoyl-CoA delta9 Desaturase (SCD1) wie sie z.B. in WO2007009236, WO2007044085, WO2007046867, WO2007046868, WO20070501124 beschrieben sind;
Oxyntomodulin;
Oleoyl-Estron;
oder Agonisten oder partiellen Agonisten des Schilddrüsenhormonrezeptors (thyroid hormone receptor agonists) wie z. B: KB-2115 oder solche, wie in WO20058279, WO200172692, WO200194293, WO2003084915, WO2004018421, WO2005092316, WO2007003419, WO2007009913, WO2007039125 beschrieben, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.

Bei einer Ausführungsform ist der weitere Wirkstoff ein Modulator des Enzyms SIRT1.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer Ausführungsform ist der weitere Wirkstoff ein Diphenylazetidinonderivat, wie z.B. in US 6,992,067 oder US 7,205,290 beschrieben.

Bei einer Ausführungsform wird die Verbindung der Formel I in Kombination mit Ballaststoffen, vorzugsweise unlöslichen Ballaststoffen (siehe z.B. Carob/ Caromax® (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax® kann in einer Zubereitung erfolgen, oder durch getrennte Gabe von Verbindungen der Formel I und Caromax® Caromax® kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

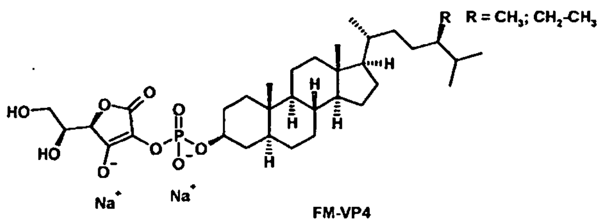
$R = CH_3; CH_2-CH_3$

FM-VP4

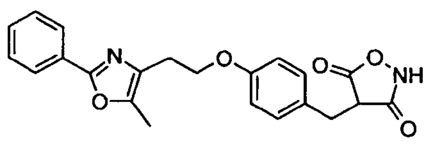

JTT-501

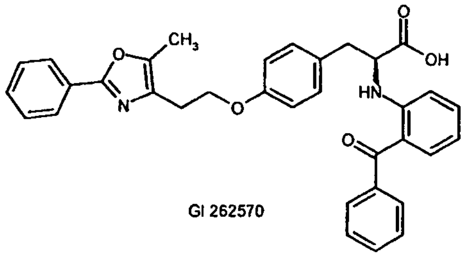

GI 262570

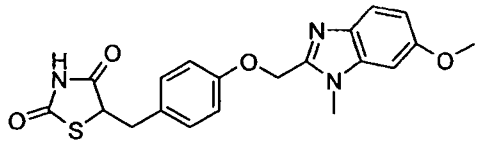

CS-011
Rivoglitazone

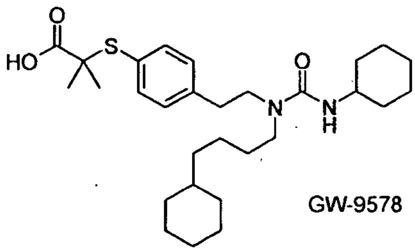

GW-9578

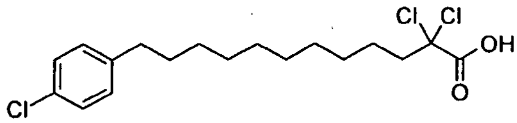

K-111

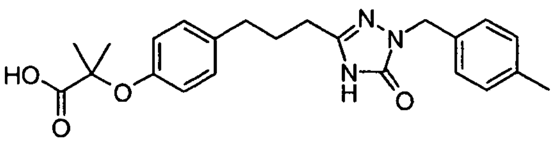

LY-674

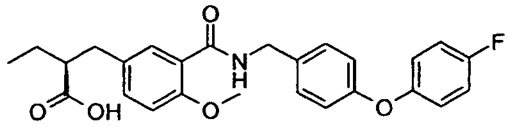

KRP-101

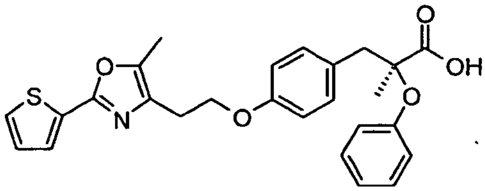

LY-510929

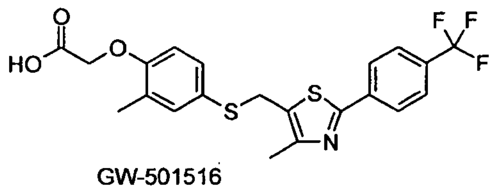

GW-501516

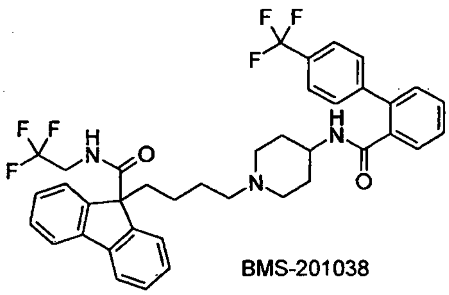

BMS-201038

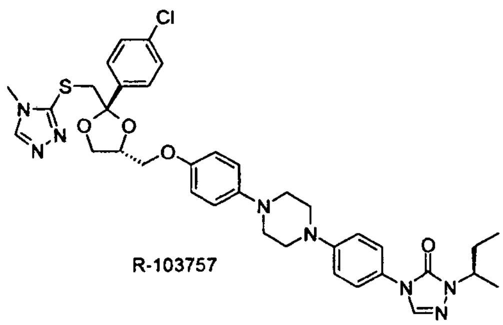

R-103757

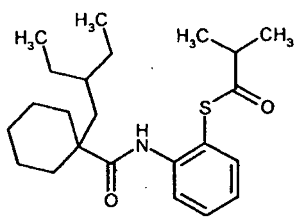

JTT-705

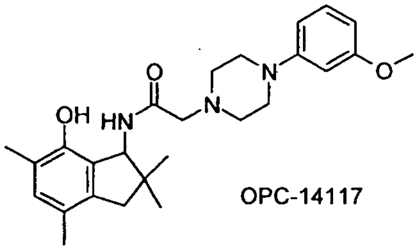

OPC-14117

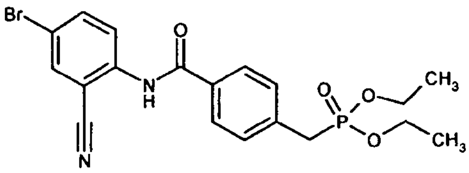

NO-1886

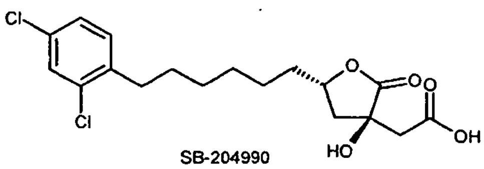

SB-204990

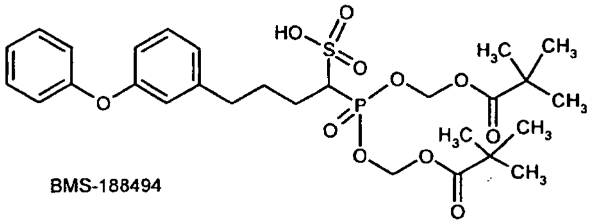

BMS-188494

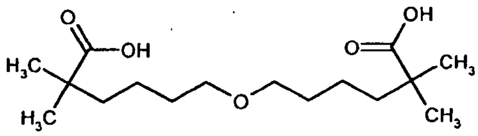

CI-1027

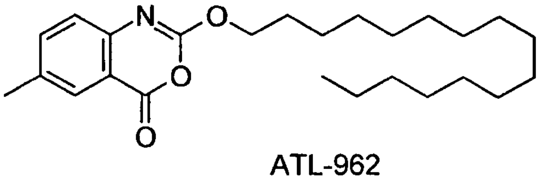

ATL-962

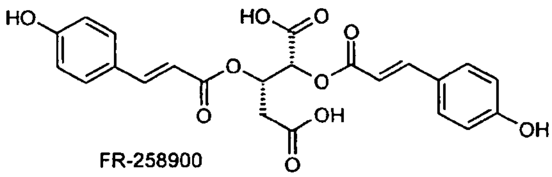

FR-258900

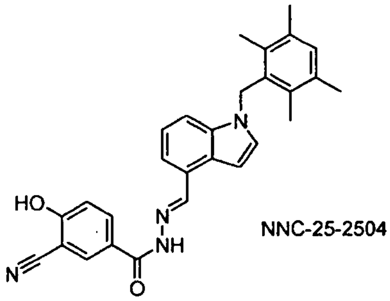

NNC-25-2504

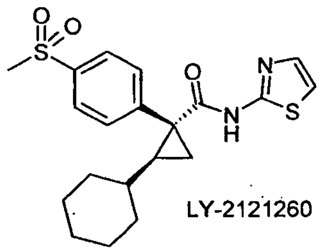

LY-2121260

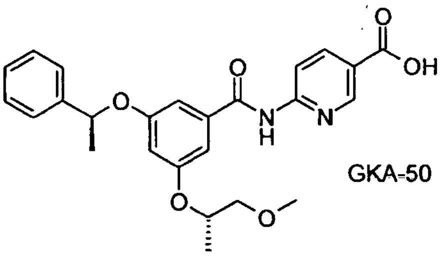

GKA-50

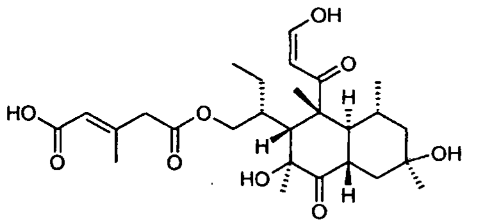

FR-225654

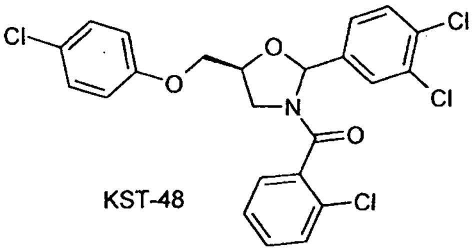

KST-48

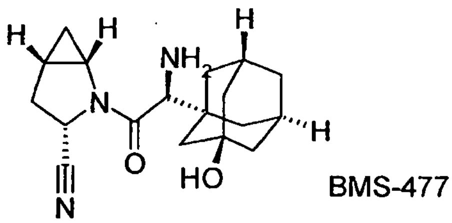

BMS-477118

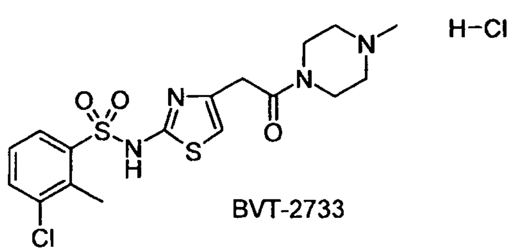

BVT-2733

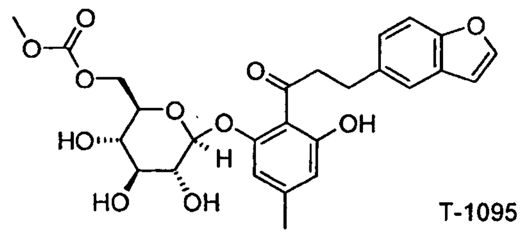

T-1095

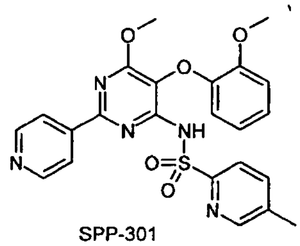

SPP-301

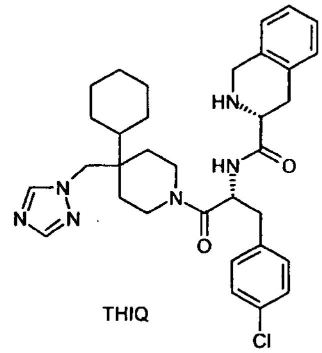

THIQ

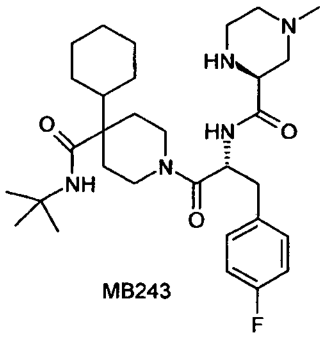

MB243

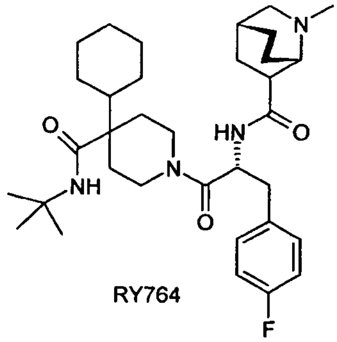

RY764

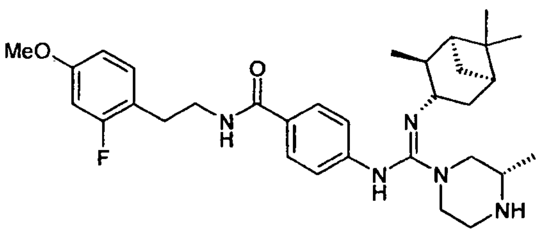

CHIR-785

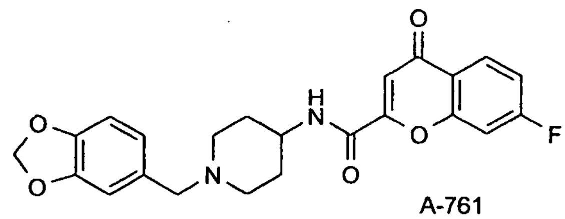

A-761

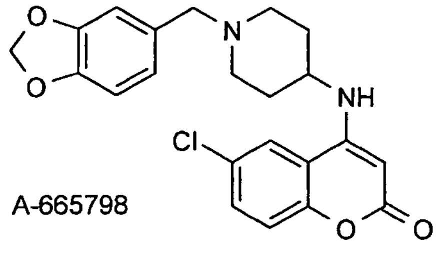

A-665798

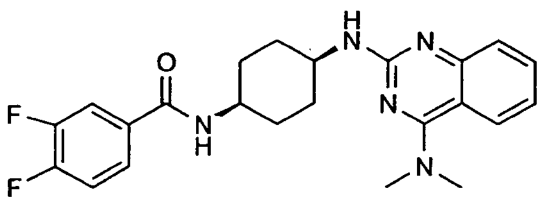

ATC-0175

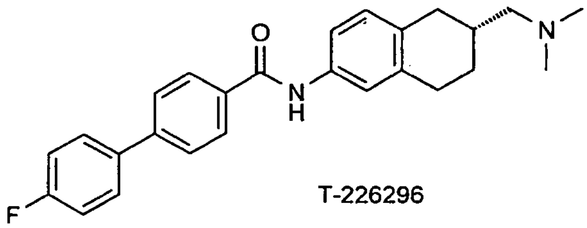

T-226296

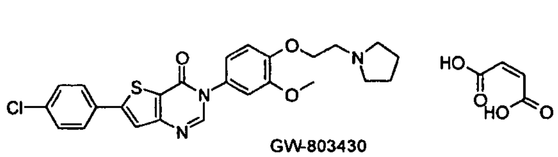

GW-803430

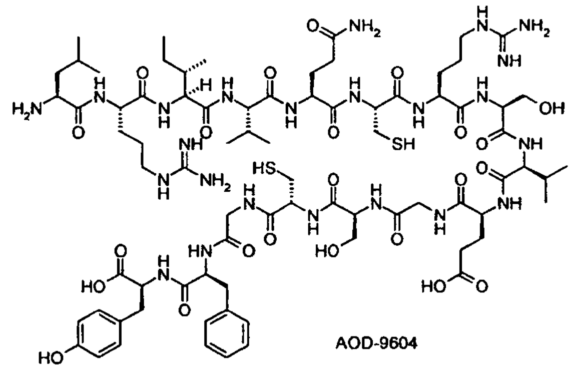

AOD-9604

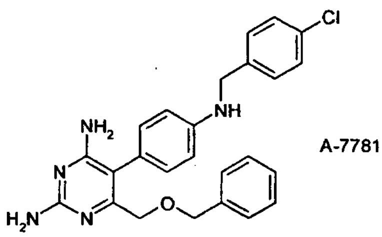

A-778193

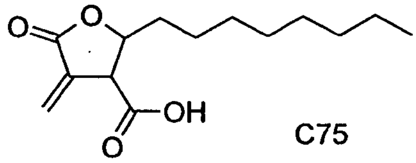

C75

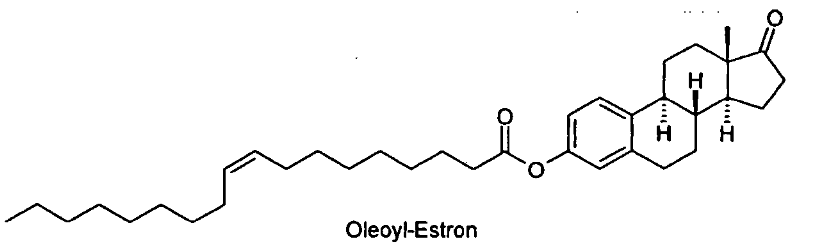

Oleoyl-Estron

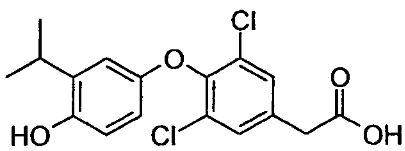

KB-2115

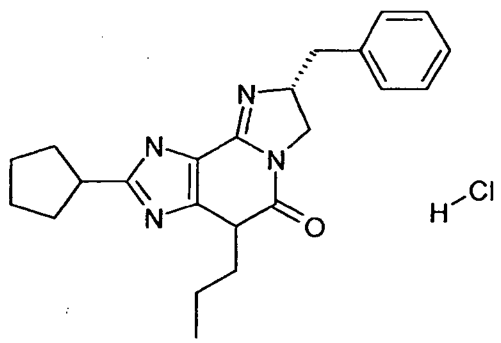

KCP-265

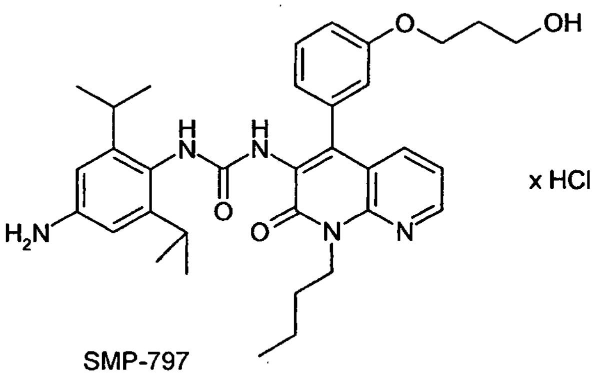

x HCl

SMP-797

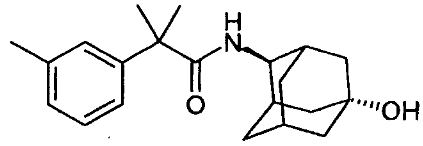

JNJ-25918646

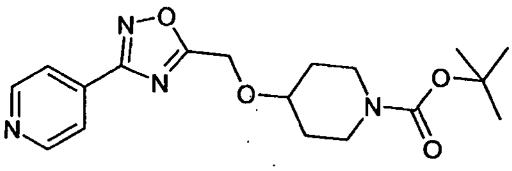

PSN-632408

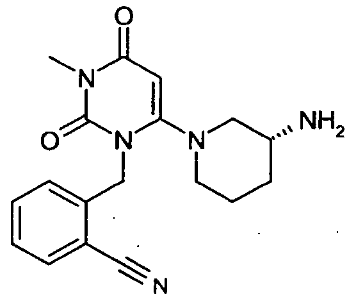

SYR-322

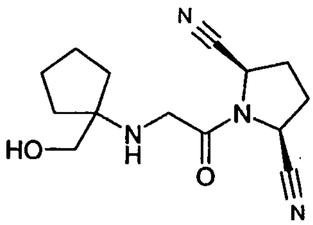

x HCl

DP-893

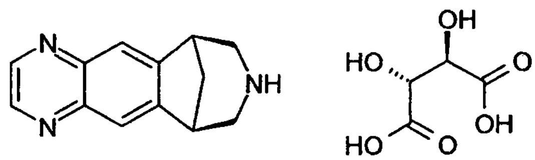

Varenicline Tartrat

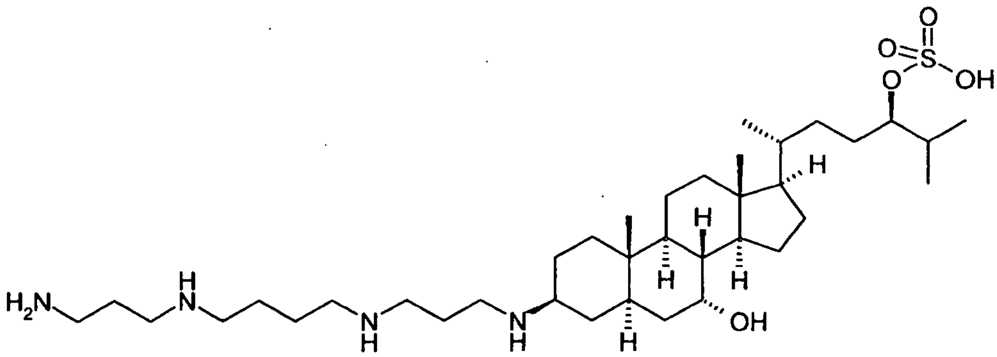

Trodusquemine

x HCl

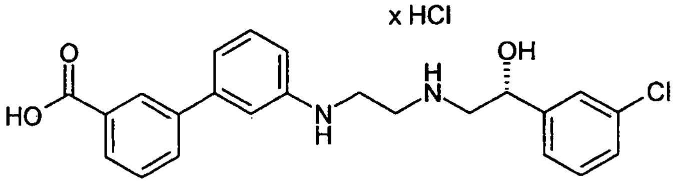

Solabegron

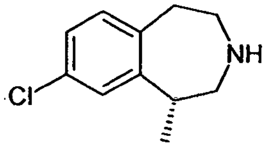

x HCl

Lorcaserin Hydrochlorid

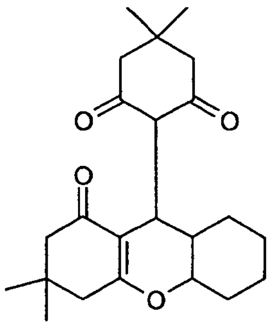

L-152804

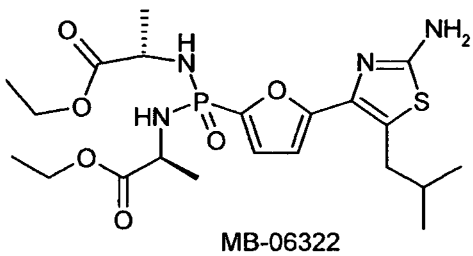

MB-06322
CS-917

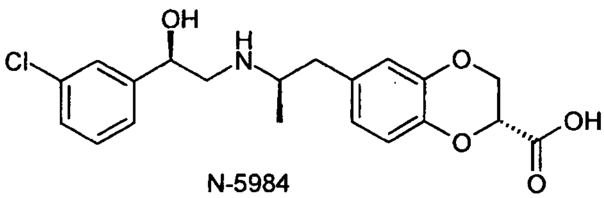

N-5984

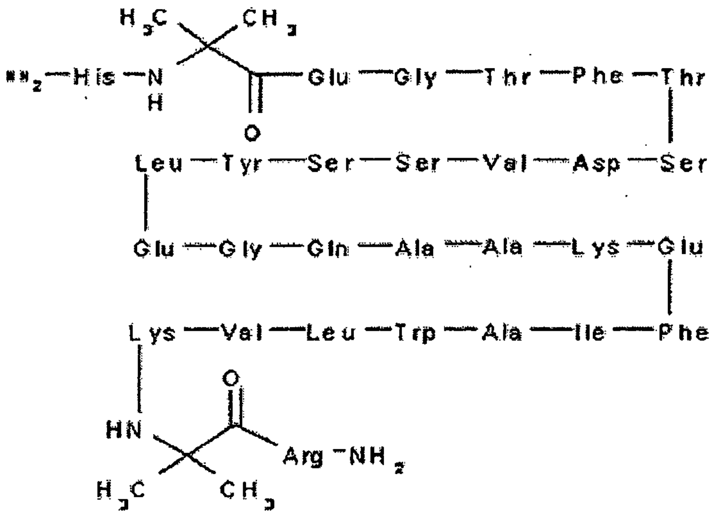

BIM-51077

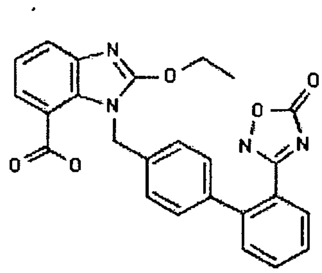

TAK-536

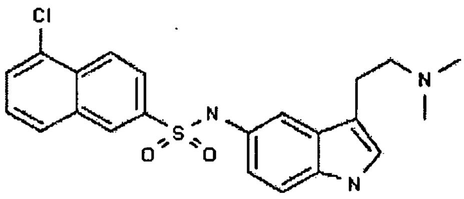

E-6837

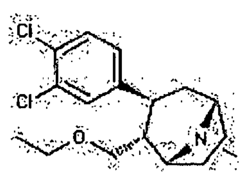

Tesofensine

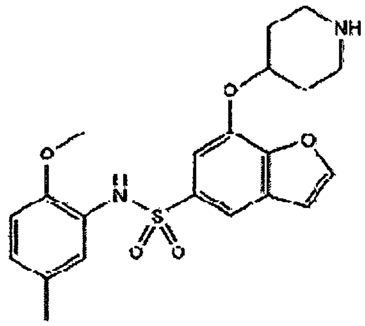

BVT-74316

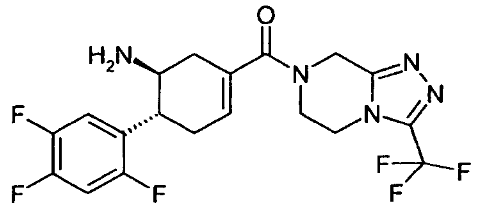

x $CF_3COOH$

ABT-341

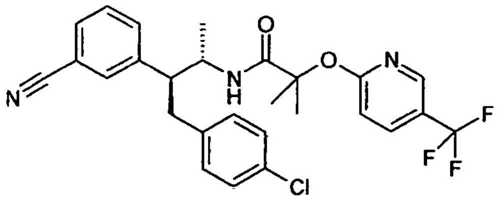

MK-0364

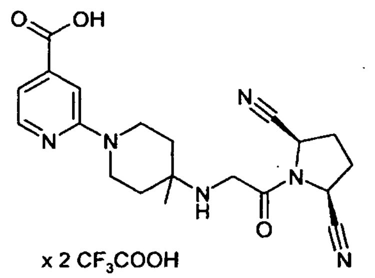

x 2 $CF_3COOH$

ABT-279

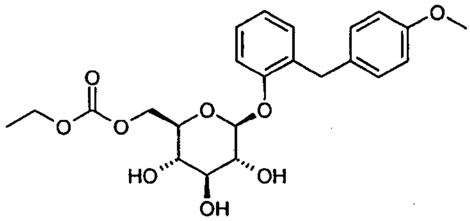

Sergliflozin

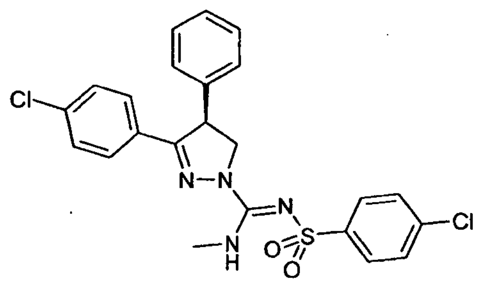

SLV-319

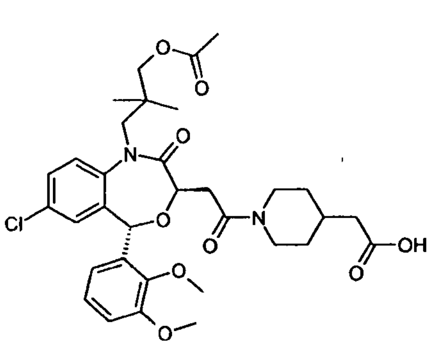

TAK-475

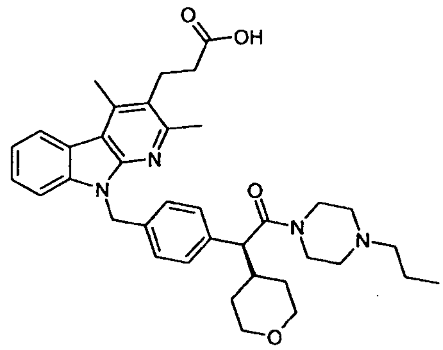

AS-1552133

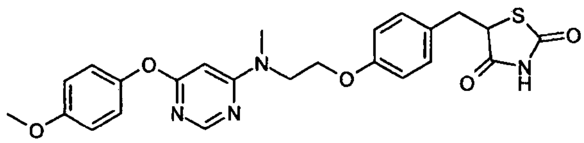

x $H_2SO_4$

CKD-501 (Lobeglitazon Sulfat)

Weiterhin sind folgende Wirkstoffe für Kombinationspräparate geeignet:

Alle Antiepileptika, die in der Roten Liste 2006, Kapitel 15 genannt sind;
alle Antihypertonika, die in der Roten Liste 2006, Kapitel 17 genannt sind;
alle Hypotonika, die in der Roten Liste 2006, Kapitel 19 genannt sind;
alle Antikoagulantia, die in der Roten Liste 2006, Kapitel 20 genannt sind;
alle Arteriosklerosemittel, die in der Roten Liste 2006, Kapitel 25 genannt sind;
alle Betarezeptoren-, Calciumkanalblocker und Hemmstoffe des Renin-Angiotensin-Systems, die in der Roten Liste 2006, Kapitel 27 genannt sind;
alle Diuretika und Durchblutungsfördernde Mittel, die in der Roten Liste 2006, Kapitel 36 und 37 genannt sind;
alle Entwöhnungsmittel/Mittel zur Behandlung von Suchterkrankungen, die in der Roten Liste 2006, Kapitel 39 genannt sind;
alle Koronarmittel und Magen-Darm-Mittel, die in der Roten Liste 2006, Kapitel 55 und 60 genannt sind;
alle Migränemittel, Neuropathiepräparate und Parkinsonmittel, die in der Roten Liste 2006, Kapitel 61, 66 und 70 genannt sind.

Gegenstand der Erfindung sind weiterhin Verfahren zur Herstellung der Verbindungen der allgemeinen Formel I, dadurch gekennzeichnet, dass man die Verbindungen der Formel I so gewinnt, dass analog den folgenden Reaktionsschemata vorgegangen wird:
Verfahren "A":

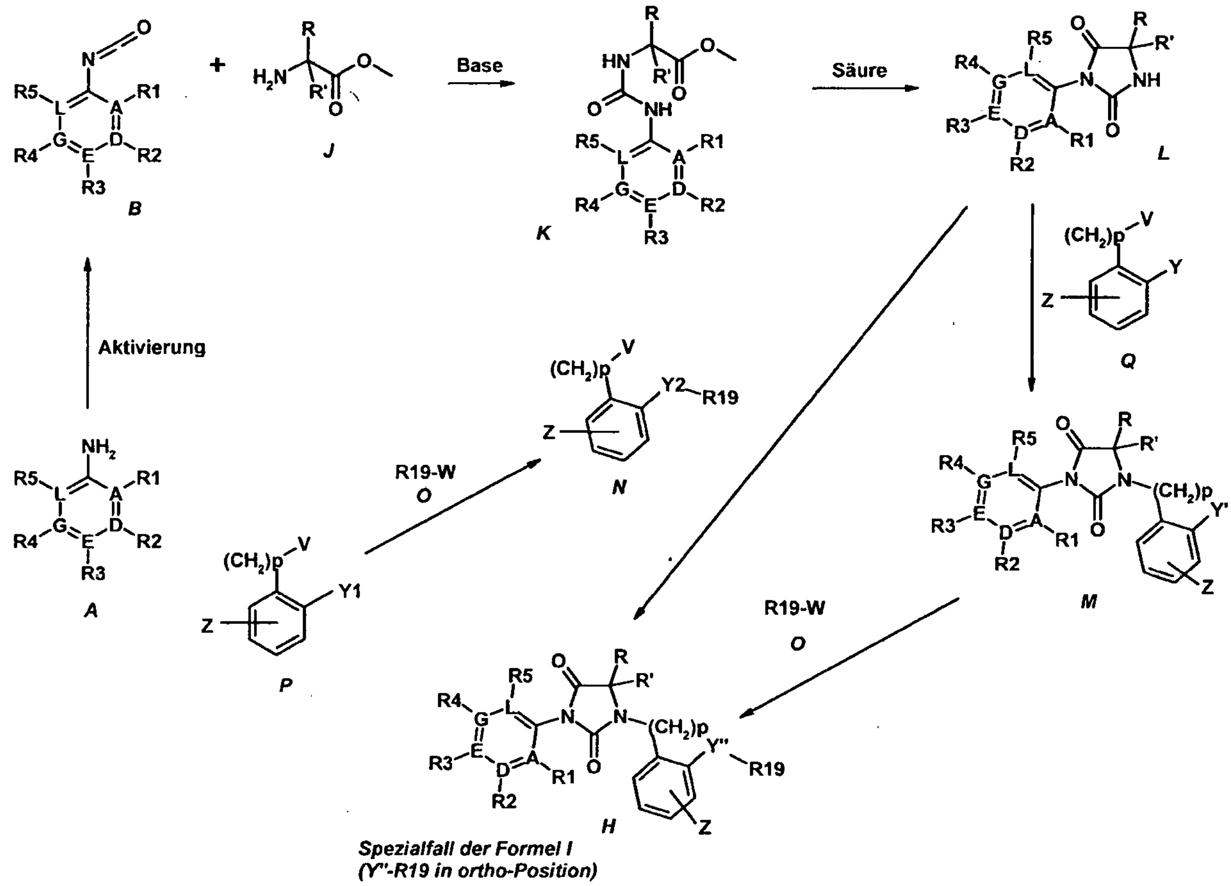


*Spezialfall der Formel I*
*(Y"-R19 in ortho-Position)*

## Verfahren "A"

In einem ersten Verfahren "A" wird so vorgegangen, dass ein geeignet substituiertes Anilin der Formel ***A***, in welchem die Reste R1 bis R5 u. U. in geschützter Form vorliegen, in ein Isocyanat der Formel ***B*** umgesetzt wird. Diese Umsetzung kann z. B. mit Phosgen in Toluol oder mit Diphosgen oder Triphosgen durchgeführt werden. Das Isocyanat **B** wird anschließend mit dem Methylester oder einem anderen Ester (z.B. tert.-Butyl) der Aminosäure ***J,*** in welcher R und R' die in Formel I genannten Bedeutungen haben, oder einem Salz eines Esters der Aminosäure ***J*** unter Zugabe von Base (z. B. Triethylamin) zu einem Harnstoff der Formel ***K*** umgesetzt. Dieser Harnstoff kann unter basischen oder sauren Bedingungen, vorzugsweise sauren Bedingungen, zum Imidazolidin-2,4-dion der Formel ***L*** ringgeschlossen werden. Die weitere Umsetzung zu einer Verbindung der Formel ***H***, welches den ortho-substituierten Spezialfall einer Verbindung der Formel I darstellt, kann z. B. so erfolgen, dass mit einer geeignet substituierten Verbindung Q, wobei Z ein oder mehrere Substituenten wie weiter oben in Formel I beschrieben sein kann, und Y entweder ein Halogenatom, vorzugsweise ein Bromatom, oder aber eine geeignet geschützte Aminofunktion (z. B. Isoindol-1,3-dion-2-yl oder N=CH-$N(CH_3)_2$), darstellt, und V entweder auch ein Halogenatom, vorzugsweise ein Chlor- oder Bromatom, oder aber zum Beispiel einen $O-SO_2-C_6H_4-4-CH_3$-Rest oder einen $O-SO_2-CH_3$-Rest oder einen $O-SO_2-CF_3$-Rest darstellt, unter Erhalt der Verbindung ***M*** alkyliert wird. ***M*** kann unter Buchwald-Hartwig-Bedingungen (z. B.: S.L.Buchwald et al.: Acc. Chem. Res. 1998, 31, 805; J.F.Hartwig et al.: J. Org. Chem. 1999, 64, 5575-5580; J.P.Wolfe et al.: J. Org. Chem. 2000, 65, 144-1157; M.D.Charles et al.: Org. Lett. 2005, 7, 3965-68) zu Verbindungen der Fomel ***H*** weiter umgesetzt werden. Dabei hat Y' in ***M*** die Bedeutung Br bzw. $NH_2$, wenn im Reaktionspartner ***O*** die Bedeutung von W $NH_2$ bzw. Br ist.

Oder die weitere Umsetzung der Verbindung ***L*** zur Verbindung ***H*** kann so erfolgen, dass ***L*** mit einer Verbindung der Formel ***N***, wobei V die eben geschilderten Bedeutungen haben kann, und wobei Y2 die Bedeutung NH oder N-Schutzgruppe haben kann, alkylierend umgesetzt wird. Die Verbindung ***N*** ihrerseits kann durch Reaktion von ***P***, worin V die oben beschriebenen Bedeutungen haben kann, und wobei Y1 Brom oder $NH_2$ bedeutet mit einer eventuell substituierten R19-W-Verbindung ***O*** unter z. B. Buchwald-Hartwig-Bedingungen erhalten werden. Dabei hat W die Bedeutung $NH_2$, wenn Y1 die Bedeutung Br hat und Br, wenn Y1 die Bedeutung $NH_2$ hat. R19 hat die Bedeutung substituiertes oder unsubstituiertes Aryl, Heteroaryl oder bicyclisches Heteroaryl.

Eventuell vorhandene Schutzgruppen der Verbindung ***H*** können am Ende entfernt werden und der Rest Y" kann nach Bedarf mittels Standardreaktionen von NH oder N-Schutzgruppe nach NR17 weiter umgesetzt werden.

Die hier gezeigte Formel ***H*** stellt einen Spezialfall der Formel I dar, worin sich der Rest Y"-R19 in Formel I in der ortho-Position befindet; dieser Rest kann sich sinngemäß auch in meta- oder para-Position befinden.

Eine Variante des Verfahrens "A" stellt das Verfahren "A'" dar:

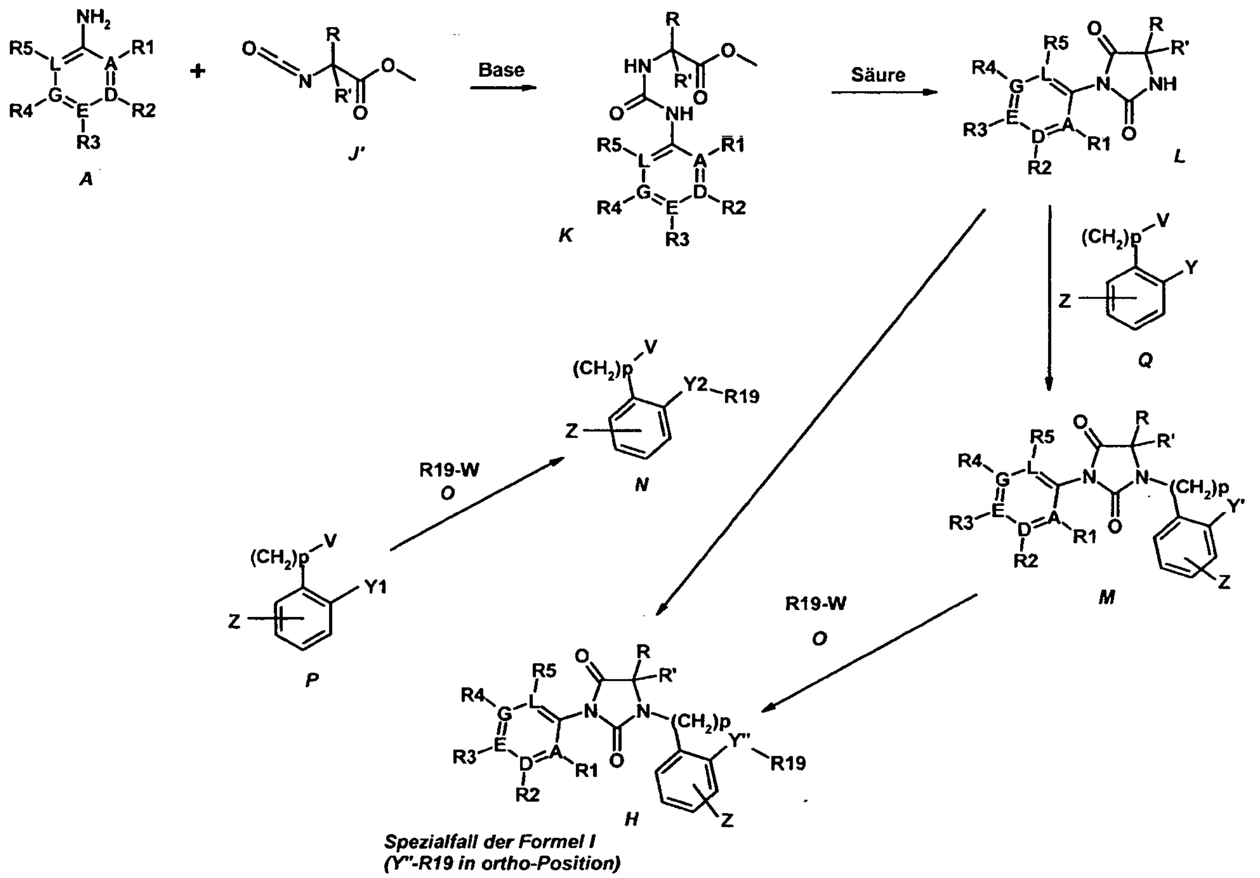


***Spezialfall der Formel I***
***(Y"-R19 in ortho-Position)***

## Verfahren „A'"

Im Verfahren "***A'***" wird das Amin ***A*** mit dem Isocyanat des Aminosäureesters ***J'*** unter Bildung der Verbindung ***K*** zur Reaktion gebracht. Die weiteren Schritte können wie bei Verfahren "A" erfolgen.

In einem anderen Verfahren "B"

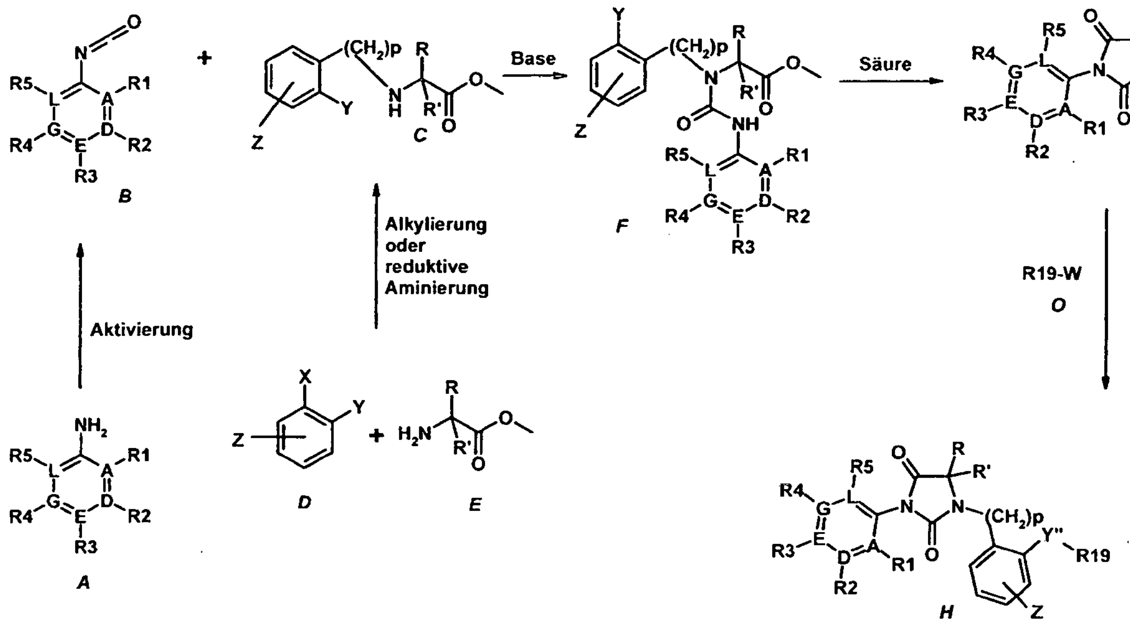


*Spezialfall der Formel I*
*(Y"-R19 in ortho-Position)*

## Verfahren „B"

wird das Isocyanat ***B*** mit einem geeignet substituierten Aminosäureesterderivat ***C***, worin die jeweiligen Substituenten gegebenenfalls mit Schutzgruppen versehen sind, und wobei der im Schema gezeigte Methylester ein nicht limitierendes Beispiel für einen Ester ist, und wobei Y Brom oder eine geschützte Aminofunktion (z. B. $N\text{-}CO\text{-}CH_3$ oder $N{=}CH\text{-}N(CH_3)_2$) ist unter Zugabe einer Base (z. B. Triethylamin) zu einem Harnstoff der Formel ***F*** umgesetzt. Das Aminosäureesterderivat ***C*** kann aus der Verbindung ***D***, worin Z ein oder mehrere Substituenten wie weiter oben in Formel I beschrieben sein kann, und wobei Y Brom oder eine geschützte Aminofunktion und X eine $(CH_2)_p$-U-Gruppierung ist, worin U die Bedeutung Cl, Br, J, $O\text{-}SO_2\text{-}C_6H_4\text{-}4\text{-}CH_3$, $O\text{-}SO_2\text{-}CH_3$ oder $O\text{-}SO_2\text{-}CF_3$ haben kann, mit einem Aminosäureester der Formel ***E***, worin R und R' die in Formel I genannten Bedeutungen haben, unter alkylierenden Bedingungen hergestellt werden. Alternativ kann die Verbindung der Formel C durch reduktive Aminierung des Aldehyds ***D*** (Z und Y wie oben beschrieben und X = $(CH_2)_p\text{-}CHO$) mit dem Aminosäurederivat ***E*** gewonnen werden. Der Harnstoff ***F*** kann unter basischen oder sauren Bedingungen, vorzugsweise sauren Bedingungen, zum Imidazolidin-2,4-dion der Formel ***G*** ringgeschlossen werden. Je nachdem ob Y in der Verbindung der Formel ***G*** Brom oder $NH_2$ bedeutet, können durch Umsatz mit Verbindungen der Formel O***,*** worin W entweder $NH_2$ oder Brom bedeutet unter Buchwald-Hartwig-Bedingungen Verbindungen der Formel ***H*** hergestellt werden.

Eventuell vorhandene Schutzgruppen der Verbindung ***H*** können am Ende entfernt werden und der Rest Y" kann nach Bedarf mittels Standardreaktionen von NH nach NR17 weiter umgesetzt werden.

Die hier gezeigte Formel ***H*** stellt einen Spezialfall der Formel I dar, worin sich der Rest Y"-R19 in Formel I in der ortho-Position befindet; dieser Rest kann sich sinngemäß auch in meta- oder para-Position befinden.

In einem weiteren Verfahren (Verfahren "C")

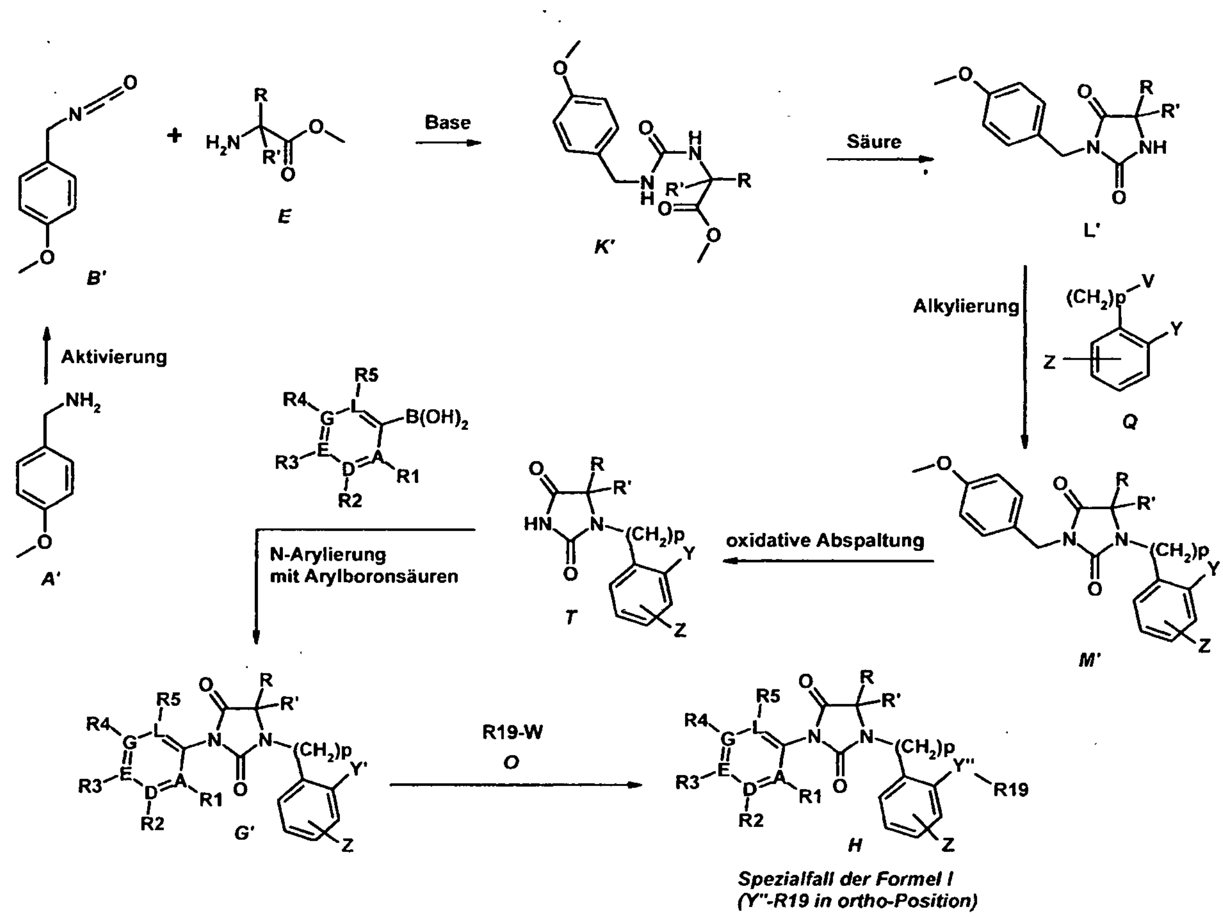


## Verfahren „C"

wird p-Methoxybenzylisocyanat ***B'*** mit einem Aminosäureester wie z. B. ***E,*** in welchem R und R' die in Formel I genannten Bedeutungen haben, unter basischen Bedingungen zum Harnstoff ***K'*** umgesetzt. Der Harnstoff ***K'*** kann unter basischen oder sauren Bedingungen, vorzugsweise sauren Bedingungen, zum Imidazolidin-2,4-dion der Formel ***L'*** ringgeschlossen werden. Die Verbindungen ***M'*** werden gewonnen, indem die Verbindungen ***L'*** mit den Verbindungen ***Q*** unter alkylierenden Bedingungen umgesetzt werden. Dabei haben Z, V und Y der Verbindungen ***Q*** die Bedeutungen wie sie im Verfahren "A" genannt sind. Die p-Methoxybenzylgruppe in den Verbindungen ***M'*** kann oxidativ unter Erhalt der Verbindungen ***T*** abgespalten werden. Die N-Arylierung des Imidstickstoffatoms in Verbindungen der Formel ***T*** unter Einsatz von Arylboronsäuren der Formel ***S*** nach Verfahren wie sie z. B. bei J.-B.Lan et al.: SYNLETT 2004, 1095-1097 oder D.M.T.Chan et al.: Tetrahedron Lett. 1998, 39, 2933-2936 beschrieben sind, liefert Verbindungen der Formel ***G'***. Je nachdem ob Y' in der Verbindung der Formel ***G'*** Brom oder $NH_2$ bedeutet, können durch Umsatz mit Verbindungen der Formel ***O,*** worin W entweder $NH_2$ oder Brom bedeutet, unter Hartwig-Buchwald-Bedingungen Verbindungen der Formel ***H*** hergestellt werden.

Eventuell vorhandene Schutzgruppen der Verbindung ***H*** können am Ende entfernt werden und der Rest Y" kann nach Bedarf mittels Standardreaktionen von NH nach NR17 weiter umgesetzt werden.

Die hier gezeigte Formel ***H*** stellt einen Spezialfall der Formel I dar, worin sich der Rest Y"-R19 in Formel I in der ortho-Position befindet; dieser Rest kann sich sinngemäß auch in meta- oder para-Position befinden.

Ein weiteres Verfahren "D" findet insbesondere Anwendung in der Synthese von alkyl-, cycloalkyl-, cycloalkenyl, arylalkylen-, heteroarylalkylen-, aryloxy-, heteroaryloxy, alkyloxy-, alkylthio-, cycloalkylthio-, arylthio-, heteroarylthio-, alkylcarbonyl-, cycloalkylcarbonyl-, arylcarbonyl-, heteroarylcarbonyl-, aryl- und heteroaryl-substituierten N3-aryl- oder N3-heteroaryl-substituierten Imidazolidin-2,4-dionen.

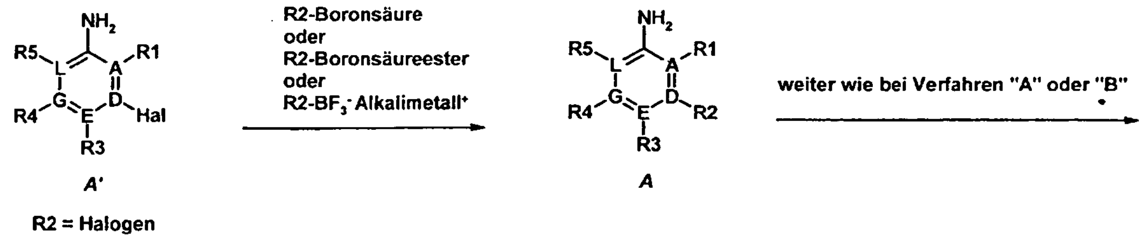


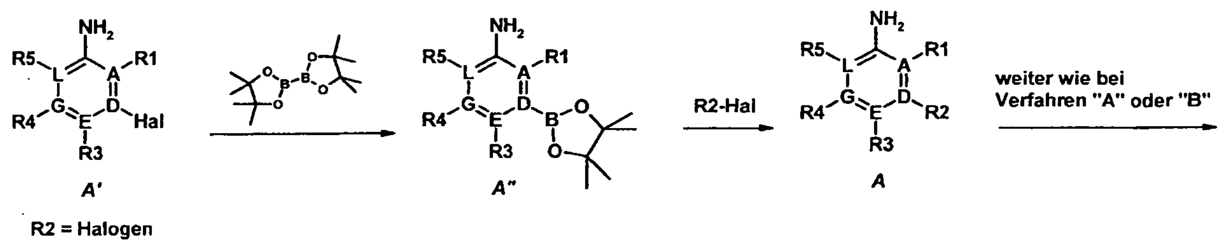


## Verfahren „D"

Zur Herstellung von Verbindungen, worin z. B. R2 = Alkyl, Cycloalkyl, Cycloalkenyl, Aryl oder Heteroaryl oder einen anderen der oben beschriebenen Reste darstellt, kann so vorgegangen werden, dass eine Verbindung der Formel ***A***', worin die Aminofunktion gegebenenfalls mit einer Schutzgruppe versehen ist und R2 gleich Halogen, bevorzugt Brom oder Chlor, ist, mit einer Alkyl-, Cycloalkyl, Cycloalkenyl-, Aryl- oder Heteroarylboronsäure oder einem Esterderivat davon oder einem R2-Trifluoroborat unter Bedingungen umgesetzt wird wie sie z.B. bei J. Zhou und G. C. Fu, J. Am. Chem. Soc. 126 (2004) 1340-1341; F. Gonzäles-Bobes und G. C. Fu, J. Am. Chem. Soc. 128 (2006) 5360-5361; D. J. Wallace und C.-Y. Chen, Tetrahedron Letters 43 (2002) 6987-6990; T. E. Barder et al., J. Am. Chem. Soc. 127 (2005) 4685-4696; D. W. Old et al., J. Am. Chem. Soc. 120 (1998) 9722; T. E. Barder und St. L. Buchwald, Org. Lett. 6 (2004) 2649-2652 beschrieben.sind. Die weitere Umsetzung der so mit R2 substituierten Verbindung ***A*** kann so erfolgen wie es für das Verfahren "A" und "B" beschrieben ist.

Bei dem Verfahren "D" kann auch so vorgegangen werden, dass die Verbindung ***A***', wobei R2 gleich Halogen, bevorzugt Chlor oder Brom, ist, unter Palladium-Katalyse mit einer Dibor-Verbindung, z. B. Bis(pinacolato)dibor, zum Arylboronat der Formel ***A***" mit R2 gleich -B(O-C$(CH_3)_2$-C$(CH_3)_2$-O) umgesetzt wird. In einem weiteren Schritt kann diese Verbindung mit einer Organohalogenverbindung R2-Hal zu Verbindungen der Formel ***A***, worin R2 z. B. Cycloalkyl oder Aryl bedeutet, umgesetzt werden. Die Folgereakionen zur Gewinnung der Verbindungen der Formel ***H*** können wiederum nach Verfahren "A" oder "B" erfolgen.

Verbindungen der Formel ***A***, worin R2 gleich -O/S-Alkyl, -O/S-Cycloalkyl, -O/S-$CH_2$-Aryl, - O/S-$CH_2$-Heteroaryl, -O/S-Aryl, -O/S-Heteroaryl ist, können aus Verbindungen der Formel ***A***', worin R2 gleich Halogen, bevorzugt Brom oder Chlor, ist, durch Umsatz mit den entsprechenden Alkoholen oder Phenolen bzw. Mercaptanen oder Mercaptoarylen und - heteroarylen und Cäsiumcarbonat unter Palladium- oder Kupfer-Katalyse hergestellt werden (siehe auch R. Frlan und D. Kikelj; Synthesis 14 (2006) 2271-2285; A. V. Vorogushin et al., J. Am. Chem. Soc. 127 (2005) 8146-8149; F. Y. Kwong und St. L. Buchwald, Org. Lett. 4 (2002) 3517-3520).

Verbindungen der Formel ***A***, worin R2 gleich -$CH_2$-Aryl oder -$CH_2$-Heteroaryl ist, können z.B. aus Verbindungen der Formel ***A***", durch Umsatz mit Halogenmethylarylen oder Halogenmethylheteroarylen, wobei Halogen bevorzugt Brom oder Chlor ist, unter basischen Bedingungen und Palladium-Katalyse erhalten werden (siehe auch S. M. Nobre und A. L. Monteiro, Tetrahedron Letters 45 (2004) 8225-8228; S. Langle et al., Tetrahedron Letters 44 (2003) 9255-9258).

Verbindungen wie sie über das Verfahren "D" zugänglich sind, können auch in einem weiteren Verfahren "E" gewonnen werden:

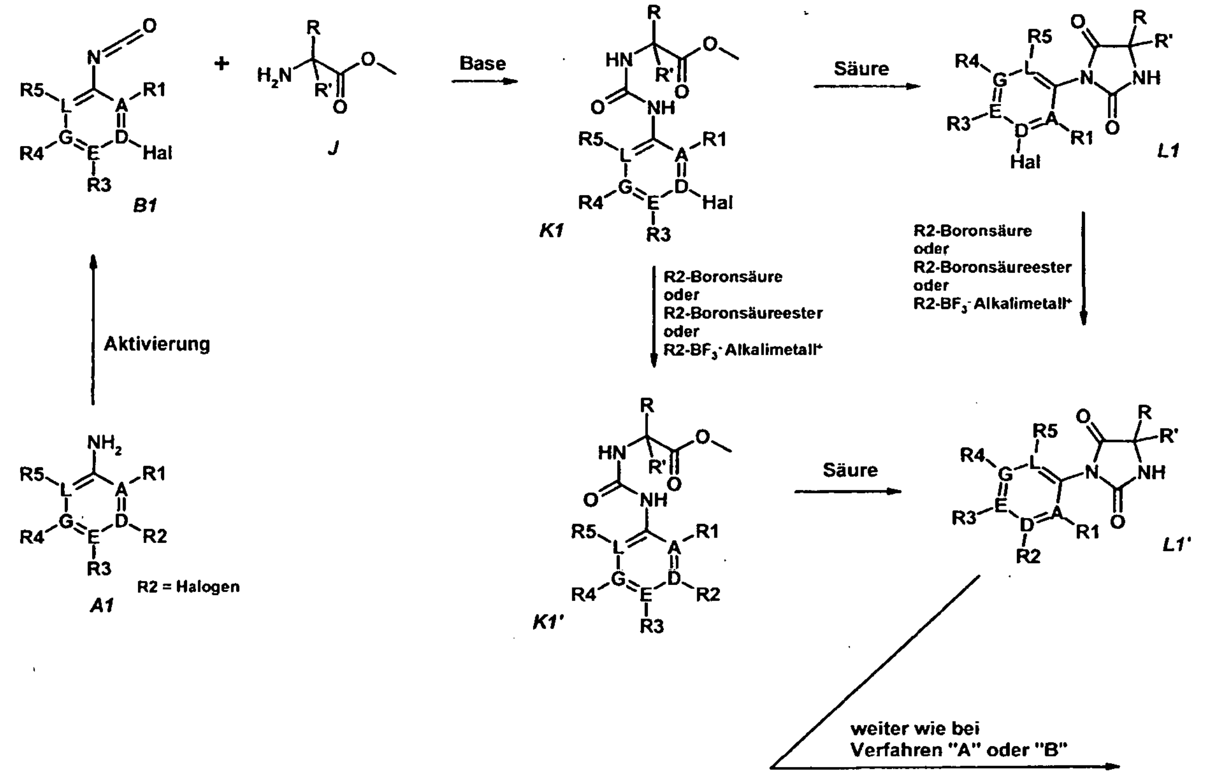
B1
J
Base
K1
Säure
L1
Aktivierung
R2-Boronsäure
oder
R2-Boronsäureester
oder
R2-$BF_3^-$ Alkalimetall$^+$
R2-Boronsäure
oder
R2-Boronsäureester
oder
R2-$BF_3^-$ Alkalimetall$^+$
A1
R2 = Halogen
K1'
Säure
L1'
weiter wie bei
Verfahren "A" oder "B"

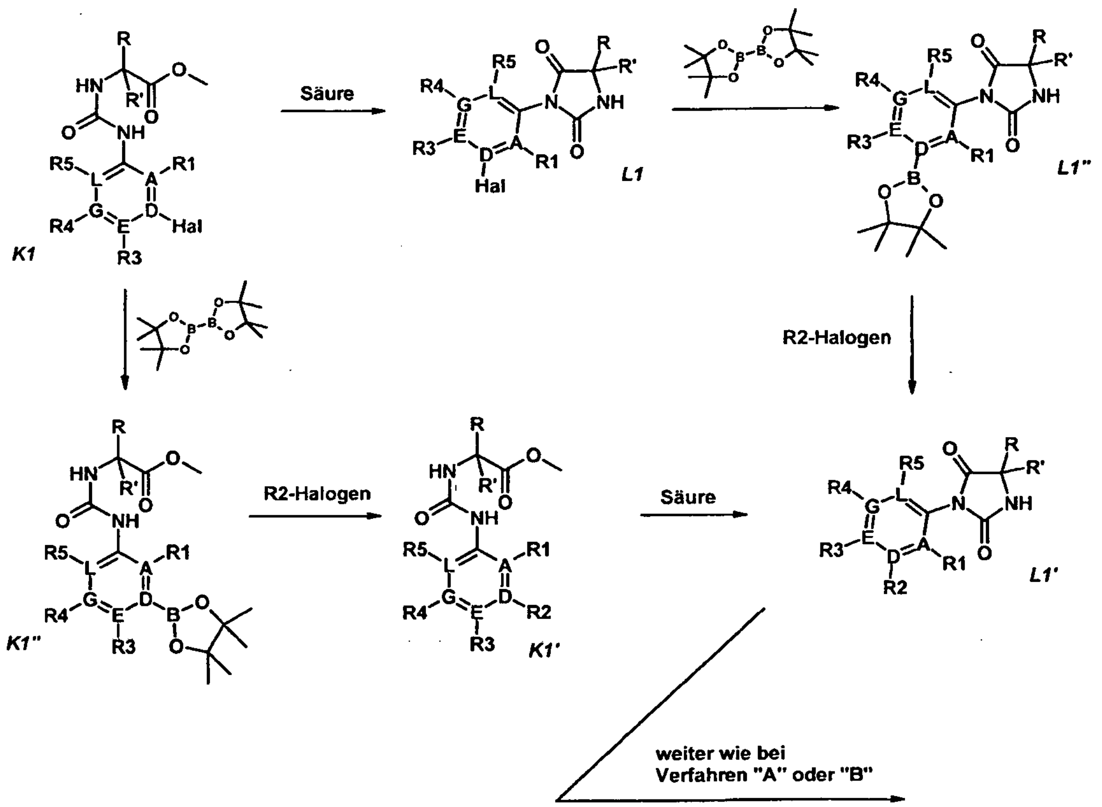
K1
Säure
L1
L1"
R2-Halogen
K1"
R2-Halogen
K1'
Säure
L1'
weiter wie bei
Verfahren "A" oder "B"

Verfahren „E“

Zur Herstellung von Verbindungen, worin z. B. R2 = Alkyl, Cycloalkyl, Cycloalkenyl, Aryl oder Heteroaryl oder einen anderen der oben beschriebenen Reste darstellt, kann so vorgegangen werden, dass eine Verbindung der Formel ***K1*** oder ***L1***, welche z.B. wie im Verfahren "A" beschrieben gewonnen werden, und worin R2 gleich Halogen, bevorzugt Brom oder Chlor, ist, mit einer Alkyl-, Cycloalkyl, Cycloalkenyl-, Aryl- oder Heteroarylboronsäure oder einem Esterderivat davon oder einem R2-Trifluoroborat unter Bedingungen umgesetzt wird wie sie z.B. bei J. Zhou und G. C. Fu, J. Am. Chem. Soc. 126 (2004) 1340-1341; F. Gonzäles-Bobes und G. C. Fu, J. Am. Chem. Soc. 128 (2006) 5360-5361; D. J. Wallace und C.-Y. Chen, Tetrahedron Letters 43 (2002) 6987-6990; T. E. Barder et al., J. Am. Chem. Soc. 127 (2005) 4685-4696; D. W. Old et al., J. Am. Chem. Soc. 120 (1998) 9722; T. E. Barder und St. L. Buchwald, Org. Lett. 6 (2004) 2649-2652 beschrieben sind. Die weitere Umsetzung der so mit R2 substituierten Verbindungen ***K1'*** und ***L1'*** kann so erfolgen wie es für das Verfahren "A" und "B" beschrieben ist.

Wie es für das Verfahren "D" beschrieben ist, kann auch im Verfahren "E" so vorgegangen werden, dass die Verbindungen ***K1*** oder ***L1*** mit einer Dibor-Verbindung, z. B. Bis(pinacolato)dibor, zum Arylboronat der Formel ***K1***" oder ***L1***" mit R2 gleich

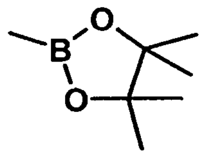

umgesetzt wird. In einem weiteren Schritt können diese Verbindungen mit einer Organohalogenverbindung R2-Hal zu Verbindungen der Formel ***K1***' oder ***L1***', worin R2 z. B. Cycloalkyl oder Aryl bedeutet, umgesetzt werden. Die Folgereakionen zur Gewinnung der erfindungsgemäßen Verbindungen kann so erfolgen wie es für das Verfahren "A" oder "B" weiter vorn beschrieben ist.

Auch die Verbindungen mit den weiteren Bedeutungen von R2, deren Herstellung nach dem Verfahren "D" erfolgen kann, können nach Verfahren "E" durch Umsatz der Stufen ***K1*** oder ***L1*** gewonnen werden.

Die nachfolgenden Beispiele dienen zur näheren Erläuterung der Erfindung.

<u>Allgemeine experimentelle Verfahren:</u>

$^1$H-NMR:

Die $^1$H-NMR Spektren wurden in deuteriertem Dimethylsulfoxid an einem 500 MHz-Gerät (DRX 500, Firma Bruker) bei $300^0$K gemessen. Angaben: $\delta$ in ppm, Multiplizität (s für Singulett, d für Dublett, t für Triplett, q für Quartett, m für Multiplett, x H (Anzahl der Wasserstoffatome)

HPLC/MS:

Die HPLC-MS-Messungen wurden an einem LCT-Gerät der Firma Waters durchgeführt. Säule: YMC Jsphere 33x2 4 $\mu$m; Gradient [A]: (Acetonitril+ 0.05% Trifluoressigsäure) : (Wasser + 0.05% Trifluoressigsäure) 5:95 (0 Minuten) nach 95:5 (3 Minuten); Gradient [B]: (Acetonitril+ 0.05% Trifluoressigsäure) : (Wasser + 0.05% Trifluoressigsäure) 5:95 (0 Minuten) nach 95:5 (2.5 Minuten) nach 95:5 (3.0 Minuten); Gradient [C]: (Acetonitril+ 0.05% Trifluoressigsäure) : (Wasser + 0.05% Trifluoressigsäure) 5:95 (0 Minuten) nach 95:5 (3.4 Minuten) nach 95:5 (4.4 Minuten); Gradient [D]: (Acetonitril+ 0.05% Trifluoressigsäure) : (Wasser + 0.05% Trifluoressigsäure) 2:98 (1 Minute) nach 95:5 (5 Minuten) nach 95:5 (6.25 Minuten); Gradient [E]: (Acetonitril+ 0.05% Trifluoressigsäure) : (Wasser + 0.05% Trifluoressigsäure) 5:95 (0 Minuten) nach 5:95 (0.5 Minuten) nach 95:5 (3.5 Minuten) nach 95:5 (4.0 Minuten); Gradient [F]: Säule: YMC Jsphere ODS H80 20x2 mm , 4 $\mu$m; (Wasser + 0.05% Trifluoressigsäure) : (Acetonitril + 0.05% Trifluoressigsäure) 96:4 (0 Minuten) nach 5:95 (2 Minuten) nach 96:4 (2.4 Minuten); Detektor: Tecan-LCT.

Chromatographische Reinigungsmethoden:

[RP1]: Fluss: 30 ml/min; Gradient: Acetonitril/Wasser + 0,1% Trifluoressigsäure; 30 min.
Säule: XTerra C18 5 $\mu$m 30x100 mm; Detektion: MS (ESI), UV (DAD).
[RP2]: Fluss: 150 ml/min; Gradient: Acetonitril/Wasser + 0,1% Trifluoressigsäure; 20 min.
Säule: XTerra C18 10 $\mu$m 50x250 mm; Detektion: MS (ESI), UV (DAD).

Beispiel 1: 4-[4,4-Dimethyl-2,5-dioxo-3-(2-phenylamino-benzyl)-Imidazolidin-1-yl]-2-trifluoro-methyl-benzonitril

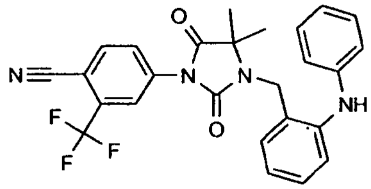

1) Herstellung von 4-(4,4-Dimethyl-2,5-dioxo-imidazolidin-1-yl)-2-trifluoromethylbenzonitril (**1.1**):

Die Verbindung **1.1** kann nach Verfahren "A" dargestellt werden. Dazu wurden 14,74 g (79,21 mMol) 4-Amino-2-trifluoromethyl-benzonitril in 200 ml trockenem Acetonitril gelöst. Diese Lösung wurde unter Rühren zu einer auf 70°C erwärmten 20%igen Lösung von Phosgen in Toluol zugetropft und anschließend 1 h gerührt. Die abgekühlte Reaktionslösung wurde im Vakuum eingeengt, der Rückstand mit Toluol aufgenommen und wieder im Vakuum eingeengt. Schließlich wurde der Rückstand in 150 ml trockenem Acetonitril gelöst und die Lösung unter Rühren mit 15,5 g (79,21 mMol) 2-Amino-2-methyl-propionsäure-tert.-butylester Hydrochlorid versetzt. Zu die Reaktionsmischung wurden langsam 12,02 g (118,8 mMol) Triethylamin zugetropft und anschließend 45 min. bei Raumtemperatur gerührt. Danach wurde die Mischung vorsichtig mit 50 ml konzentrierter Salzsäure versetzt und 1 h bei 70°C gerührt. Die abgekühlte Reaktionsmischung wurde im Vakuum eingeengt und der Rückstand mit Essigsäureethylester und Wasser versetzt. Die organische Phase wurde abgetrennt, mit gesättigter Natriumhydrogencarbonatlösung und anschließend mit Wasser gewaschen, über Natriumsulfat getrocknet, filtriert und im Vakuum eingeengt. Der Rückstand wurde chromatographisch über Kieselgel mit Heptan / Essigsäureethylester 2:1 gereinigt. Man erhielt 21,2 g (90% Ausbeute) 4-(4,4-Dimethyl-2,5-dioxoimidazolidin-1-yl)-2-trifluoromethyl-benzonitril **1.1** mit dem Schmelzpunkt 208 - 211°C.

2) Herstellung von 4-[3-(2-Brombenzyl)-4,4-dimethyl-2,5-dioxo-imidazolidin-1-yl]-2-trifluoromethyl-benzonitril (**1.2**):

Die Verbindung **1.2** kann nach Verfahren "A" dargestellt werden. Dazu wurden 21,2 g (71,32 mMol) der Verbindung **1.1** und 17,83 g (71,32 mMol) 2-Brombenzylbromid in 200 ml trockenem Acetonitril gelöst, mit 12,32 g Kaliumcarbonat versetzt und 5 h bei Raumtemperatur gerührt. Zur Aufarbeitung wurde die Reaktionsmischung mit Wasser versetzt, die Mischung mit Essigsäureethylester ausgeschüttelt, die organische Phase über Natriumsulfat getrocknet, filtriert und im Vakuum eingeengt. Der Rückstand wurde chromatographisch über Kieselgel mit Heptan / Essigsäureethylester 3:1 gereinigt. Man erhielt 28,5 g (86% Ausbeute) 4-[3-(2-Brom-benzyl)-4,4-dimethyl-2,5-dioxo-imidazolidin-1-yl]-2-trifluoromethyl-benzonitril (**1.2**) mit dem Schmelzpunkt 56 - 58°C.

3) Herstellung von 4-[4,4-Dimethyl-2,5-dioxo-3-(2-phenylamino-benzyl)-Imidazolidin-1-yl]-2-trifluoro-methyl-benzonitril (**1**):

Zur Herstellung der Verbindung des Beispiels 1 kann nach Verfahren "A" vorgegangen werden. Dazu wurden unter einer Argon-Atmosphäre nacheinander 49,98 mg (0,107 mMol) der Verbindung **1.2**, 14,98 mg (0.161 mMol) Anilin, 104,8 mg Cäsiumcarbonat, 2,4 mg Palladium-II-acetat und 12,4 mg 9,9-Dimethyl-4,5-bis(diphenylphosphino)xanthen zu 20 ml trockenem Dioxan gegeben und die Mischung 8 h bei 80°C gerührt. Die abgekühlte Reaktionsmischung wurde mit Wasser und Essigsäureethylester versetzt, die organische Phase abgetrennt, über Natriumsulfat getrocknet und im Vakuum eingeengt. Der Rückstand wurde chromatographisch gereinigt (Methode [RP1]). Man erhielt 45 mg (88% Ausbeute) der Verbindung des Beispiels **1** mit dem Molekulargewicht 478,16 ($C_{26}H_{21}F_3N_4O_2$); Retentionszeit $R_t$ = 2.33 min. [B]; MS (ESI): 479,43 ($MH^+$).

Die Verbindungen des Beispiels **2**, 4-{3-[2-(4-Fluoro-phenylamino)-benzyl]-4,4-dimethyl-2,5-dioxo-imidazolidin-1-yl}-2-trifluoromethyl-benzonitril (Molekulargewicht 496,15 ($C_{26}H_{20}F_4N_4O_2$); Retentionszeit $R_t$ = 2.25 min. [B]; MS (ESI): 497,28 ($MH^+$), der Beispiele
4, 4-{3-[2-(4-Chlor-phenylamino)-benzyl]-4,4-dimethyl-2,5-dioxo-imidazolidin-1-yl}-2-trifluoromethyl-benzonitril (Molekulargewicht 512,12 ($C_{26}H_{20}ClF_3N_4O_2$); Retentionszeit $R_t$ = 2.35 min. [B]; MS (ESI): 513,01 ($MH^+$),
5,4-{3-[2-(4-Trifluoromethyl-phenylamino)-benzyl]-4,4-dimethyl-2,5-dioxo-imidazolidin-1-yl}-2-trifluoromethyl-benzonitril (Molekulargewicht 546,14 ($C_{27}H_{20}F_6N_4O_2$); Retentionszeit $R_t$ = 2.34 min. [B]; MS (ESI): 547,09 ($MH^+$),
**11**, 4-{3-[2-(2,4-Difluoro-phenylamino)-benzyl]-4,4-dimethyl-2,5-dioxo-imidazolidin-1-yl}-2-trifluoromethyl-benzonitril (Molekulargewicht 514,14 ($C_{26}H_{19}F_5N_4O_2$); Retentionszeit $R_t$ = 2.32 min. [B]; MS (ESI): 515,17 ($MH^+$),

**12**, 4-{3-[2-(4-Trifluoromethoxy-phenylamino)-benzyl]-4,4-dimethyl-2,5-dioxo-imidazolidin-1-yl}-2-trifluoromethyl-benzonitril (Molekulargewicht 562,14 ($C_{27}H_{20}F_6N_4O_3$); Retentionszeit $R_t$ = 2.46 min. [B]; MS (ESI): 563,15 ($MH^+$),
**13**, 4-{3-[2-(2,4-Dichlor-phenylamino)-benzyl]-4,4-dimethyl-2,5-dioxo-imidazolidin-1-yl}-2-trifluoromethyl-benzonitril (Molekulargewicht 546,08 ($C_{26}H_{19}Cl_2F_3N_4O_2$); Retentionszeit $R_t$ = 2.52 min. [B]; MS (ESI): 547,14 ($MH^+$),
**20**, 4-{3-[2-(3,4-Dichlor-phenylamino)-benzyl]-4,4-dimethyl-2,5-dioxo-imidazolidin-1-yl}-2-trifluoromethyl-benzonitril (Molekulargewicht 546,08 ($C_{26}H_{19}Cl_2F_3N_4O_2$); Retentionszeit $R_t$ = 2.50 min. [B]; MS (ESI): 547,11 ($MH^+$),
**21**, 4-{3-[2-(2-Chlor-phenylamino)-benzyl]-4,4-dimethyl-2,5-dioxo-imidazolidin-1-yl}-2-trifluoromethyl-benzonitril (Molekulargewicht 512,12 ($C_{26}H_{20}ClF_3N_4O_2$); Retentionszeit $R_t$ = 2.37 min. [B]; MS (ESI): 513,11 ($MH^+$),
**22**, 4-{3-[2-(3-Chlor-phenylamino)-benzyl]-4,4-dimethyl-2,5-dioxo-imidazolidin-1-yl}-2-trifluoromethyl-benzonitril (Molekulargewicht 512,12 ($C_{26}H_{20}ClF_3N_4O_2$); Retentionszeit $R_t$ = 2.39 min. [B]; MS (ESI): 513,13 ($MH^+$),
**34**, 4-{3-[2-(3-Trifluoromethyl-phenylamino)-benzyl]-4,4-dimethyl-2,5-dioxo-imidazolidin-1-yl}-2-trifluoromethyl-benzonitril (Molekulargewicht 546,14 ($C_{27}H_{20}F_6N_4O_2$); Retentionszeit $R_t$ = 2.39 min. [B]; MS (ESI): 547,18 ($MH^+$),
**44**, 4-{3-[2-(2-Fluoro-phenylamino)-benzyl]-4,4-dimethyl-2,5-dioxo-imidazolidin-1-yl}-2-trifluoromethyl-benzonitril (Molekulargewicht 496,15 ($C_{26}H_{20}F_4N_4O_2$); Retentionszeit $R_t$ = 2.28 min. [B]; MS (ESI): 497,18 ($MH^+$),
**67**, 4-{3-[2-(4-Methoxy-phenylamino)-benzyl]-4,4-dimethyl-2,5-dioxo-imidazolidin-1-yl}-2-trifluoromethyl-benzonitril (Molekulargewicht 508,17 ($C_{27}H_{23}F_3N_4O_3$); Retentionszeit $R_t$ = 2.29 min. [B]; MS (ESI): 509,22 ($MH^+$),
wurden wie die Verbindung des Beispiels **1** hergestellt mit dem Unterschied, dass in der dritten Stufe der Synthese statt Anilin

4-Fluoroanilin (für **2**),
4-Chloranilin (für **4**),
4-Trifluoromethylanilin (für **5**),
2,4-Difluoroanilin (für **11**),
4-Trifluoromethoxyanilin (für **12**),
2,4-Dichloranilin (für **13**),
3,4-Dichloanilin (für **20**),
2-Chloranilin (für **21**),
3-Chloranilin (für **22**),
3-Trifluoromethylanilin (für **34**),
2-Fluoroanilin (für **44**),
4-Methoxyanilin (für **67**),

eingesetzt wurde.

Beispiel **6**: 4-[3-(5-Methoxy-2-phenylamino-benzyl)-4,4-dimethyl-2,5-dioxo-imidazolidin-1-yl]-2-trifluoromethyl-benzonitril

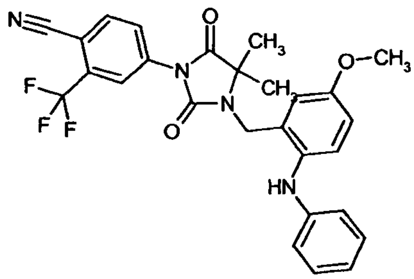

1) Herstellung von 4-[3-(2-Brom-5-methoxy-benzyl)-4,4-dimethyl-2,5-dioxo-imidazolidin-1-yl]-2-trifluoromethyl-benzonitril **6.2**:

1,5 g (5,05 mMol) der Verbindung **1.1** wurden mit 1,695 g (6,06 mMol) 2-Brom-5-methoxybenzylbromid und 2,06 d Cäsiumcarbonat in 10 ml Acetonitril wie im Beispiel **1**, Stufe 2 beschrieben umgesetzt und aufgearbeitet. Man erhielt 2,45 g (98% Ausbeute) 4-[3-(2-Brom-5-methoxy-benzyl)-4,4-dimethyl-2,5-dioxo-imidazolidin-1-yl]-2-trifluoromethyl-benzonitril (**6.2**). $^1$HNMR: 8.35, d, 1H; 8.25, s, 1H; 8.1, d, 1H; 7.55, d, 1H; 7.1, s,1H; 6.88, d, 1H; 4.6, s, 2H; 3.76, s, 3H; 1.42, s, 6H.

2) Herstellung von 4-[3-(5-Methoxy-2-phenylamino-benzyl)-4,4-dimethyl-2,5-dioxo-imidazolidin-1-yl]-2-trifluoromethyl-benzonitril **6**:

Die weitere Umsetzung mit Anilin zur Verbindung des Beispiels **6** erfolgte wie für Beispiel 1, Stufe 3 beschrieben. Man erhielt 4-[3-(5-Methoxy-2-phenyl-aminobenzyl)-4,4-dimethyl-2,5-dioxo-imidazolidin-1-yl]-2-trifluoromethyl-benzonitril **6**. (Molekulargewicht 508,17 ($C_{27}H_{23}F_3N_4O_3$); Retentionszeit $R_t$ = 2.19 min. [B]; MS (ESI): 509,14 ($MH^+$).

Die Verbindungen des Beispiels 7, 4-{3-[2-(4-Fluoro-phenylamino)-5-methoxy-benzyl]-4,4-dimethyl-2,5-dioxo-imidazolidin-1-yl}-2-trifluoromethyl-benzonitril (Molekulargewicht 526,16 ($C_{27}H_{22}F_4N_4O_3$); Retentionszeit $R_t$= 2.19 min. [B]; MS (ESI): 527,13 ($MH^+$), der Beispiele **8**, 4-{3-[5-Methoxy-2-(4-trifluoromethyl-phenylamino)-benzyl]-4,4-dimethyl-2,5-dioxo-imidazolidin-1-yl}-2-trifluoromethyl-benzonitril (Molekulargewicht 576,15 ($C_{28}H_{22}F_6N_4O_3$); Retentionszeit $R_t$ = 2.30 min. [B]; MS (ESI): 577,15 ($MH^+$),
**9**, 4-{3-[2-(4-Chlor-phenylamino)-5-methoxy-benzyl]-4,4-dimethyl-2,5-dioxo-imidazolidin-1-yl}-2-trifluoromethyl-benzonitril (Molekulargewicht 542,13 ($C_{27}H_{22}ClF_3N_4O_3$); Retentionszeit $R_t$ = 2.29 min. [B]; MS (ESI): 543,12 ($MH^+$),
**10**, 4-{3-[2-(4-Cyano-3-trifluoromethyl-phenylamino)-5-methoxy-benzyl]-4,4-dimethyl-2,5-dioxo-imidazolidin-1-yl}-2-trifluoromethyl-benzonitril (Molekulargewicht 601,15 ($C_{29}H_{21}F_6N_5O_3$); Retentionszeit $R_t$ = 2.12 min. [B]; MS ESI): 602,19 ($MH^+$) wurden wie die Verbindung des Beispiels **6** hergestellt mit dem Unterschied, dass in der zweiten Stufe der Synthese statt Anilin
4-Fluoroanilin (für **7**),
4-Trifluoromethylanilin (für **8**),
4-Chloranilin (für **9**),
4-Cyano-3-trifluoromethylanilin (für **10**)
eingesetzt wurde.

Beispiel **15**: 4-{3-[5-Fluoro-2-(4-fluoro-phenylamino)-benzyl]-4,4-dimethyl-2,5-dioxo-imidazolidin-1-yl}-2-trifluoromethyl-benzonitril

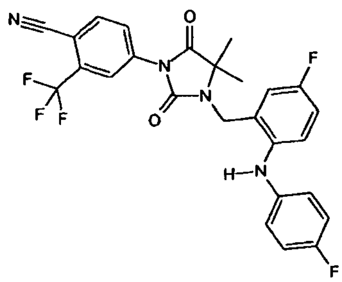

1) Herstellung von 4-[3-(2-Brom-5-fluoro-benzyl)-4,4-dimethyl-2,5-dioxo-imidazolidin-1-yl]-2-trifluoromethyl-benzonitril **15.2:**

Die Verbindung **15.2** wurde wie für Beispiel **6.2** beschrieben hergestellt, indem die Verbindung **1.1** mit 2-Brom-5-fluorobenzylbromid umgesetzt wurde. Man erhielt 4-[3-(2-Brom-5-fluoro-benzyl)-4,4-dimethyl-2,5-dioxo-imidazolidin-1-yl]-2-trifluoromethyl-benzonitril in einer Ausbeute von 95%. (Molekulargewicht 483,02 ($C_{20}H_{14}BrF_4N_3O_2$); Retentionszeit $R_t$ = 2.10 min. [B]; MS ESI): 484,26 ($MH^+$)

2) Herstellung von 4-{3-[5-Fluoro-2-(4-fluoro-phenylamino)-benzyl]-4,4-dimethyl-2,5-dioxo-imidazolidin-1-yl}-2-trifluoromethyl-benzonitril **15**:

Die weitere Umsetzung mit 4-Fluoroanilin zur Verbindung des Beispiels **15** erfolgte wie für Beispiel **1**, Stufe 3 beschrieben. Man erhielt 4-{3-[5-Fluoro-2-(4-fluorophenylamino)-benzyl]-4,4-dimethyl-2,5-dioxo-imidazolidin-1-yl}-2-trifluoromethylbenzonitril **15**. (Molekulargewicht 514,14 ($C_{26}H_{19}F_5N_4O_2$); Retentionszeit $R_t$ = 2.28 min. [B]; MS (ESI): 515,21 ($MH^+$).

Die Verbindungen des Beispiels **16**, 4-{3-[2-(4-Chlor-phenylamino)-5-fluoro-benzyl]-4,4-dimethyl-2,5-dioxo-imidazolidin-1-yl}-2-trifluoromethyl-benzonitril (Molekulargewicht 530,11 ($C_{26}H_{19}ClF_4N_4O_2$); Retentionszeit $R_t$ = 2.38 min. [B]; MS (ESI): 531,21 ($MH^+$), des Beispiels **18**, 4-[3-(5-Fluoro-2-phenylamino-benzyl)-4,4-dimethyl-2,5-dioxoimidazolidin-1-yl]-2-trifluoromethyl-benzonitril (Molekulargewicht 496,15 ($C_{26}H_{20}F_4N_4O_2$); Retentionszeit $R_t$ = 2.28 min. [B]; MS (ESI): 497,20 ($MH^+$)

wurden wie die Verbindung des Beispiels **15** hergestellt mit dem Unterschied, dass in der zweiten Stufe der Synthese statt 4-Fluoroanilin
4-Chloranilin (für **16**),
Anilin (für **18**)
eingesetzt wurde.

Beispiel **17**: 4-{3-[2-(4-Fluoro-phenylamino)-5-trifluoromethyl-benzyl]-4,4-dimethyl-2,5-dioxo-imidazolidin-1-yl}-2-trifluoromethyl-benzonitril

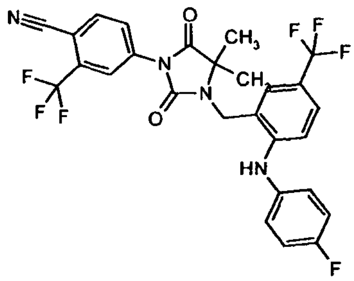

1) Herstellung von 4-[3-(2-Brom-5-trifluoromethyl-benzyl)-4,4-dimethyl-2,5-dioxo-imidazolidin-1-yl]-2-trifluoromethyl-benzonitril **17.2:**

Die Verbindung **17.2** wurde wie für Beispiel **6.2** beschrieben hergestellt, indem die Verbindung **1.1** mit 2-Brom-5-trifluoromethylbenzylbromid umgesetzt wurde. Man erhielt 4-[3-(2-Brom-5-trifluoromethyl-benzyl)-4,4-dimethyl-2,5-dioxo-imidazolidin-1-yl]-2-trifluoromethyl-benzonitril in einer Ausbeute von 96%. (Molekulargewicht 533,01 ($C_{21}H_{14}BrF_6N_3O_2$); Retentionszeit $R_t$ = 2.21 min. [B]; MS ESI): 534,14 ($MH^+$)

2) Herstellung von 4-{3-[2-(4-Fluoro-phenylamino)-5-trifluoromethyl-benzyl]-4,4-dimethyl-2,5-dioxo-imidazolidin-1-yl}-2-trifluoromethyl-benzonitril **17**:

Die weitere Umsetzung mit 4-Fluoroanilin zur Verbindung des Beispiels **17** erfolgte wie für Beispiel **1**, Stufe 3 beschrieben. Man erhielt 4-{3-[2-(4-Fluoro-phenylamino)-5-trifluoromethyl-benzyl]-4,4-dimethyl-2,5-dioxo-imidazolidin-1-yl}-2-trifluoromethyl-benzonitril **17**. (Molekulargewicht 564,13 ($C_{27}H_{19}F_7N_4O_2$);
Retentionszeit $R_t$ = 3.13 min. [C]; MS (ESI): 565,17 ($MH^+$).

Die Verbindung des Beispiels **19**, 4-{3-[2-(4-Chlor-phenylamino)-5-trifluoromethyl-benzyl]-4,4-dimethyl-2,5-dioxo-imidazolidin-1-yl}-2-trifluoromethyl-benzonitril (Molekulargewicht 580,11 ($C_{27}H_{19}ClF_6N_4O_2$); Retentionszeit $R_t$ = 3.21 min. [C]; MS (ESI): 581,18 ($MH^+$) wurde wie die Verbindung des Beispiels **17** hergestellt mit dem Unterschied, dass in der zweiten Stufe der Synthese statt 4-Fluoroanilin
4-Chloranilin (für **19**)
eingesetzt wurde.

Beispiel **23**: 3-(4-Fluoro-3-trifluoromethyl-phenyl)-5,5-dimethyl-1-(2-phenylaminobenzyl)-imidazolidin-2,4-dion

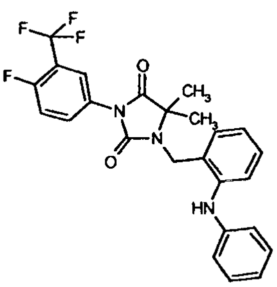

1) Herstellung von 3-(4-Fluoro-3-trifluoromethyl-phenyl)-5,5-dimethyl-imidazolidin-2,4-dion (**23.1**):

Die Verbindung **23.1** kann nach Verfahren "A" dargestellt werden. Dazu wurden 1,5 g (9,76 mMol) 2-Amino-2-methyl-propionsäuremethylester Hydrochlorid in 20 ml trockenem Tetrahydrofuran suspendiert, mit 1,38 ml (9,76 mMol) Triethylamin und 2 g (9,76 mMol) 1-Fluoro-4-isocyanato-2-trifluoromethyl-benzol versetzt. Die Mischung wurde 1 h bei 70°C gerührt; danach ließ man etwas abkühlen, fügte 10 ml konzentrierte Salzsäure zu und rührte für 2 h bei 70°C. Die abgekühlte Reaktionsmischung wurde mit Essigsäureethylester und Wasser versetzt; die organische Phase wurde abgetrennt, über Natriumsulfat getrocknet, filtriert und im Vakuum eingeengt. Der Rückstand wurde chromatographisch gereinigt (Methode [RP2]) und wurde nach Lösen in Essigsäureethylester, Trocknen der Lösung, Einengen im Vakuum und erneutem Lösen in Dichlormethan mit n-Heptan zur Kristallisation gebracht. Man erhielt 2,8 g 3-(4-Fluoro-3-trifluoromethyl-phenyl)-5,5-dimethyl-imidazolidine-2,4-dion (**23.1**) mit dem Schmelzpunkt 111 - 114°C. Molekulargewicht 290,06 ($C_{12}H_{10}F_4N_2O_2$); Retentionszeit $R_t$ = 1.55 min. [B]; MS (ESI): 291,27 ($MH^+$).

2) Herstellung von 1-(2-Brom-benzyl)-3-(4-fluoro-3-trifluoromethyl-phenyl)-5,5-dimethyl-imidazolidin-2,4-dion (**23.2**):

Die Verbindung **23.2** kann nach Verfahren "A" dargestellt werden. Dazu wurde die Verbindung **23.1** analog dem Vorgehen wie für die Herstellung von **1.2** beschrieben mit 2-Brombenzylbromid umgesetzt. Man erhielt 1-(2-Brom-benzyl)-3-(4-fluoro-3-trifluoromethyl-phenyl)-5,5-dimethyl-imidazolidin-2,4-dion in einer Ausbeute von 93%. Molekulargewicht 458,02 ($C_{19}H_{15}BrF_4N_2O_2$); Retentionszeit $R_t$ = 2.80 min. [C];
MS (ESI): 459,04 ($MH^+$).

3) Herstellung von 3-(4-Fluoro-3-trifluoromethyl-phenyl)-5,5-dimethyl-1-(2-phenylamino-benzyl)-imidazolidin-2,4-dion **23:**

Zur Herstellung der Verbindung des Beispiels **23** kann nach Verfahren "A" vorgegangen werden. Analog dem Vorgehen für das Beispiel **1**, Stufe 3 wurde die Verbindung **23.2** mit Anilin zur Reaktion gebracht. Man erhielt die Verbindung **23** in einer Ausbeute von 81%. Molekulargewicht 471,14 ($C_{25}H_{21}F_4N_3O_2$); Retentionszeit $A_t$ = 2.34 min. [B]; MS (ESI): 472,11 ($MH^+$).

Die Verbindungen des Beispiels **24**, 1-[2-(4-Fluoro-phenylamino)-benzyl]-3-(4-fluoro-3-trifluoromethyl-phenyl)-5,5-dimethyl-imidazolidin-2,4-dion, (Molekulargewicht 489,14 ($C_{25}H_{20}F_5N_3O_2$); Retentionszeit $R_t$ = 2.34 min. [B]; MS (ESI): 490,11 ($MH^+$);
der Beispiele **25**, 1-[2-(4-Chlor-phenylamino)-benzyl]-3-(4-fluoro-3-trifluoromethyl-phenyl)-5,5-dimethyl-imidazolidin-2,4-dion, (Molekulargewicht 505,11 ($C_{25}H_{20}ClF_4N_3O_2$); Retentionszeit $R_t$ = 2.45 min. [B]; MS (ESI): 506,11 ($MH^+$);
**52**, 1-[2-(2,4-Difluoro-phenylamino)-benzyl]-3-(4-fluoro-3-trifluoromethyl-phenyl)-5,5-dimethyl-imidazolidin-2,4-dion, (Molekulargewicht 507,13 ($C_{25}H_{19}F_6N_3O_2$); Retentionszeit $R_t$ = 2.34 min. [B]; MS (ESI): 507,98 ($MH^+$),
**134**, 4-{2-[3-(4-Fluoro-3-trifluoromethyl-phenyl)-5,5-dimethyl-2,4-dioxo-imidazolidin-1-ylmethyl]-phenylamino}-benzonitril, (Molekulargewicht 496,15 ($C_{26}H_{20}F_4N_4O_2$); Retentionszeit $R_t$ = 2.14 min. [B]; MS (ESI): 497,18 ($MH^+$)
wurden wie die Verbindung des Beispiels **23** hergestellt mit dem Unterschied, dass in der dritten Stufe der Synthese statt Anilin
4-Fluoranilin (für **24**),
4-Chloranilin (für **25**),
2,4-Difluoranilin (für **52**),
4-Aminobenzonitril (für **134**)
eingesetzt wurde.

Beispiel **28**: 4-[2,4-Dioxo-1-(2-phenylamino-benzyl)-1,3-diaza-spiro[4.5]dec-3-yl]-2-trifluoromethyl-benzonitril

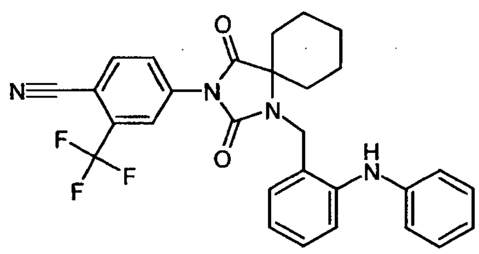

1) Herstellung von 4-(2,4-Dioxo-1,3-diaza-spiro[4.5]dec-3-yl)-2-trifluoromethyl-benzonitril (**28.1**):

Die Verbindung **28.1** kann nach Verfahren "A" dargestellt werden. Dazu wurden 5,3 ml Phosgen-Lösung (20% in Toluol) unter Argonatmosphäre vorgelegt. Bei 75°C wurde eine Lösung von 4-Cyano-3-trifluoromethyl-anilin in 15 ml trockenem Acetonitril langsam zugetropft. Nach beendeter Zugabe wurde die Mischung noch 90 min bei 75°C gerührt. Das Gemisch wurde im Vakuum eingeengt. Der Rückstand wurde danach mehrmals in Toluol aufgenommen und erneut im Vakuum eingeengt. Schließlich wurde der Rückstand in 15 ml trockenem Tetrahydrofuran gelöst, mit 0,72 g 1-Amino-1-cyclohexan-carbonsäure und tropfenweise mit 1,05 ml Triethylamin versetzt und die Mischung 2 h bei Raumtemperatur gerührt. Nach Stehen über Nacht bei Raumtemperatur wurde die Reaktionsmischung mit 5 ml konzentrierter Salzsäure versetzt und 2 h unter Rückfluß gerührt. Die abgekühlte Reaktionsmischung wurde mit gesättigter Natriumhydrogencarbonatlösung versetzt und mit Essigsäureethylester extrahiert. Die organische Phase wurde über Magnesiumsulfat getrocknet, filtriert und im Vakuum eingeengt. Man erhielt 0,62 g 4-(2,4-Dioxo-1,3-diaza-spiro[4.5]dec-3-yl)-2-trifluoromethyl-benzonitril (**28.1**). $^1$H MR: 9.21, s, 1H; 8.30, d, 1H; 8.19, s, 1H; 8.02, d, 1H; 1.8-1.5, m, 9H; 1.4-1.25, m, 1H.

2) Herstellung von 4-[1-(2-Brom-benzyl)-2,4-dioxo-1,3-diaza-spiro[4.5]dec-3-yl]-2-trifluoromethyl-benzonitril (**28.2**):

Die Verbindung **28.2** kann nach Verfahren "A" dargestellt werden. Dazu wurde die Verbindung **28.1** mit 2-Brombenzylbromid wie in Beispiel **1.2** beschrieben umgesetzt. Man erhielt die Verbindung **28.2** in einer Ausbeute von 98%. $^1$H NMR: 8.45, d, 1H; 8.26, s, 1H; 8.10, d, 1H; 7.7-7.25, m, 4H; 4.6, s, 2H; 2.1-1.55, m, 9H; 1.2, m, 1H.

3) Herstellung von 4-[2,4-Dioxo-1-(2-phenylamino-benzyl)-1,3-diaza-spiro[4.5]dec-3-yl]-2-trifluoromethyl-benzonitril (**28**):

Zur Herstellung der Verbindung des Beispiels **28** kann nach Verfahren "A" vorgegangen werden. Analog dem bei Beispiel 1.2 beschriebenen Verfahren wurden **28.2** und Anilin zur Reaktion gebracht. **28:** Molekulargewicht 518,19 ($C_{29}H_{25}F_3N_4O_2$);
Retentionszeit $R_t$ = 3.19 min. [C]; MS (ESI): 519,24 ($MH^+$).

Beispiel **29**: 4-[4,4-Dimethyl-2,5-dioxo-3-(3-phenylamino-benzyl)-imidazolidin-1-yl]-2-trifluoromethyl-benzonitril

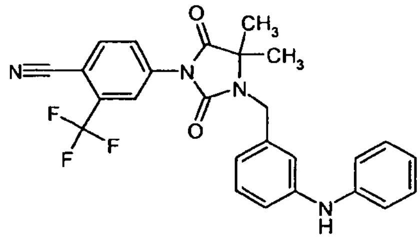

Die Verbindung des Beispiels **29** wurde analog der Vorgehensweise für die Verbindung des Beispiels 1 erhalten, indem die Verbindung **1.1** anstatt mit 2-Brombenzylbromid mit 3-Brombenzylbromid zur Verbindung **29.2** umgesetzt wurde ($^1$H NMR: 8.35, d, 1H; 8.25, s, 1H; 8.10, d, 1H; 7.7, s, 1H; 7.5, m, 2H; 7.3, t, 1H; 4.6, s, 2H; 1.4, s, 6H). **29.2** wurde in einem weiteren Schritt wie in Beispiel 1, Stufe 3 beschrieben mit Anilin zur Verbindung des Beispiels **29** umgesetzt. Molekulargewicht 478,16 ($C_{26}H_{21}F_3N_4O_2$); Retentionszeit $R_t$ = 2.53 min. [B]; MS (ESI): 479,48 ($MH^+$).

Beispiel 30: 4-[4,4-Dimethyl-2,5-dioxo-3-(4-phenylamino-benzyl)-imidazolidin-1-yl]-2-trifluoromethyl-benzonitril

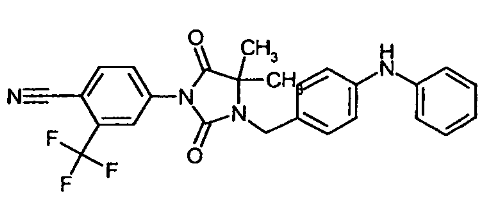

Die Verbindung des Beispiels 30 wurde analog der Vorgehensweise für die Verbindung des Beispiels 1 erhalten, indem die Verbindung 1.1 anstatt mit 2-Brombenzylbromid mit 4-Brombenzylbromid zur Verbindung 30.2 umgesetzt wurde ($^1$H NMR: 8.35, d, 1H; 8.25, s, 1H; 8.10, d, 1H; 7.55, d, 2H; 7.4, d, 2H; 4.6, s, 2H; 1.4, s, 6H). **30.2** wurde in einem weiteren Schritt wie in Beispiel **1**, Stufe 3 beschrieben mit Anilin zur Verbindung des Beispiels **30** umgesetzt. Molekulargewicht 478,16 ($C_{26}H_{21}F_3N_4O_2$); Retentionszeit $R_t$ = 2.54 min. [B]; MS (ESI): 479,41 ($MH^+$).

Beispiel **35**: 4-{3-[2,4-Dichlor-6-(4-fluoro-phenylamino)-benzyl]-4,4-dimethyl-2,5-dioxo-imidazolidin-1-yl}-2-trifluoromethyl-benzonitril

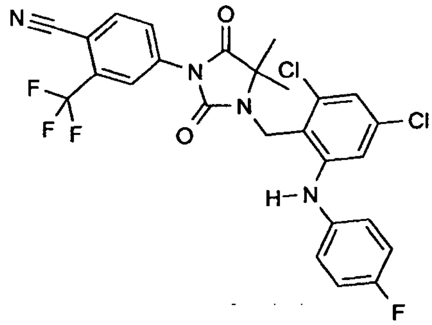

Herstellung von 2-Brom-4,6-dichlorbenzylbromid **35.3:**

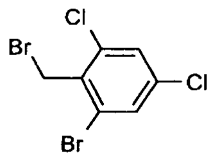

a) 2-Brom-4,6-dichlorbenzoesäure **35.5:**
Zu einer Suspension von 6,5 g Kupfer-II-bromid in 100 ml trockenem Acetonitril wurden bei 0°C 4,85 ml tert.-Butylnitrit zugetropft. Zu dieser dunkelgrünen Lösung wurden innerhalb von 5 Minuten in Portionen 5 g 2-Amino-4,6-dichlorbenzoesäure zugegeben. Die Mischung wurde 2 h bei 0°C gerührt; danach ließ man auf Raumtemperatur erwärmen und rührte über Nacht weiter. Im Vakuum wurde die Mischung auf etwa das halbe Volumen eingeengt; man fügte 70 ml 1 N Salzsäure zu und extrahierte die Mischung mit 60 ml Diisopropylether. Die organische Phase wurde mit 70 ml 2 N Natronlauge versetzt. Die Wasserphase wurde abgetrennt und mit Salzsäure auf einen pH-Wert von 2 eingestellt. Die wässrige Phase wurde mit Diisopropylether ausgeschüttelt, die organische Phase über Magnesiumsulfat getrocknet, filtriert und im Vakuum eingeengt. Man erhielt 2-Brom-4,6-dichlorbenzoesäure **35.5.** $^1$H NMR: 14.2, s, 1H; 7.9, d, 1H; 7.8, d, 1 H.
b) (2-Brom-4,6-dichlor-phenyl)-methanol **35.4:**
2,5 g der Säure **35.5** wurden in 25 ml trockenem Tetrahydrofuran gelöst und bei 0° C unter Rühren tropfenweise mit 9,26 ml einer 1 molaren Lösung von Lithiumaluminiumhydrid in Tetrahydrofuran versetzt. Die Reaktionsmischung wurde noch 30 min bei 0° C und 2 h bei Raumtemperatur gerührt. Zur Aufarbeitung wurde die Mischung unter Kühlung mit 2,5 N Schwefelsäure auf pH 2 eingestellt und mit Essigsäureethylester und Wasser versetzt. Die organische Phase wurde abgetrennt, über Magnesiumsulfat getrocknet und im Vakuum eingeengt. Man erhielt (2-Brom-4,6-dichlor-phenyl)-methanol **35.4**, welches ohne weitere Reinigung in die nächste Stufe eingesetzt wurde.
c) 2-Brom-4,6-dichlorbenzylbromid **35.3:**
3,1 g des Benzylalkohols **35.4** wurden in 40 ml trockenem Dichlormethan gelöst und bei 5° C tropfenweise mit einer Lösung von 0,455 ml Phosphortribromid in 10 ml Dichlormethan versetzt. Die Reaktionsmischung rührte über Nacht und wurde anschließend mit 5 ml einer gesättigten wässrigen Natriumcarbonatlösung neutralisiert. Die organische Phase wurde abgetrennt, über Magnesiumsulfat getrocknet, filtriert und im Vakuum eingeengt. Der Rückstand wurde chromatographisch über Kieselgel mit n-Heptan als Laufmittel gereinigt. Man erhielt 2-Brom-4,6-dichlorbenzylbromid **35.3**. $^1$H NMR: 7.9, s, 1H; 7.8, s, 1H; 4.75, s, 2H.

1) Herstellung von 4-[3-(2-Brom-4,6-dichlor-benzyl)-4,4-dimethyl-2,5-dioxo-imidazolidin-1-yl]-2-trifluoromethyl-benzonitril **35.2:**

Die Verbindung **35.2** wurde wie für Beispiel **6.2** beschrieben hergestellt, indem die Verbindung **1.1** mit 2-Brom-4,6-dichlorbenzylbromid **35.3** umgesetzt wurde. Man erhielt 4-[3-(2-Brom-4,6-dichlor-benzyl)-4,4-dimethyl-2,5-dioxo-imidazolidin-1-yl]-2-trifluoromethyl-benzonitril in einer Ausbeute von.83%. $^1H$ NMR: 8.35, d, 1H; 8.2, s, 1H; 8.05, d, 1H; 7.9, s, 1H; 7.8, s, 1H; 4.9, s, 2H; 1.35, s, 6H.

2) Herstellung von 4-{3-[2,4-Dichlor-6-(4-fluoro-phenylamino)-benzyl]-4,4-dimethyl-2,5-dioxo-imidazolidin-1-yl}-2-trifluoromethyl-benzonitril **35**:

Die weitere Umsetzung mit 4-Fluoroanilin zur Verbindung des Beispiels **35** erfolgte wie für Beispiel **1**, Stufe 3 beschrieben. Man erhielt 4-{3-[2,4-Dichlor-6-(4-fluorophenylamino)-benzyl]-4,4-dimethyl-2,5-dioxo-imidazolidin-1-yl}-2-trifluoromethyl-benzonitril **35**. (Molekulargewicht 564,07 ($C_{26}H_{18}Cl_2F_4N_4O_2$); Retentionszeit $R_t$ = 2.98 min. [B]; MS (ESI): 606,23 ($MH^+$ + $CH_3CN$).

Die Verbindung des Beispiels **36**, 4-{3-[2,4-Dichlor-6-(2,4-difluoro-phenylamino)-benzyl]-4,4-dimethyl-2,5-dioxo-imidazolidin-1-yl}-2-trifluoromethyl-benzonitril (Molekulargewicht 582,06 ($C_{26}H_{17}Cl_2F_5N_4O_2$); Retentionszeit $R_t$ = 2.98 min. [B]; MS (ESI): 583,32 ($MH^+$), des Beispiels **37**, 4-{3-[2,4-Dichlor-6-(4-chlor-phenylamino)-benzyl]-4,4-dimethyl-2,5-dioxo-imidazolidin-1-yl}-2-trifluoromethyl-benzonitril (Molekulargewicht 580,04 ($C_{26}H_{18}Cl_3F_3N_4O_2$); Retentionszeit $R_t$ = 3.09 min. [B]; MS (ESI): 581,32 ($MH^+$), des Beispiels **104**, 4-{3,5-Dichlor-2-[3-(4-cyano-3-trifluoromethyl-phenyl)-5,5-dimethyl-2,4-dioxo-imidazolidin-1-ylmethyl]-phenylamino}-benzoesäuremethylester (Molekulargewicht 604,08 ($C_{28}H_{21}Cl_2F_3N_4O_4$); Retentionszeit $R_t$ = 2.52 min. [B]; MS (ESI): 605,17 ($MH^+$),
wurden wie die Verbindung des Beispiels **35** hergestellt mit dem Unterschied, dass in der zweiten Stufe der Synthese statt 4-Fluoroanilin
2,4-Difluoroanilin (für **36**),
4-Chloranilin (für **37**),
4-Aminobenzoesäuremethylester (für **104**)
eingesetzt wurde.

Beispiel **38:** 4-{3-[3,5-Dichlor-2-(4-fluoro-phenylamino)-benzyl]-4,4-dimethyl-2,5-dioxo-imidazolidin-1-yl}-2-trifluoromethyl-benzonitril

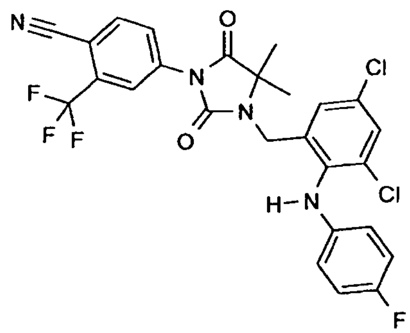

Herstellung von 2-Brom-3,5-dichlorbenzylbromid 38.3:

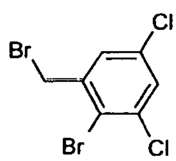

2-Brom-3,5-dichlorbenzylbromid **38.3** wurde auf demselben Wege wie 2-Brom-4,6-dichlorbenzylbromid **35.3** aber ausgehend von 3,5-Dichloranthranilsäure über 2-Brom-3,5-dichlorbenzoesäure **38.5** und (2-Brom-3,5-dichlor-phenyl)-methanol **38.4** hergestellt. 2-Brom-3,5-dichlorbenzylbromid **38.3:** $^1$H NMR: 7.8, s, 1H; 7.55, s, 1H; 4.75, s, 2H.

1) Herstellung von 4-[3-(2-Brom-3,5-dichlor-benzyl)-4,4-dimethyl-2,5-dioxo-imidazolidin-1-yl]-2-trifluoromethyl-benzonitril **38.2:**

Die Verbindung **38.2** wurde wie für Beispiel **6.2** beschrieben hergestellt, indem die Verbindung **1.1** mit 2-Brom-3,5-dichlorbenzylbromid umgesetzt wurde. Man erhielt 4-[3-(2-Brom-3,5-dichlor-benzyl)-4,4-dimethyl-2,5-dioxo-imidazolidin-1-yl]-2-trifluoromethyl-benzonitril in einer Ausbeute von 55%. $^1$H NMR: 8.35, d, 1H; 8.25, s, 1H; 8.1, d, 1H; 7.7, s, 1H; 7.65, s, 1H; 4.65, s, 2H; 1.45, s, 6H.

2) Herstellung von 4-{3-[3,5-Dichlor-2-(4-fluoro-phenylamino)-benzyl]-4,4-dimethyl-2,5-dioxo-imidazolidin-1-yl}-2-trifluoromethyl-benzonitril **38**:

Die weitere Umsetzung mit 4-Fluoroanilin zur Verbindung des Beispiels **38** erfolgte wie für Beispiel **1**, Stufe 3 beschrieben. Man erhielt 4-{3-[3,5-Dichlor-2-(4-fluorophenylamino)-benzyl]-4,4-dimethyl-2,5-dioxo-imidazolidin-1-yl}-2-trifluoromethyl-benzonitril **38.** (Molekulargewicht 564,07 ($C_{26}H_{18}Cl_2F_4N_4O_2$); Retentionszeit $R_t$ = 2.37 min. [B]; MS (ESI): 565,17 ($MH^+$).

Die Verbindung des Beispiels **39**, 4-{3-[3,5-Dichlor-6-(2,4-difluoro-phenylamino)-benzyl]-4,4-dimethyl-2,5-dioxo-imidazolidin-1-yl}-2-trifluoromethyl-benzonitril (Molekulargewicht 582,06 ($C_{26}H_{17}Cl_2F_5N_4O_2$); Retentionszeit $R_t$ = 2.42 min. [B]; MS (ESI): 583,16 ($MH^+$), des Beispiels **40**, 4-{3-[3,5-Dichlor-6-(4-chlor-phenylamino)-benzyl]-4,4-dimethyl-2,5-dioxo-imidazolidin-1-yl}-2-trifluoromethyl-benzonitril (Molekulargewicht 580,04 ($C_{26}H_{18}Cl_3F_3N_4O_2$); Retentionszeit $R_t$ = 2.47 min. [B]; MS (ESI): 581,14 ($MH^+$),
wurden wie die Verbindung des Beispiels **38** hergestellt mit dem Unterschied, dass in der zweiten Stufe der Synthese statt 4-Fluoroanilin
2,4-Difluoroanilin (für **39**),
4-Chloranilin (für **40**)
eingesetzt wurde.

Beispiel **41**: 4-{3-[5-Chlor-2-(4-fluoro-phenylamino)-benzyl]-4,4-dimethyl-2,5-dioxo-imidazolidin-1-yl}-2-trifluoromethyl-benzonitril

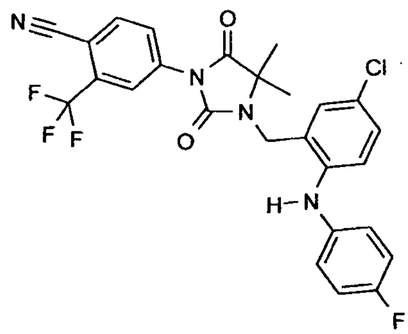

1) Herstellung von 4-[3-(2-Brom-5-chlor-benzyl)-4,4-dimethyl-2,5-dioxo-imidazolidin-1-yl]-2-trifluoromethyl-benzonitril **41.2:**

Die Verbindung **41.2** wurde wie für Beispiel **6.2** beschrieben hergestellt, indem die Verbindung **1.1** mit 2-Brom-5-chlorbenzylbromid (hergestellt aus 2-Brom-5-chlorbenzoesäure durch Reduktion mit Lithiumaluminiumhydrid und Umsetzung des so gewonnenen Benzylalkohols mit Phosphortribromid) umgesetzt wurde. Man erhielt 4-[3-(2-Brom-5-chlor-benzyl)-4,4-dimethyl-2,5-dioxo-imidazolidin-1-yl]-2-trifluoromethyl-benzonitril in einer Ausbeute von 64%. $^1$H NMR: 8.35, d, 1H; 8.25, s, 1H; 8.1, d, 1H; 7.65, m, 2H; 7.33, d, 1H; 4.6, s, 2H; 1.45, s, 6H.

2) Herstellung von 4-{3-[5-Chlor-2-(4-fluoro-phenylamino)-benzyl]-4,4-dimethyl-2,5-dioxo-imidazolidin-1-yl}-2-trifluoromethyl-benzonitril **41:**

Die weitere Umsetzung mit 4-Fluoroanilin zur Verbindung des Beispiels **41** erfolgte wie für Beispiel **1**, Stufe 3

beschrieben. Man erhielt 4-{3-[5-Chlor-2-(4-fluorophenylamino)-benzyl]-4,4-dimethyl-2,5-dioxo-imidazolidin-1-yl}-2-trifluoromethylbenzonitril **41.** (Molekulargewicht 530,11 ($C_{26}H_{19}ClF_4N_4O_2$); Retentionszeit $R_t$ = 2.37 min. [B]; MS (ESI): 531,17 ($MH^+$).

Die Verbindung des Beispiels **42**, 4-{3-[5-Chlor-6-(2,4-difluoro-phenylamino)-benzyl]-4,4-dimethyl-2,5-dioxo-imidazolidin-1-yl}-2-trifluoromethyl-benzonitril (Molekulargewicht 548,10 ($C_{26}H_{18}ClF_5N_4O_2$); Retentionszeit $R_t$ = 2.38 min. [B]; MS (ESI): 549,17 ($MH^+$), des Beispiels **43**, 4-{3-[5-Chlor-6-(4-chlor-phenylamino)-benzyl]-4,4-dimethyl-2,5-dioxo-imidazolidin-1-yl}-2-trifluoromethyl-benzonitril (Molekulargewicht 546,08 ($C_{26}H_{19}C1_2F_3N_40_2$); Retentionszeit $R_t$ = 2.48 min. [B]; MS (ESI): 547,15 ($MH^+$),
wurden wie die Verbindung des Beispiels **41** hergestellt mit dem Unterschied, dass in der zweiten Stufe der Synthese statt 4-Fluoroanilin
2,4-Difluoroanilin (für **42**),
4-Chloranilin (für **43**)
eingesetzt wurde.

Beispiel **48**: 4-{3-[2,5-Bis-(4-chlor-phenylamino)-benzyl]-4,4-dimethyl-2,5-dioxo-imidazolidin-1-yl}-2-trifluoromethyl-benzonitril

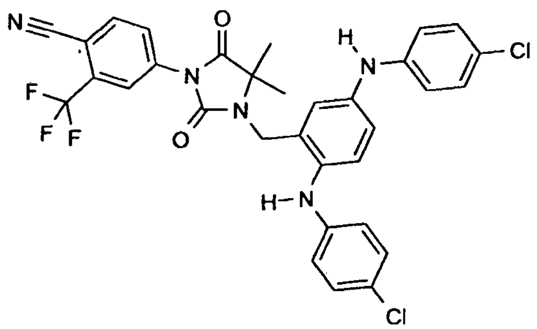

Bei der Herstellung der Verbindung des Beispiels **43** (40% Ausbeute) fiel als weiteres Produkt die Verbindung des Beispiels **48** in 23% Ausbeute an. Molekulargewicht 637,12 ($C_{32}H_{24}Cl_2F_3N_5O_2$); Retentionszeit $R_t$ = 2.58 min. [B]; MS (ESI): 638,20 ($MH^+$).

Beispiel **55**: 4-{3-[2-(4-Fluoro-phenylamino)-4-trifluoromethyl-benzyl]-4,4-dimethyl-2,5-dioxo-imidazolidin-1-yl}-2-trifluoromethyl-benzonitril

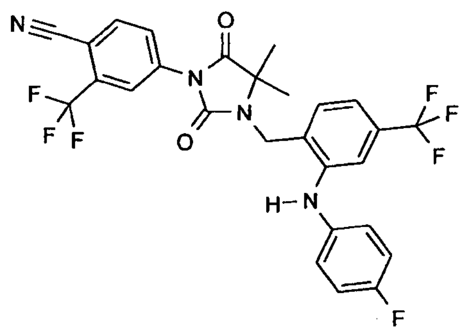

1) Herstellung von 4-[3-(2-Brom-4-trifluoromethyl-benzyl)-4,4-dimethyl-2,5-dioxo-imidazolidin-1-yl]-2-trifluoromethyl-benzonitril **55.2:**

Die Verbindung **55.2** wurde wie für Beispiel **6.2** beschrieben hergestellt, indem die Verbindung **1.1** mit 2-Brom-4-trifluoromethylbenzylbromid umgesetzt wurde. Man erhielt 4-[3-(2-Brom-4-trifluoromethyl-benzyl)-4,4-dimethyl-2,5-dioxo-imidazolidin-1-yl]-2-trifluoromethyl-benzonitril in einer Ausbeute von 78%. $^1$H NMR: 8.36, d, 1H; 8.25, s, 1H; 8.1, d,

1H; 8.05, s, 1H; 7.85, d, 1H; 7.73, d, 1H; 4.7, s, 2H; 1.48, s, 6H.

2) Herstellung von 4-{3-[2-(4-Fluoro-phenylamino)-4-trifluoromethyl-benzyl]-4,4-dimethyl-2,5-dioxo-imidazolidin-1-yl}-2-trifluoromethyl-benzonitril **55:**

Die weitere Umsetzung mit 4-Fluoroanilin zur Verbindung des Beispiels **55** erfolgte wie für Beispiel **1**, Stufe 3 beschrieben. Man erhielt 4-{3-[2-(4-Fluoro-phenylamino)-4-trifluoromethyl-benzyl]-4,4-dimethyl-2,5-dioxo-imidazolidin-1-yl}-2-trifluoromethyl-benzonitril **55.** Molekulargewicht 564,13 ($C_{27}H_{19}F_7N_4O_2$);
Retentionszeit $R_t$ = 2.40 min. [B]; MS (ESI): 565,09 ($MH^+$).

Die Verbindung des Beispiels **56**, 4-{3-[2-(2,4-Difluoro-phenylamino)-4-trifluoro-methyl-benzyl]-4,4-dimethyl-2,5-dioxo-imidazolidin-1-yl}-2-trifluoromethyl-benzonitril (Molekulargewicht 582,13 ($C_{27}H_{18}F_8N_4O_2$); Retentionszeit $R_t$ = 2.40 min. [B]; MS (ESI): 583,07 ($MH^+$)),
des Beispiels **57**, 4-{3-[2-(4-Chlor-phenylamino)-4-trifluoromethyl-benzyl]-4,4-dimethyl-2,5-dioxo-imidazolidin-1-yl}-2-trifluoromethyl-benzonitril (Molekulargewicht 580,11 ($C_{27}H_{19}C1F_6N_4O_2$); Retentionszeit $R_t$ = 2.51 min. [B]; MS (ESI): 581,06 ($MH^+$)),
des Beispiels **106**, 4-{2-[3-(4-Cyano-3-trifluoromethyl-phenyl)-5,5-dimethyl-2,4-dioxo-imidazolidin-1-ylmethyl]-5-trifluoromethyl-phenylamino}-benzoesäuremethylester (Molekulargewicht 604,15 ($C_{29}H_{22}F_6N_4O_4$); Retentionszeit $R_t$ = 2.31 min. [B]; MS (ESI): 605,21 ($MH^+$)),
des Beispiels **122**, 4-{2-[3-(4-Cyano-3-trifluoromethyl-phenyl)-5,5-dimethyl-2,4-dioxo-imidazolidin-1-ylmethyl]-5-trifluoromethyl-phenylamino}-benzoesäure tert. butyl ester (Molekulargewicht 646,20 ($C_{32}H_{28}F_6N_4O_4$); Retentionszeit $R_t$ = 2.50 min. [B]; MS (ESI): 591,14 ($MH^+$-$C_4H_8$)),
des Beispiels **165**, 4-{2-[3-(4-Cyano-3-trifluoromethyl-phenyl)-5,5-dimethyl-2,4-dioxo-imidazolidin-1-ylmethyl]-5-trifluoromethyl-phenylamino}-benzoesäureethylester (Molekulargewicht 618,17 ($C_{30}H_{24}F_6N_4O_4$); Retentionszeit $R_t$ = 2.38 min. [B]; MS (ESI): 619,15 ($MH^+$))
wurden wie die Verbindung des Beispiels **55** hergestellt mit dem Unterschied, dass in der zweiten Stufe der Synthese statt 4-Fluoroanilin
2,4-Difluoroanilin (für **56**),
4-Chloranilin (für **57**),
4-Aminobenzoesäuremethylester (für **106**),
4-Aminobenzoesäure tert. butyl ester (für **122**),
4-Aminobenzoesäureethylester (für **165**)
eingesetzt wurde.

Beispiel **58**: 2-{2-[3-(4-Cyano-3-trifluoromethyl-phenyl)-5,5-dimethyl-2,4-dioxo-imidazolidin-1-ylmethyl]-phenylamino}-5-fluoro-benzamid

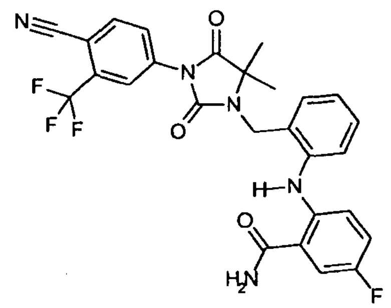

150 mg (0.288 mmol) der Verbindung des Beispiels 53 wurden in 1,5 ml Dichlormethan gelöst. Bei 5° C wurden 19,5 mg Tetrabutyl-ammoniumhydrogensulfat, 180 mg Natriumhydroxid in 0,133 ml Wasser und 0,148 ml Wasserstoffperoxid (30%ig) zugegeben. Die Mischung wurde auf Raumtemperatur erwärmt und 2 h gerührt. Danach wurden 148 µl Wasserstoffperoxid nachgegeben und zwei weitere Stunde gerührt. Danach wurden nochmals 148 µl Wasserstoffperoxid nachgegeben und 2 weitere Stunden gerührt. Zur Aufarbeitung wurde etwas Wasser und Dichlormethan zur Reaktionsmischung zugegeben, die organische Phase wurde abgetrennt und chromatographisch gereinigt (Methode [RP1]). Man erhielt 2-{2-[3-(4-Cyano-3-trifluoromethyl-phenyl)-5,5-dimethyl-2,4-dioxo-imidazolidin-1-ylmethyl]-phenylamino}-5-fluoro-benzamid **58** in einer Ausbeute von 52%. Molekulargewicht 539,15 ($C_{27}H_{21}F_4N_5O_3$); Retentionszeit $R_t$ = 2.04 min. [B];

MS (ESI): 540,06 ($MH^+$).

Die Verbindung des Beispiels **54** 4-{3-[2-(4-Cyano-phenylamino)-benzyl]-4,4-dimethyl-2,5-dioxo-imidazolidin-1-yl}-2-trifluoromethyl-benzamid wurde analog unter Einsatz der Verbindung **33** hergestellt. Molekulargewicht 521,16 ($C_{27}H_{22}F_3N_5O_3$); Retentionszeit $R_t$ = 1.72 min. [B]; MS (ESI): 522,10 ($MH^+$).

Beispiel **59:** 4-{4,4-Dimethyl-3-[2-(methyl-phenyl-amino)-benzyl]-2,5-dioxo-imidazolidin-1-yl}-2-trifluoromethyl-benzonitril

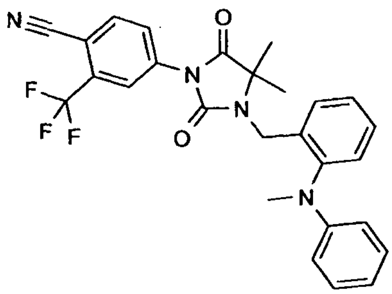

53 mg der Verbindung des Beispiels 1 wurden in 1,1 ml Acetonitril gelöst. Zu dieser Lösung wurden 10,4 mg Natriumcyanoborhydrid zugefügt. Anschließend wurden 0,18 ml einer 37%igen Formalinlösung und 0,05 ml Eisessig zugetropft. Die Mischung wurde 2 h bei Raumtemperatur gerührt; danach und nochmals 2 h später wurden die gleichen Mengen Natriumborcyanhydrid und Formalinlösung nochmals zugetropft. Zur Aufarbeitung würde über eine Kieselgelkartusche filtriert und das Filtrat im Vakuum eingeengt und chromatographisch gereinigt (Methode [RP1]). Man erhielt 4-{4,4-Dimethyl-3-[2-(methyl-phenyl-amino)-benzyl]-2,5-dioxo-imidazolidin-1-yl}-2-trifluoromethyl-benzonitril **59** in einer Ausbeute von 60%. Molekulargewicht 492,17 ($C_{27}H_{23}F_3N_4O_2$); Retentionszeit $R_t$ = 2.33 min. [B]; MS (ESI): 493,05 ($MH^+$).

Beispiel **62**: 4-{4,4-{2-[3-(4-Cyano-3-trifluoromethyl-phenyl)-5,5-dimethyl-2,4-dioxo-imidazolidin-1-ylmethyl]-phenylamino }-benzoesäure

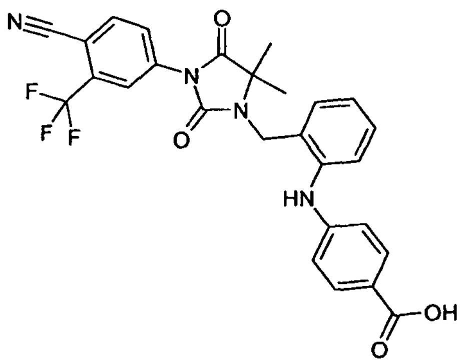

64 mg der Verbindung des Beispiels **60** wurden in 0,32 ml Dioxan (warm) gelöst, mit 0,36 ml konzentrierter Salzsäure versetzt und 1 h bei 75° C gerührt. Zur Aufarbeitung wurde die Reaktionsmischung mit etwas Acetonitril versetzt und chromatographisch gereinigt (Methode [RP1]). Nach Gefriertrocknung erhielt man 4-{4,4-{2-[3-(4-Cyano-3-trifluoromethyl-phenyl)-5,5-dimethyl-2,4-dioxo-imidazolidin-1-ylmethyl]-phenylamino}-benzoesäure **62**.
Molekulargewicht 522,15 ($C_{27}H_{21}F_3N_4O_4$); Retentionszeit $R_t$ = 1.88 min. [B];
MS (ESI): 523,16 ($MH^+$).

Auf die gleiche Weise wurden die Verbindungen **80, 92, 118, 119, 130, 131,132, 133, 156, 159, 166 168 169 227 228 209, 210, 212** aus ihren Estern **72 82 115 117 124 123 126 125 192 194, 195,193, 196, 225, 226, 202, 203, 211** gewonnen:

**80:** 4-{2-[3-(4-Cyano-3-trifluoromethyl-phenyl)-5,5-dimethyl-2,4-dioxo-imidazolidin-1-ylmethyl]-phenylamino}-3-fluorobenzoesäure; $^1H$ NMR: 12.6, s, 1H; 8.32, d, 1H; 8.2, s (breit), 2H; 8.03, d, 1H; 7.6, m, 3H; 7.35, t, 1H; 7.25, m, 2H; 6.7,

t, 1H; 4.58, s, 2H; 1.35, s, 6H.

**92**: (4-{2-[3-(4-Cyano-3-trifluoromethyl-phenyl)-5,5-dimethyl-2,4-dioxo-imidazolidin-1-ylmethyl]-phenylamino}-phenyl)-essigsäure; Molekulargewicht 536,16 ($C_{28}H_{23}F_3N_4O_4$); Retentionszeit $R_t$ = 1.95 min. [B]; MS (ESI): 537,26 ($MH^+$).

**118:** 4-{2-[5,5-Dimethyl-3-(4-methylsulfanyl-3-trifluoromethyl-phenyl)-2,4-dioxo-imidazolidin-1-ylmethyl]-phenylamino}-benzoesäure; Molekulargewicht 543,14 ($C_{27}H_{24}F_3N_3O_4S$); Retentionszeit $R_t$ = 2.38 min. [C]; MS (ESI): 544,30 ($MH^+$).

**119**: 4-{2-[3-(4-Methansulfonyl-3-trifluoromethyl-phenyl)-5,5-dimethyl-2,4-dioxo-imidazolidin-1-ylmethyl]-phenylamino}-benzoesäure; Molekulargewicht 575,13 ($C_{27}H_{24}F_3N_3O_6S$); Retentionszeit $R_t$ = 2.17 min. [C]; MS (ESI): 576,13 ($MH^+$).

**130**: 4-{2-[3-(4-Cyano-3-trifluoromethyl-phenyl)-5,5-dimethyl-2,4-dioxo-imidazolidin-1-ylmethyl]-5-methoxy-phenylamino}-benzoesäure; Molekulargewicht 552,16 ($C_{28}H_{23}F_3N_4O_5$); Retentionszeit $R_t$ = 1.91 min. [B]; MS (ESI): 553,15 ($MH^+$).

**131**: 4-{5-Chlor-2-[3-(4-cyano-3-trifluoromethyl-phenyl)-5,5-dimethyl-2,4-dioxo-imidazolidin-1-ylmethyl]-phenylamino}-benzoesäure; Molekulargewicht 556,11 ($C_{27}H_{20}ClF_3N_4O_4$); Retentionszeit $R_t$ = 2.00 min. [B]; MS (ESI): 556,11 ($MH^+$).

**132**: 4-{5-Cyano-2-[3-(4-cyano-3-trifluoromethyl-phenyl)-5,5-dimethyl-2,4-dioxo-imidazolidin-1-ylmethyl]-phenylamino}-benzoesäure; Molekulargewicht 547,14 ($C_{28}H_{20}F_3N_5O_4$); Retentionszeit $R_t$ =1.86 min. [B]; MS (ESI): 548,14 ($MH^+$).

**133**: 4-{2-[3-(4-Cyano-3-trifluoromethyl-phenyl)-5,5-dimethyl-2,4-dioxo-imidazolidin-1-ylmethyl]-5-fluoro-phenylamino}-benzoesäure; Molekulargewicht 540,14 ($C_{27}H_{20}F_4N_4O_4$); Retentionszeit $R_t$ = 1.96 min. [B]; MS (ESI): 541,14 ($MH^+$).

**156**: 4-{5-Fluoro-2-[3-(4-fluoro-3-trifluoromethyl-phenyl)-5,5-dimethyl-2,4-dioxo-imidazolidin-1-ylmethyl]-phenylamino}-benzoesäure; Molekulargewicht 533,13 ($C_{26}H_{20}F_5N_3O_4$); Retentionszeit $R_t$ = 2.00 min. [B]; MS (ESI): 534,25 ($MH^+$).

**159**: 4-{2-[3-(4-Cyano-3-cyclopropyl-phenyl)-5,5-dimethyl-2,4-dioxo-imidazolidin-1-ylmethyl]-5-fluoro-phenylamino}-benzoesäure; Molekulargewicht 512,18 ($C_{29}H_{25}FN_4O_4$); Retentionszeit $R_t$ =1.94 min. [B]; MS (ESI): 513,24 ($MH^+$).

**166**: 4-{2-[3-(4-Cyano-3-methyl-phenyl)-5,5-dimethyl-2,4-dioxo-imidazolidin-1-ylmethyl]-5-fluoro-phenylamino}-benzoesäure; Molekulargewicht 486,17 ($C_{27}H_{23}FN_4O_4$); Retentionszeit $R_t$ = 1.85 min. [B]; MS (ESI): 487,31 ($MH^+$).

**168**: 4-{2-[3-(2-tert-Butyl-pyridin-4-yl)-5,5-dimethyl-2,4-dioxo-imidazolidin-1-ylmethyl]-5-fluoro-phenylamino}-benzoesäure x HCl; Molekulargewicht (freie Säure) 504,21 ($C_{28}H_{29}FN_4O_4$); Retentionszeit $R_t$ = 1.50 min. [B]; MS (ESI): 505,18 ($MH^+$).

**169**: 4-{2-[3-(3-tert-Butyl-4-cyano-phenyl)-5,5-dimethyl-2,4-dioxo-imidazolidin-1-ylmethyl]-5-fluoro-phenylamino}-benzoesäure; Molekulargewicht 528,21 ($C_{30}H_{29}FN_4O_4$); Retentionszeit $R_t$ = 2.06 min. [B]; MS (ESI): 529,16 ($MH^+$).

**227**: 4-{2-[3-(6-Cyano-biphenyl-3-yl)-5,5-dimethyl-2,4-dioxo-imidazolidin-1-ylmethyl]-5-fluoro-phenylamino}-benzoesäure; Molekulargewicht 548,18 ($C_{32}H_{25}FN_4O_4$); Retentionszeit $R_t$=1.96 min. [B]; MS (ESI): 549,23 ($MH^+$).

**228**: 4-{2-[3-(4'-Chlor-6-cyano-biphenyl-3-yl)-5,5-dimethyl-2,4-dioxo-imidazolidin-1-ylmethyl]-5-fluoro-phenylamino}-benzoesäure; Molekulargewicht 582,14 ($C_{32}H_{24}ClFN_4O_4$); Retentionszeit $R_t$ = 2.06 min. [B]; MS (ESI): 583,19 ($MH^+$).

**209**: 6-{2-[3-(4-Cyano-3-trifluoromethyl-phenyl)-5,5-dimethyl-2,4-dioxo-imidazolidin-1-ylmethyl]-phenylamino}-naphthalin-2-carbonsäure; Molekulargewicht 572,16 ($C_{31}H_{23}F_3N_4O_4$); Retentionszeit $R_t$ = 2.54 min. [C]; MS (ESI): 573,41 ($MH^+$).

**210**: 4'-{2-[3-(4-Cyano-3-trifluoromethyl-phenyl)-5,5-dimethyl-2,4-dioxo-imidazolidin-1-ylmethyl]-phenylamino}-biphenyl-4-carbonsäure; Molekulargewicht 598,18 ($C_{33}H_{25}F_3N_4O_4$); Retentionszeit $R_t$ = 2.52 min. [B]; MS ($ES^-$): 597,10 ($M-H^+$).

**212**: 5-{2-[3-(4-Cyano-3-trifluoromethyl-phenyl)-5,5-dimethyl-2,4-dioxo-imidazolidin-1-ylmethyl]-phenylamino}-2-methoxy-benzoesäure; Molekulargewicht 552,16 ($C_{28}H_{23}F_3N_4O_5$); Retentionszeit $R_t$ = 1.94 min. [B]; MS (ESI): 553,23 ($MH^+$).

Auf die gleiche Weise, jedoch unter Einsatz von Bromwasserstoffsäure in Eisessig, wurden die Verbindungen **99, 100, 101, 108** aus ihren Estern **87, 88, 89, 96** gewonnen:

**99**: 2-{2-[3-(4-Cyano-3-trifluoromethyl-phenyl)-5,5-dimethyl-2,4-dioxo-imidazolidin-1-ylmethyl]-phenylamino}-benzoesäure; Molekulargewicht 522,15 ($C_{27}H_{21}F_3N_4O_4$); Retentionszeit $R_t$ = 2.12 min. [B]; MS (ESI): 523,11 ($MH^+$).

**100:** 3-{2-[3-(4-Cyano-3-trifluoromethyl-phenyl)-5,5-dimethyl-2,4-dioxo-imidazolidin-1-ylmethyl]-phenylamino}-benzoesäure; Molekulargewicht 522,15 ($C_{27}H_{21}F_3N_4O_4$); Retentionszeit $R_t$ = 1.94 min. [B]; MS (ESI): 523,11 ($MH^+$).

**101**: 4-{2-[3-(4-Cyano-3-trifluoromethyl-phenyl)-5,5-dimethyl-2,4-dioxo-imidazolidin-1-ylmethyl]-phenylamino}-isophthalsäure; Molekulargewicht 566,14 ($C_{28}H_{21}F_3N_4O_6$); Retentionszeit $R_t$ = 1.79 min. [B]; MS (ESI): 567,12 ($MH^+$).

**108**: 2-{2-[3-(4-Cyano-3-trifluoromethyl-phenyl)-5,5-dimethyl-2,4-dioxo-imidazolidin-1-ylmethyl]-phenylamino}-5-fluorobenzoesäure; Molekulargewicht 540,14 ($C_{27}H_{20}F_3N_4O_4$); Retentionszeit $R_t$ = 2.12 min. [B]; MS (ESI): 541,22 ($MH^+$).

Auf die gleiche Weise wurden auch die Verbindungen **102** und **103** aus dem Ester **90** und die Verbindungen **105** und **107** aus den Estern **104** und **106** gewonnen:

**102**: 5-{2-[3-(4-Cyano-3-trifluoromethyl-phenyl)-5,5-dimethyl-2,4-dioxo-imidazolidin-1-ylmethyl]-phenylamino}-pyridin-2-carbonsäure; Molekulargewicht 523,14 ($C_{26}H_{20}F_3N_5O_4$); Retentionszeit $R_t$ = 1.52 min. [B]; MS (ESI): 524,09 ($MH^+$).

**103:** 5-{4-Brom-2-[3-(4-cyano-3-trifluoromethyl-phenyl)-5,5-dimethyl-2,4-dioxo-imidazolidin-1-ylmethyl]-phenylamino}-pyridin-2-carbonsäure; Molekulargewicht 601,05 ($C_{26}H_{19}BrF_3N_5O_4$); Retentionszeit $R_t$ = 1.68 min. [B]; MS (ESI): 602,01 ($MH^+$).

**105:** 4-{3,5-Dichlor-2-[3-(4-cyano-3-trifluoromethyl-phenyl)-5,5-dimethyl-2,4-dioxo-imidazolidin-1-ylmethyl]-phenylamino}-benzoesäure; Molekulargewicht 590,07 ($C_{27}H_{19}Cl_2F_3N_4O_4$); Retentionszeit $R_t$ = 2.18 min. [B]; MS (ESI): 591,12

(MH+).

**107**: 4-{2-[3-(4-Cyano-3-trifluoromethyl-phenyl)-5,5-dimethyl-2,4-dioxo-imidazolidin-1-ylmethyl]-5-trifluoromethyl-phenylamino}-benzoesäure; Molekulargewicht 590,13 ($C_{28}H_{20}F_6N_4O_4$); Retentionszeit $R_t$ = 2.00 min. [B]; MS (ESI): 591,20 (MH+).

Beispiel **63:** 2-tert-Butyl-4-[4,4-dimethyl-2,5-dioxo-3-(2-phenylamino-benzyl)-imidazolidin-1-yl]-benzonitril

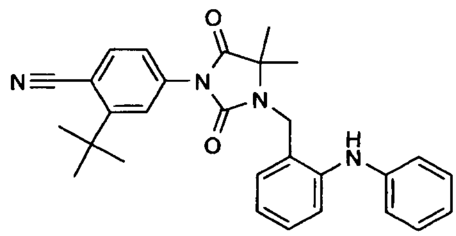

1) 2-tert-Butyl-4-nitro-benzonitril **63.3**:

1.1g 2-tert-Butyl-4-nitro-phenylamin wurden in 5 ml Eisessig gelöst und unter Eiskühlung mit 2 ml 30%iger Schwefelsäure versetzt. Innerhalb von 30 Minuten wurden bei 0°C langsam 460 mg Natriumnitrit zugegeben und die Mischung anschließend 5 h bei 0°C gerührt. Die Lösung wurde filtriert und zu einer 75°C warmen Lösung von 1,9 g Kaliumcyanid und 1,3 g Kupfercyanid in 20 ml Wasser zugetropft. Es wurde noch 1 h bei 75°C gerührt. Die abgekühlte Reaktionsmischung wurde abgesaugt und mit Wasser gewaschen. Der Rückstand wurde in Methanol verrührt, filtriert und das Filtrat im Vakuum eingeengt. Der Rückstand wurde über Kieselgel mit n-Heptan / Essigsäureethylester 80 / 20 chromatographisch gereinigt. Man erhielt 63.3 in 61% Ausbeute. $^1$H NMR: 8.25-8.15, m, 3H; 1.51, s, 9H.

2) Herstellung von 4-Amino-2-tert-butyl-benzonitril **63.4:**

0,6 g der Verbindung des Beispiels **63.3** wurden bei Raumtemperatur in 19,4 ml Jodwasserstoffsäure (57%) suspendiert und 4 h bei 80°C gerührt. Die abgekühlte Reaktionsmischung wurde in Essigsäureethylester aufgenommen und nacheinander vorsichtig mit gesättigter Natriumthiosulfatlösung, gesättigter Natriumhydrogencarbonatlösung und gesättigter Kochsalzlösung gewaschen, über Magnesiumsulfat getrocknet, filtriert und im Vakuum eingeengt. Der Rückstand wurde über Kieselgel mit n-Heptan / Essigsäureethylester 70 / 30 chromatographisch gereinigt. Man erhielt **63.4** mit 88% Ausbeute. Molekulargewicht 174.11 ($C_{11}H_{14}N_2$);
Retentionszeit $R_t$ = 1.89 min. [B]; MS (ESI): 175,22 (MH+).

3) Herstellung von 2-tert-Butyl-4-(4,4-dimethyl-2,5-dioxo-imidazolidin-1-yl)-benzonitril **63.1:**

Zur Herstellung der Verbindung **63.1** kann nach Verfahren "A" vorgegangen werden. Dazu wurden in einem Kolben 2,5 ml einer 20%igen Lösung von Phosgen in Toluol unter einer Argonatmosphäre vorgelegt. Zu dieser Lösung wurden 0,45 g der Verbindung des Beispiels **63.4** gelöst in 10 ml Acetonitril langsam zugetropft und anschließend 90 Minuten bei 75°C gerührt. Die abgekühlte Reaktionsmischung wurde im Vakuum eingeengt, mit Toluol versetzt und erneut eingeengt, Der Rückstand wurde in 10 ml trockenem Tetrahydrofuran gelöst und die Lösung mit 0,36 g 2-Amino-2-methylpropionsäuremethylester Hydrochlorid versetzt. Zu dieser Mischung wurden langsam 0,5 ml Triethylamin zugetropft und danach 2 h bei Raumtemperatur gerührt. Nach Zugabe von 2,4 ml konzentrierter Salzsäure wurde die Mischung 2 h unter Rückfluß gerührt. Zur Aufarbeitung wurde die abgekühlte Reaktionsmischung vorsichtig mit gesättigter Natriumhydrogencarbonatlösung versetzt und mit Essigsäureethylester extrahiert. Die organischen Extrakte wurden über Magnesiumsulfat getrocknet, filtriert und im Vakuum eingeengt. Der Rückstand wurde über Kieselgel mit n-Heptan / Essigsäureethylester 70 / 30 chromatographisch gereinigt. Die Verbindung **63.1** wurde in 76% Ausbeute erhalten. Molekulargewicht 285,14 ($C_{16}H_{19}N_3O_2$); Retentionszeit $R_t$ = 1.88 min. [B]; MS (ES$^-$): 284,50 ((M-H)$^+$).

4) Herstellung von 4-[3-(2-Brom-benzyl)-4,4-dimethyl-2,5-dioxo-imidazolidin-1-yl]-2-tert-butyl-benzonitril **63.2**:

Die Verbindung **63.2** wurde wie für Beispiel **6.2** beschrieben hergestellt, indem die Verbindung **63.1** mit 2-Brom-benzylbromid umgesetzt wurde. Man erhielt 4-[3-(2-Brom-benzyl)-4,4-dimethyl-2,5-dioxo-imidazolidin-1-yl]-2-tert-butyl-benzonitril in einer Ausbeute von 98%. Molekulargewicht 453,10 ($C_{23}H_{24}Br_3N_3O_2$); Retentionszeit $R_t$ = 2.65 min.

[B]; MS (ESI): 454,38 ($MH^+$).

5) Herstellung von 2-tert-Butyl-4-[4,4-dimethyl-2,5-dioxo-3-(2-phenylamino-benzyl)-imidazolidin-1-yl]-benzonitril **63:**

Die weitere Umsetzung mit Anilin zur Verbindung des Beispiels **63** erfolgte wie für Beispiel **1**, Stufe 3 beschrieben. Man erhielt 2-tert-Butyl-4-[4,4-dimethyl-2,5-dioxo-3-(2-phenylamino-benzyl)-imidazolidin-1-yl]-benzonitril **63.** Molekulargewicht 466,23 ($C_{29}H_{30}N_4O_2$); Retentionszeit $R_t$ = 3.09 min. [C]; MS (ESI): 467,29 ($MH^+$).

Die Verbindung des Beispiels **64**, 2-tert-Butyl-4-{3-[2-(2,4-difluoro-phenylamino)-benzyl]-4,4-dimethyl-2,5-dioxo-imidazolidin-1-yl}-benzonitril (Molekulargewicht 502,21 ($C_{29}H_{28}F_2N_4O_2$); Retentionszeit $R_t$ = 2.81 min. [B]; MS (ESI): 503,45 ($MH^+$)), wurde analog der Verbindung des Beispiels 63 durch Umsatz der Verbindung 63.2 mit 2,4-Difluoroanilin erhalten.

Die Verbindung des Beispiels **157**, 4-{2-[3-(3-tert-Butyl-4-cyano-phenyl)-5,5-dimethyl-2,4-dioxo-imidazolidin-1-ylmethyl]-5-fluoro-phenylamino}-benzoesäuremethylester wurde in analoger Weise erhalten. Dazu wurde **63.1** mit 2-Brom-1-brommethyl-4-fluoro-benzol zu 4-[3-(2-Brom-4-fluoro-benzyl)-4,4-dimethyl-2,5-dioxo-imidazolidin-1-yl]-2-tert-butyl-benzonitril (**157.2**; Molekulargewicht 471,09 ($C_{23}H_{23}BrFN_3O_2$); Retentionszeit $R_t$ = 2.31 min. [B]; MS (ESI): 472,24 ($MH^+$)) umgesetzt. Die Reaktion von 4-Aminobenzoesäuremethylester mit **157.2** unter Bedingungen wie sie weiter oben beschrieben sind ergab die Verbindung des Beispiels **157**; Molekulargewicht 542,23 ($C_{31}H_{31}FN_4O_4$); Retentionszeit $R_t$ = 2.31 min. [B]; MS (ESI): 543,31 ($MH^+$).

In analoger Weise wurde durch Umsatz von **157.2** mit 4-Aminobenzoesäure tert. butyl ester die Verbindung **196** (4-{2-[3-(3-tert-Butyl-4-cyano-phenyl)-5,5-dimethyl-2,4-dioxo-imidazoli din-1-ylmethyl]-5-fluoro-phenylamino}-benzoesäure tert-butyl ester; Molekulargewicht 584,27 ($C_{34}H_{37}FN_4O_4$); Retentionszeit $R_t$ = 2.60 min. [B]; MS (ESI): 529,18 ($MH^+ - C_4H_8$)) erhalten.

In analoger Weise wurde durch Umsatz von **157.2** mit 2,4-Difluoroanilin die Verbindung **221** (2-tert-Butyl-4-{3-[2-(2,4-difluoro-phenylamino)-4-fluoro-benzyl]-4,4-dimethyl-2,5-dioxo-imidazolidin-1-yl}-benzonitril; Molekulargewicht 520,20 ($C_{29}H_{27}F_3N_4O_2$); Retentionszeit $R_t$ = 2.80 min. [B]; MS (ESI): 521,23 ($MH^+$)) erhalten.

Beispiel **68:** 5,5-4-{2-[3-(4-Cyano-3-trifluoromethyl-phenyl)-5,5-dimethyl-2,4-dioxo-imidazolidin-1-ylmethyl]-phenylamino}-benzolsulfonamid

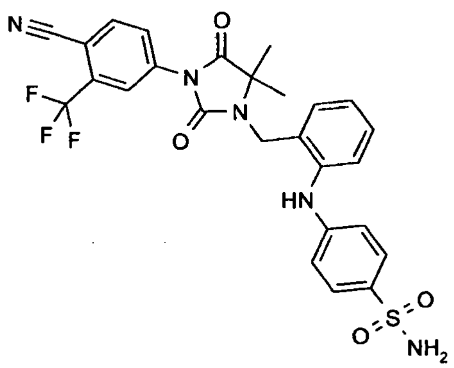

1) Alternativsynthese von 4-[3-(2-Amino-benzyl)-4,4-dimethyl-2,5-dioxo-imidazolidin-1-yl]-2-trifluoromethyl-benzonitril **61.2:**

Die Verbindung des Beispiels **61.2** kann auch auf einem anderen Wege nach Verfahren "A" hergestellt werden:

a) (2-Brommethyl-phenyl)-carbaminsäure-tert-butyl ester **68.1:**
1,3 g (2-Hydroxymethyl-phenyl)-carbaminsäure-tert-butyl ester wurden in 20 ml Dichlormethan gelöst; bei 5° C wurde dann eine Lösung von 630 mg Phosphortribromid in 5 ml DCM zugetropft. Die Reaktionsmischung wurde anschließend 2 h bei 5° C gerührt. Zur Aufarbeitung wurde die Mischung vorsichtig mit festem Natriumhydrogencarbonat und 1 ml Wasser versetzt, 30 min. bei 5° C gerührt, über eine Kartusche filtriert und im Vakuum eingeengt. Man erhielt (2-Brommethyl-phenyl)-carbaminsäure-tert-butyl ester in 67% Ausbeute. $^1H$ NMR: 8.71, s, 1H; 7.45, d, 1H; 7.42, d, 1H; 7.3, t, 1H; 7.1, t, 1H; 4.78, 2, 2H; 1.45, s, 9H.
b) {2-[3-(4-Cyano-3-trifluoromethyl-phenyl)-5,5-dimethyl-2,4-dioxo-imidazolidin-1-ylmethyl]-phenyl}-carbaminsäure-tert-butyl ester **68.2:**

1,1 g 4-(4,4-Dimethyl-2,5-dioxo-imidazolidin-1-yl)-2-trifluoromethylbenzonitril **1.1** und 1,1g der Verbindung **68.1** wurden in 11 ml trockenem Acetonitril gelöst, mit 1,5 g Cäsiumcarbonat versetzt und 5 h bei Raumtemperatur gerührt. Zur Aufarbeitung wurde die Reaktionsmischung mit Wasser versetzt und mit Essigsäureethylester ausgeschüttelt. Die organische Phase wurde über Natriumsulfat getrocknet, filtriert und im Vakuum eingeengt. Der Rückstand wurde chromatographisch gereinigt (Methode [RP2]). Die das gewünschte Produkt enthaltenden Fraktionen wurden destillativ von Acetonitril befreit; der wässrige Rückstand wurde mit gesättigter Natriumhydrogencarbonatlösung neutralisiert, mit Dichlormethan ausgeschüttelt, über Natriumsulfat getrocknet, filtriert und im Vakuum eingeengt. Man erhielt {2-[3-(4-Cyano-3-trifluoromethyl-phenyl)-5,5-dimethyl-2,4-dioxo-imidazolidin-1-ylmethyl]-phenyl}-carbaminsäure-tert-butyl ester **68.2.** $^1$HNMR: 8.81, s, 1H; 8.25, d, 1H; 8.23, s, 1H; 8.08, d, 1H; 7.41, d, 1H; 7.3, d, 1H; 7.22, t, 1H; 7.12, t, 1H; 4.55, s, 2H; 1.45, s, 9H; 1.35, s, 6H.

c) 4-[3-(2-Amino-benzyl)-4,4-dimethyl-2,5-dioxo-imidazolidin-1-yl]-2-trifluoromethyl-benzonitril Hydrochlorid (Salz von **61.2**):

0,369 g der Verbindung **68.2** wurden in 3,7 ml Essigsäureethylester gelöst und mit 1,47 ml einer 2 molaren Lösung von HCl in Diethylether versetzt und 24 h bei Raumtemperatur gerührt. Man gab noch zweimal jeweils 4 Äquivalente etherische HCl-Lösung zu und ließ die Lösung jeweils 24 h stehen. Zur Aufarbeitung wurde die Reaktionslösung im Vakuum eingeengt, der Rückstand in Acetonitril / Wasser gelöst und gefriergetrocknet. Man erhielt 4-[3-(2-Amino-benzyl)-4,4-dimethyl-2,5-dioxo-imidazolidin-1-yl]-2-trifluoromethyl-benzonitril Hydrochlorid in quantitativer Ausbeute. Molekulargewicht (freie Base) 402,13 ($C_{20}H_{17}F_3N_4O_2$); Retentionszeit $R_t$ = 1.59 min. [B]; MS (ESI): 403,14 ($MH^+$).

2) Herstellung von 4-Brom-N-[1-dimethylaminomethyliden]-benzolsulfonsäureamid **68.3**:

0,325 g 4-Brombezolsulfonsäureamid wurden in 1,6 ml trockenem Dimethylformamid mit 0,82 g Dimethylformamiddimethylacetal versetzt und 90 min. bei Raumtemperatur gerührt. Zur Aufarbeitung wurde die Reaktionsmischung mit 10 ml Wasser versetzt, der Niederschlag abgesaugt, filtriert und im Vakuum getrocknet. Man erhielt 4-Brom-N-[1-dimethylaminomethyliden]-benzolsulfonsäureamid in einer Ausbeute von 85%. Molekulargewicht 289,97 ($C_9H_{11}BrN_2O_2S$); Retentionszeit $R_t$ = 1.3 min. [B]; MS (ESI): 290,96 ($MH^+$).

3) Herstellung von 4-{2-[3-(4-Cyano-3-trifluoromethyl-phenyl)-5,5-dimethyl-2,4-dioxo-imidazolidin-1-ylmethyl]-phenylamino}-N-[1-dimethylamino-methyliden]-benzolsulfonsäureamid **68.4:**

Analog der Vorgehensweise wie für das Beispiel **61** beschrieben wurde die freie Base aus **68.1.c** mit der Verbindung **68.3** umgesetzt. Man erhielt 4-{2-[3-(4-Cyano-3-trifluoromethyl-phenyl)-5,5-dimethyl-2,4-dioxo-imidazolidin-1-ylmethyl]-phenylamino}-N-[1-dimethylamino-methyliden]-benzolsulfonsäureamid in einer Ausbeute von 94%. Molekulargewicht 612,17 ($C_{29}H_{27}F_3N_6O_4S$); Retentionszeit $R_t$ = 2.37 min. [C]; MS (ESI): 613,24 ($MH^+$).

4) 5,5-4-{2-[3-(4-Cyano-3-trifluoromethyl-phenyl)-5,5-dimethyl-2,4-dioxo-imidazolidin-1-ylmethyl]-phenylamino}-benzolsulfonamid **68**:

0,143 g der Verbindung 68.4 wurden in 2,3 ml Ethanol gelöst, mit 1,15 ml konzentrierter Salzsäure versetzt und 2 h unter Rückfluß gerührt. Die abgekühlte Reaktionsmischung wurde mit festem Natriumhydrogencarbonat vorsichtig neutralisiert und im Vakuum eingeengt. Der Rückstand wurde mit Wasser versetzt, dreimal mit Essigsäureethylester ausgeschüttelt, die organischen Phasen über Magnesiumsulfat getrocknet, filtriert und im Vakuum eingeengt. Der Rückstand wurde chromatographisch gereinigt (Methode [RP1]). Man erhielt 5,5-4-{2-[3-(4-Cyano-3-trifluoromethyl-phenyl)-5,5-dimethyl-2,4-dioxo-imidazolidin-1-ylmethyl]-phenylamino}-benzolsulfonamid in 64& Ausbeute. Molekulargewicht 557,13 ($C_{26}H_{22}F_3N_5O_4S$); Retentionszeit $R_t$ = 1.85 min. [B]; MS (ESI): 558,28 ($MH^+$).

Die Verbindung **81** (4-{2-[3-(4-Cyano-3-trifluoromethyl-phenyl)-5,5-dimethyl-2,4-dioxo-imidazolidin-1-ylmethyl]-phenylamino}-3-fluoro-benzolsulfonamid; Molekulargewicht 575,12 ($C_{26}H_{21}F_4N_5O_4S$); Retentionszeit $R_t$ = 1.87 min. [B]; MS (ESI): 576,11 ($MH^+$)) wurde nach dem eben beschriebenen Verfahren über 4-{2-[3-(4-Cyano-3-trifluoromethyl-phenyl)-5,5-dimethyl-2,4-dioxo-imidazolidin-1-ylmethyl]-phenylamino}-N-[1-dimethylaminomethyliden]-3-fluoro-benzolsulfonsäureamid **81.1**, Molekulargewicht 630,16 ($C_{29}H_{26}F_4N_6O_4S$); Retentionszeit $R_t$ = 1.92 min. [B]; MS (ESI): 631,11 ($MH^+$), erhalten, welches aus 4-Brom-N-[1-dimethylamino-methyliden]-3-fluoro-benzolsulfonsäureamid **81.2** und der freien Base aus 68.1.c gewonnen worden war.

Die Verbindung **98** 4-{2-[3-(4-Cyano-3-trifluoromethyl-phenyl)-5,5-dimethyl-2,4-dioxo-imidazolidin-1-ylmethyl]-phenylamino}-2,5-difluoro-benzolsulfonsäureamid; Molekulargewicht 593,11 ($C_{26}H_{20}F_5N_5O_4S$); Retentionszeit $R_t$ = 1.88 min. [B]; MS (ESI): 594,18 ($MH^+$)) wurde nach dem eben beschriebenen Verfahren über 4-{2-[3-(4-Cyano-3-trifluoromethyl-phenyl)-5,5-dimethyl-2,4-dioxo-imidazolidin-1-ylmethyl]-phenylamino}-N-[1-dimethylamino-methyliden]-2,5-difluoro-benzolsulfonsäureamid **98.1**, Molekulargewicht 648,15 ($C_{29}H_{25}F_5N_6O_4S$); Retentionszeit $R_t$ = 1.95 min. [B]; MS (ESI): 649,08 ($MH^+$), erhalten, welches aus 4-Brom-N-[1-dimethylamino-methyliden]-2,5-difluorobenzolsulfonsäureamid **98.2** und der freien Base aus **68.1.c** gewonnen worden war.

Beispiel **93** und **94**:

(4-{2-[3-(4-Cyano-3-trifluoromethyl-phenyl)-5,5-dimethyl-2,4-dioxo-imidazolidin-1-ylmethyl]-phenylamino}-phenyl)-phosphonsäure-mono-ethylester **93** und

(4-{2-[3-(4-Cyano-3-trifluoromethyl-phenyl)-5,5-dimethyl-2,4-dioxo-imidazolidin-1-ylmethyl]-phenylamino}-phenyl)-phosphonsäure **94**

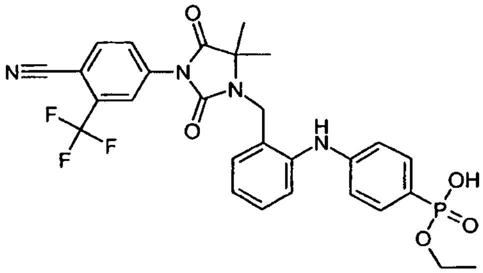

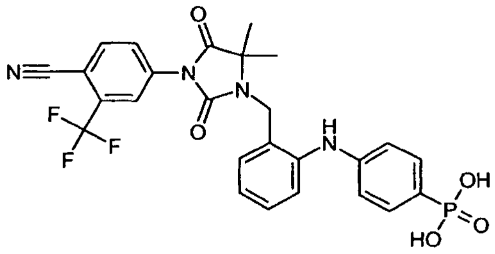

0,17 g der Verbindung des Beispiels **79** wurden bei Raumtemperatur in 3 ml trockenem Dioxan gelöst, mit 0,23 ml konzentrierter Salzsäure versetzt und 4 h bei 80°C gerührt. Danach wurden 0,43 ml konzentrierte Salzsäure nachgegeben und weitere 8h bei 80°C gerührt. Die abgekühlte Reaktionsmischung wurde im Vakuum eingeengt und der Rückstand chromatographisch gereinigt (Methode RP1]). Man erhielt den Monoester **93** und die Phosphonsäure **94**.
93: Molekulargewicht 586,15 ($C_{28}H_{26}F_3N_4O_5P$); Retentionszeit $R_t$ = 1.75 min. [B];
MS (ESI): 587,10 ($MH^+$).
94: Molekulargewicht 558,12 ($C_{26}H_{22}F_3N_4O_5P$); Retentionszeit $R_t$ = 1.92 min. [C];
MS (ESI): 559,26 ($MH^+$).

Beispiel 95: 4-{3-[2-(4-Amino-3-fluoro-phenylamino)-benzyl]-4,4-dimethyl-2,5-dioxo-imidazolidin-1-yl}-2-trifluoromethylbenzonitril

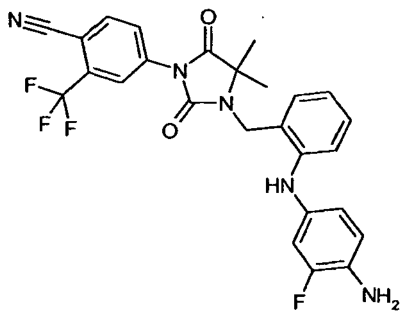

1) Herstellung von N'-(2-Fluoro-4-nitro-phenyl)-N,N-dimethyl-formamidin 95.1:

5 g 2-Fluoro-4-nitroanilin wurden in 35 ml trockenem Dimethylformamid gelöst, bei Raumtemperatur mit 19,09 g Dimethylformamiddimethylacetal versetzt und 90 min. gerührt. Zur Aufarbeitung wurden 250 ml Wasser zur Reaktionsmischung gegeben, der Niederschlag abgesaugt, mit Wasser gewaschen und getrocknet. Man erhielt N'-(2-Fluoro-4-nitro-phenyl)-N,N-dimethyl-formamidin in einer Ausbeute von 85%.
Molekulargewicht 211.07 ($C_9H_{10}FN_3O_2$); Retentionszeit $R_t$ = 0.49 min. [B]; MS (ESI): 212,03 ($MH^+$).

2) Herstellung von N'-(4-Amino-2-fluoro-phenyl)-N,N-dimethyl-formamidin 95.2:

5,37 g der Verbindung 95.1 wurden in 110 ml Methanol gelöst. Es wurden 747 mg Raney-Nickel zugefügt, und

anschließend wurde die Mischung bei 5 bar bis zur beendeten Wasserstoffaufnahme hydriert. Zur Aufarbeitung wurde vom Katalysator abfiltriert und das Filtrat weitgehend im Vakuum eingeengt. Das Produkt wurde durch Zugabe von Wasser ausgefällt, filtriert und mit wenig Wasser gewaschen. Das Filtrat wurde mehrmals mit einer Mischung aus Dichlormethan und Isopropanol 4:1 extrahiert. Der Extrakt wurde im Vakuum eingeengt und getrocknet. Man erhielt N'-(4-Amino-2-fluoro-phenyl)-N,N-dimethyl-formamidin in 83% Ausbeute.
Molekulargewicht 181,10 ($C_9H_{12}FN_3$); Retentionszeit $R_t$ = 0.15 min. [B]; MS (ESI): 182,10 ($MH^+$).

3) Herstellung von N'-(4-{2-[3-(4-Cyano-3-trifluoromethyl-phenyl)-5,5-dimethyl-2,4-dioxo-imidazolidin-1-ylmethyl]-phenylamino}-2-fluoro-phenyl)-N,N-dimethyl-formamidin **95.3:**

Die weitere Umsetzung der Verbindung **95.2** mit der Verbindung **1.2** zur Verbindung des Beispiels **95.3** erfolgte wie für Beispiel **1,** Stufe 3 beschrieben. Man erhielt N'-(4-{2-[3-(4-Cyano-3-trifluoromethyl-phenyl)-5,5-dimethyl-2,4-dioxo-imidazolidin-1-ylmethyl]-phenylamino}-2-fluoro-phenyl)-N,N-dimethyl-formamidin. Molekulargewicht 566,20 ($C_{29}H_{26}F_4N_6O_2$); Retentionszeit $R_t$ = 1.55 min. [B]; MS (ESI): 567,10 ($MH^+$).

4) 4-{3-[2-(4-Amino-3-fluoro-phenylamino)-benzyl]-4,4-dimethyl-2,5-dioxo-imidazolidin-1-yl}-2-trifluoromethyl-benzonitril **95:**

520 mg der Verbindung **95.3** wurden in einer Mischung von tert.-Butanol / Wasser (10 ml) gelöst, mit 30 mg Palladiumhydroxid versetzt und bei 60° C und 6 bar hydriert. Man erhielt 4-{3-[2-(4-Amino-3-fluoro-phenylamino)-benzyl]-4,4-dimethyl-2,5-dioxo-imidazolidin-1-yl}-2-trifluoromethyl-benzonitril.
Molekulargewicht 511,16 ($C_{26}H_{21}F_4N_5O_2$); Retentionszeit $R_t$ = 1.58 min. [B]; MS (ESI): 512,26 ($MH^+$).

Beispiel **97:** 2-(4-{2-[3-(4-Cyano-3-trilluoromethyl-phenyl)-5,5-dimethyl-2,4-dioxo-imidazolidin-1-ylmethyl]-phenylamino}-phenyl)-acetamid:

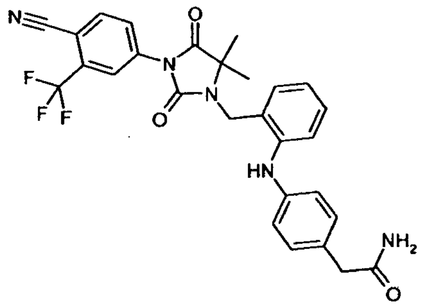

265 mg der Verbindung **78** wurden in 0,56 ml Eisessig gelöst und mit 0,44 ml Bromwasserstoffsäure (33%ig in Essigsäure) versetzt. Die Mischung wurde 2 h bei 75° C gerührt; am nächsten Tag weitere 8 h bei 100° C gerührt. Die abgekühlte Lösung wurde mit Wasser versetzt, Im Vakuum eingeengt und chromatographisch gereinigt (Methode [RP1]). Man erhielt **97** in 42% Ausbeute. Molekulargewicht 535,18 ($C_{28}H_{24}F_3N_5O_3$); Retentionszeit $R_t$ = 2.18 min. [C]; MS (ESI): 536,21 ($MH^+$).

Beispiel **113**: 4-{3-[2-(4-Amino-phenylamino)-benzyl]-4,4-dimethyl-2,5-dioxo-imidazolidin-1-yl}-2-trifluoromethyl-benzonitril

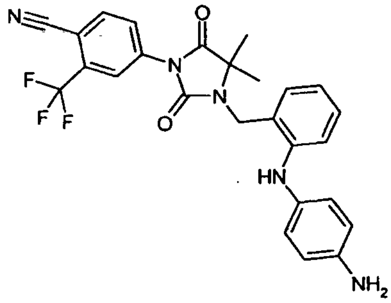

kann nicht nur über die Hydrolyse der entsprechenden Benzylidenverbindung (wie weiter oben beschrieben) gewonnen werden, sondern auch durch Reduktion der Nitroverbindung **112** mit Raney-Nickel und Wasserstoff in einer Mischung aus Methanol und Dichlormethan. $^1$H NMR: 8.35, d, 1H; 8.8.24, s, 1H; 8.07, d, 1H; 7.33, d, 1H; 7.08, t, 1H; 6.96, s, 1H; 6.83, s, 1H; 6.79, d, 2H; 6.72, t, 1H; 6.57, d, 2H; 4.8, s, 2H; 4.59, s, 2H; 1.41, s, 6H.

Beispiel 121: 4-{3-[2-(4-Amino-phenylamino)-benzyl]-4,4-dimethyl-2,5-dioxo-imidazolidin-1-yl}-2-trifluoromethyl-benzonitril x Trifluoressigsäure:

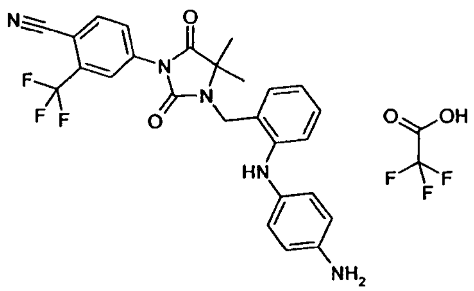

Das Trifluoressigsäuresalz **121** wurde erhalten durch Umsatz des freien Amins 113 mit Trifluoressigsäure in Acetonitril. $^1$H NMR: 9.3, s (breit), 2H; 8.35, d, 1H; 8.21, s, 1H; 8.05, d, 1H; 7.8, s, 1H; 7.5, d, 1H; 7.28, t, 1H; 7.2, d, 1H; 7.12, d, 2H; 7.08, t, 1H; 6.9, d, 2H; 4.56, s, 2H; 1.35, s, 6H.

Beispiel **136:** 4-{2-[3-(4-Cyano-3-trifluoromethyl-phenyl)-5,5-dimethyl-2,4-dioxo-imidazolidin-1-ylmethyl]-phenylamino}-benzamid:

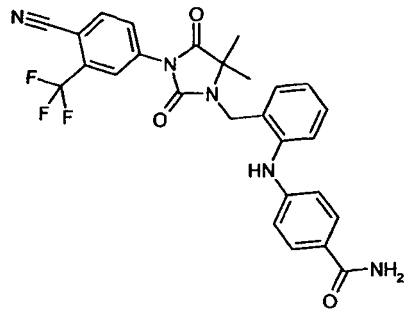

1) Herstellung von 4-(3-{2-[4-(Benzotriazol-1-carbonyl)-phenylamino]-benzyl}-4,4-dimethyl-2,5-dioxo-imidazolidin-1-yl)-2-trifluoromethyl-benzonitri **136.1:**

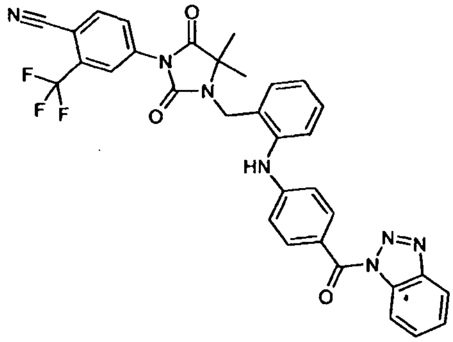

550,2 mg der Säure **62,** 207,7 mg 1-Methansulfonyl-1H-benzotriazol (A. R. Katritzky et al.: J. Org. Chem. 2005, 70, 9191-9197) und 0,205 ml Triethylamin wurden in 5,5 ml trockenem Tetrahydrofuran gelöst und 6 Stunden bei Rückflusstemperatur gerührt. Es wurden ein weiteres Äquivalent Benzotriazolderivat und Triethylamin zugegeben und über Nacht bei Rückflusstemperatur gerührt. Anderntags wurde die abgekühlte Reaktionsmischung im Vakuum eingeengt, mit 10 ml Dichlormethan aufgenommen, zweimal mit Wasser ausgeschüttelt und die organische Phase abgetrennt, über Magnesiumsulfat getrocknet, filtriert und im Vakuum eingeengt. Der Rückstand wurde chromatographisch gereinigt (Kieselgel; n-Heptan / Essigsäureethylester 1/9 bis 1/2). Man erhielt 4-(3-{2-[4-(Benzotriazol-1-carbonyl)-phenylamino]-benzyl}-4,4-dimethyl-2,5-dioxo-imidazolidin-1-yl)-2-trifluoromethyl-benzonitril. Molekulargewicht 623,18 ($C_{33}H_{24}F_3N_7O_3$); Retentionszeit Rt = 2.75 min. [B]; MS (ES-): 622,14 ($M-H^+$).

2) Herstellung von 4-{2-[3-(4-Cyano-3-trifluoromethyl-phenyl)-5,5-dimethyl-2,4-dioxo-imidazolidin-1-ylmethyl]-phenylamino}-benzamid **136:**

430,3 mg der Verbindung **136.1** wurden in 2,5 ml trockenem Tetrahydrofuran gelöst und mit 0,2 ml einer Lösung von Ammoniak in Tetrahydrofuran (7 N in Methanol) versetzt. Die Lösung stand 3 Tage bei Raumtemperatur, wurde danach im Vakuum eingeengt und chromatographisch (Methode [RP1]) gereinigt. Man erhielt 4-{2-[3-(4-Cyano-3-trifluoromethyl-phenyl)-5,5-dimethyl-2,4-dioxo-imidazolidin-1-ylmethyl]-phenylamino}-benzamid **136.** Molekulargewicht 521,16 ($C_{27}H_{22}F_3N_5O_3$);
Retentionszeit $R_t$ = 1.75 min. [B]; MS (ESI): 522,19 ($MH^+$).

Die Verbindungen der Beispiele **137,** 4-{2-[3-(4-Cyano-3-trifluoromethyl-phenyl)-5,5-dimethyl-2,4-dioxo-imidazolidin-1-ylmethyl]-phenylamino}-N-(2-hydroxy-1-hydroxymethyl-ethyl)-benzamid (Molekulargewicht 595,20 ($C_{30}H_{28}F_3N_5O_5$); Retentionszeit $R_t$ = 1.64 min.
[B]; MS (ESI): 596,22 ($MH^+$),
**149,** 4-{2-[3-(4-Cyano-3-trifluoromethyl-phenyl)-5,5-dimethyl-2,4-dioxo-imidazolidin-1-ylmethyl]-phenylamino}-N-(2-hydroxy-ethyl)-benzamid (Molekulargewicht 565,19 ($C_{29}H_{26}F_3N_5O_4$); Retentionszeit $R_t$ = 1.74 min. [B]; MS (ESI): 566,27 ($MH^+$),
**161,** N-Carbamoylmethyl-4-{2-[3-(4-cyano-3-trifluoromethyl-phenyl)-5,5-dimethyl-2,4-dioxo-imidazolidin-1-ylmethyl]-phenylamino}-benzamid (Molekulargewicht 578,18 ($C_{29}H_{25}F_3N_6O_4$); Retentionszeit $R_t$ = 1.69 min. [B]; MS (ESI): 579,16 ($MH^+$),
**162,** 2-(4-{2-[3-(4-Cyano-3-trifluoromethyl-phenyl)-5,5-dimethyl-2,4-dioxo-imidazolidin-1-ylmethyl]-phenylamino}-benzoylamino)-2-methyl-propionsäure tert.-Butyl ester (Molekulargewicht 663,26 ($C_{35}H_{36}F_3N_5O_5$); Retentionszeit $R_t$ = 2.26 min. [B]; MS (ESI): 664,39 ($MH^+$),
**163,** 4-(4,4-Dimethyl-2,5-dioxo-3-{2-[4-(piperidin-1-carbonyl)-phenylamino]-benzyl}-imidazolidin-l-yl)-2-trifluoromethyl-benzonitril (Molekulargewicht 589,62 ($C_{32}H_{30}F_3N_5O_3$); Retentionszeit $R_t$ = 2.14 min. [B]; MS (ESI): 590,18 ($MH^+$)
wurden wie für **136** beschrieben aus **136.1** durch Umsatz mit
2-Amino-propan-1,3-diol (für **137**),
2-Amino-ethanol (für **149**),
2-Amino-acetamid (für **161**),
2-Amino-2-methyl-propionsäure tert.-butyl ester (für **162**),
Piperidin (für **163**)
und Diisopropyl-ethyl-amin gewonnen.

Beispiel **190:** 4-{2-[3-(4-Cyano-3-methyl-phenyl)-5,5-dimethyl-2,4-dioxo-imidazolidin-1-ylmethyl]-5-fluoro-phenylamino}-benzamid:

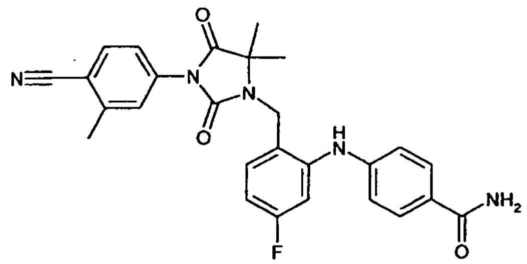

Die Verbindung des Beispiels **190** wurde hergestellt wie für **170** beschrieben, mit dem Unterschied, dass als Säurekomponente die Verbindung **166** und als Aminkomponente Ammoniak (konzentrierte wässrige Lösung) eingesetzt wurde. Molekulargewicht 485,18 ($C_{27}H_{24}FN_5O_3$); Retentionszeit $R_t$ = 1.68 min. [B]; MS (ESI): 486,22 ($MH^+$).

Beispiel 253: 5-{3-[2-(2,4-Difluoro-phenylamino)-4-fluoro-benzyl]-4,4-dimethyl-2,5-dioxo-imidazolidin-1-yl}-4'-fluoro-biphenyl-2-carbonitril:

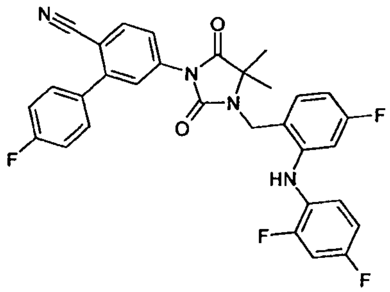

Die Verbindung des Beispiels **253** (5-{3-[2-(2,4-Difluoro-phenylamino)-4-fluoro-benzyl]-4,4-dimethyl-2,5-dioxo-imidazolidin-1-yl}-4'-fluoro-biphenyl-2-carbonitril, Molekulargewicht 558,16 ($C_{31}H_{22}F_4N_4O_2$); Retentionszeit $R_t$ = 2.38 min. [B]; MS (ESI): 559,15 ($MH^+$)) wurde ähnlich wie für die Herstellung der Verbindung des Beispiels **223** beschrieben über die Sequenz 4-Amino-2-chlor-benzonitril → 5-Amino-4'-fluoro-biphenyl-2-carbonitril (**253.3,** $^1$H NMR: 7.54-7.49, m, 3H; 7.31, t, 2H, 6.61, m, 2H; 6.25, s, 2H) → 5-(4,4-Dimethyl-2,5-dioxo-imidazolidin-1-yl)-4'-fluoro-biphenyl-2-carbonitril (**253.1**, $^1$H NMR: 8.73, s, 1H; 8.09, d, 1H; 7.74, s, 1H; 7.65, m, 3H; 7.41, m, 2H; 1.42, s, 6H) → 5-[3-(2-Brom-4-fluoro-benzyl)-4,4-dimethyl-2,5-dioxo-imidazolidin-1-yl]-4'-fluoro-biphenyl-2-carbonitril (**253.2**, $^1$H NMR: 8.1, d, 1H; 7.8, s, 1H; 7.75, d, 1H; 7.69-7.59, m, 4H; 7.41, t, 1H; 4.61, s, 2H; 1.4, s, 6H) → 253 (aus **253.2** durch Umsatz mit 2,4-Difluoro-phenylamin) gewonnen.

Herstellung von **260.1:** 4-[3-(2-Amino-4-fluoro-benzyl)-4,4-dimethyl-2,5-dioxo-midazolidin-1-yl]-2-trifluoromethyl-benzonitril:

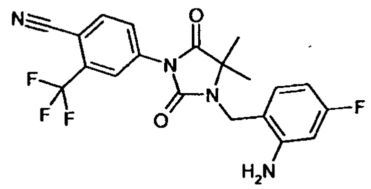

Analog der Vorgehensweise wie sie für die Herstellung von **61.2** beschrieben ist, wurde **111.2** mit Benzophenonimin zu **260.1** umgesetzt. Molekulargewicht 420,12 ($C_{20}H_{16}F_4N_4O_2$); Retentionszeit $R_t$ = 2.71 min. [E]; MS (ESI): 421,08 ($MH^+$).

Pharmakologische Prüfung:

*In vitro* Prüfungen:

*In vitro* funktionale Assays mit rekombinanten Zellen:

Funktionsüberprüfende Assays wurden mittels der FLIPR-Technik ("Fluorometric Imaging Plate Reader", Molecular Devices Corp.) durchgeführt.

Hierzu wurden ligand-induzierte Änderungen der intrazellulären Konzentration von $Ca^{2+}$ in rekombinanten HEK293 Zellen bestimmt, die sowohl einen Cannabinoidrezeptor (CB1 oder CB2) als auch G-Protein Galpha16 exprimierten. Für die Untersuchungen wurden Zellen in 96-well-Mikrotiterplatten (60000 Zellen/Vertiefung) ausgesät und übernacht wachsengelassen. Das Medium wurde entfernt und die Zellen in Puffer inkubiert, der den Fluoreszenzfarbstoff Fluo-4 enthielt. Nach dieser Beladung mit Farbstoff wurden die Zellen gewaschen, Testsubstanz in Puffer gelöst zugegeben, 20 Minuten inkubiert, ein bekannter Cannabinoid-Rezeptor-Agonist als Referenzagonist in Puffer zugegeben und zum Schluss die Änderungen der intrazellulären $Ca^{2+}$-Konzentration im FLIPR-Gerät gemessen.

Ergebnisse wurden als prozentuale Änderung relativ zur Kontrolle dargestellt (0%: analoges Experiment ohne Testsubsstanz und ohne Referenzagonist, d.h. nur mit Puffer; 100%: analoges Experiment ohne Testsubstanz, aber mit Referenzagonist im Überschuss), zur Berechnung von Dosis/Wirkungskurven verwendet und $IC_{50}$-Werte bestimmt.

Ergebnisse:

Der folgenden Tabelle 1 sind die Werte des funktionellen Assays gegenüber dem Cannabinoid 1 Rezeptor einschließlich beispielhafter Selektivitäten gegenüber dem Cannabinoid 2 Rezeptor zu entnehmen.

Tabelle 1:

| Beispiel Nr. | hCB1R: FLIPR; $IC_{50}$ [nM] | hCB2R: FLIPR; $IC_{50}$ [nM] |
|---|---|---|
| 1 | 10 | > 10000 |
| 2 | 11 | > 10000 |
| | | |
| 4 | 4 | |
| 5 | 44 | > 10000 |
| 11 | 0.7 | > 10000 |
| | | |
| 20 | 21 | |
| 21 | 10 | |
| 22 | 46 | |
| 30 | 208 | > 10000 |
| | | |
| 35 | 15 | |
| 36 | 11 | > 10000 |
| 37 | 21 | |
| 38 | 70 | |
| 39 | 69 | |
| 40 | 30 | |
| 41 | 28 | |
| 42 | 12 | > 10000 |
| 43 | 77 | |
| 44 | 7 | |

(fortgesetzt)

| Beispiel Nr. | hCB1R: FLIPR; $IC_{50}$ [nM] | hCB2R: FLIPR; $IC_{50}$ [nM] |
|---|---|---|
| 55 | 10 | |
| 56 | 8 | > 10000 |
| 57 | 36 | > 10000 |
| | | |
| 64 | 16 | > 10000 |
| | | |
| | | |
| 67 | 1 | > 10000 |
| 68 | 44 | |
| | | |
| | | |
| | | |
| | | |
| | | |
| 80 | 33 | |
| 81 | 144 | > 10000 |
| | | |
| | | |
| | | |
| | | |
| | | |
| 92 | 137 | > 10000 |
| 95 | 31 | |
| 97 | 7 | > 10000 |
| 104 | 15 | |
| 105 | 49 | |
| 106 | 12 | |
| 107 | 112 | |
| | | |
| | | |
| | | |
| 130 | 32 | |
| 131 | 15 | |
| 133 | 10 | |
| | | |
| 136 | 5 | > 10000 |
| 137 | | > 10000 |
| | | |

(fortgesetzt)

| Beispiel Nr. | hCB1R: FLIPR; $IC_{50}$ [nM] | hCB2R: FLIPR; $IC_{50}$ [nM] |
|---|---|---|
| | | |
| | | |
| | | |
| 156 | 10 | |
| | | |
| 159 | 2 | |
| | | |
| 166 | 76 | |
| 169 | 9 | |
| | | |
| | | |
| 178 | 4 | |
| | | |
| | | |
| 190 | 76 | |
| | | |
| 198 | 23 | |
| | | |
| | | |
| | | |
| | | |
| | | |
| | | |
| 209 | 62 | |
| | | |
| 221 | 10 | |
| 227 | 16 | |
| 228 | 61 | |
| | | |
| | | |
| | | |
| 239 | 103 | |
| | | |
| 253 | 29 | |
| | | |
| 255 | 28 | |

Bindung an den CB1 Rezeptor:

Testverbindungen: Die Verbindungen (3 µl, 10 mM, 100% DMSO), einpipettiert in 96-well PP Mikrotiterplatten, wurden mit 27 µl 100 % DMSO (Dimethylsulfoxid) verdünnt. Ausgehend von dieser Lösung wurden weitere 3-fach Verdünnungsschritte vorgenommen, indem jeweils 10 µl auf einer neue PP Mikrotiterplatte überführt und weitere 20 µl 100 % DMSO zugefügt wurden. Jeweils 6 µl dieser Lösungen wurden in neue 96-well PP Mikrotiterplatten transferiert und mit 144 µl Assaypuffer aufgefüllt. Die Endkonzentrationen reichten von 10 µM bis 0.005 µM.

Negativkontrolle: AM 251, gelöst in Assaypuffer mit 1% DMSO, wurde zu den Verdünnungsreihen in den Mikrotiterplatten als Kontrolle mitgeführt. Die Endkonzentration betrug 1 µM.

Leerkontrolle: Assaypuffer mit 1 % DMSO wurde in den Verdünnungsreihen der Mikrotiterplatten als Leerkontrolle mitgeführt.

Zusammenfassung der Assayparameter:

| | | |
|---|---|---|
| Assayvolumen | | 200 µl |
| Rezeptor | CHO-K1/ cannabinoid CB1 Protein | 2 µg / well |
| Ligand | [$^3$H]-SR141716A | 0.5 nM 0.0195 µCi/well |
| Ionen | Tris-HCl | 50 mM, pH 7.4 |
| | $MgCl_2$ | 5 mM |
| | EDTA | 2.5 mM |
| | BSA (fettsäurefrei) | 0.2% |
| Unspezifische Bindung | AM 251 | 1 µM |
| Verbindung | in 1% DMSO | 10 µM bis 0.0050 µM |

Analyse der Daten:

Hohe Kontrolle: $^3$H Bindung ohne Zugabe der Verbindung
Niedrige Kontrolle: $^3$H Bindung in Gegenwart von 1 µM AM 251

Die Werte wurden über die korrigierten Rohdaten berechnet.

$$\text{Inhibierung der Ligandenbindung (\%)} = 100 * \left(1 - \frac{(sample - lowcontrol)}{(highcontro\ l - lowcontrol)}\right)$$

Die aufgeführten Werte wurden als Durchschnittswerte einer Doppelbestimmung gewonnen. Die $IC_{50}$ Werte wurden aus den Messwerten mit dem Programm Xlfit, Formel 205, berechnet. Ki-Werte wurden aus den $IC_{50}$- and Kd-Werten unter Benutzung der Cheng-Prusoff Gleichung erhalten:

$$Ki = \frac{IC50}{1 + \frac{C}{Kd}} \qquad \text{(C= Konzentration des Radioliganden)}$$

Literatur: Cheng, Y.-C., und Prusoff, W.H. (1973) Biochem. Pharmacol 22, 3099-3108

Ergebnisse: $K_i$-Werte von Beispielverbindungen; Tabelle 2:

| Beispiel Nr. | hCB1R; Bindung $K_i$ [nM] |
|---|---|
| 1 | 13 |
| 5 | 7 |
| 6 | 21 |
| 7 | 15 |
| 8 | 15 |
| 9 | 13 |
| 10 | 24 |
| 11 | 1 |
| 12 | 0.9 |
| 13 | 0.9 |
| 15 | 2 |
| 16 | 2 |
| 17 | 3 |
| 18 | 5 |
| 19 | 5 |
| 23 | 10 |
| 24 | 11 |
| 25 | 6 |
| 26 | 39 |
| 27 | 17 |
| 28 | 87 |
| 29 | 107 |
| 32 | 3 |
| 34 | 14 |
| 42 | 1 |
| 50 | 11 |
| 51 | 2 |
| 52 | 3 |
| 53 | 2 |
| 55 | 8 |
| 56 | 3 |
| 60 | 1 |
| 61 | 19 |
| 62 | 16 |
| 63 | 9 |
| 64 | 5 |
| 66 | 7 |
| 67 | 1 |
| 68 | 16 |

(fortgesetzt)

| Beispiel Nr. | hCB1R; Bindung $K_i$ [nM] |
|---|---|
| 69 | 113 |
| 70 | 27 |
| 110 | 5 |
| 111 | 1 |
| 112 | 1 |
| 120 | 5 |
| 126 | 23 |
| 128 | 2 |
| 129 | 10 |
| 132 | 6 |
| 134 | 4 |
| 137 | 51 |
| 140 | 26 |
| 142 | 3 |
| 144 | 19 |
| 149 | 6 |
| 151 | 155 |
| 153 | 20 |
| 157 | 5 |
| 160 | 3 |
| 161 | 8 |
| 165 | 16 |
| 167 | 7 |
| 170 | 113 |
| 171 | 120 |
| 172 | 8 |
| 173 | 34 |
| 174 | 144 |
| 176 | 130 |
| 179 | 16 |
| 223 | 4 |
| 224 | 6 |
| 227 | 36 |

Aus dem Messdaten ist abzulesen, dass die erfindungsgemäßen Verbindungen der Formel I als CB1R Antagonisten wirken und daher gut zur Behandlung des metabolischen Syndroms, des Diabetes Typ II und der Adipositas geeignet sind.

*In vivo* Prüfungen:

"Milchkonsum bei Mäusen"

Der Test wird zur Untersuchung der anorexigenen Potenz der Testsubstanzen verwendet. Es werden weibliche NMRI-Mäuse, 25-35 g schwer, verwendet. Die Mäuse werden mindestens eine Woche an die Haltungsbedingungen und 2 Tage an die angebotene Kondensmilch gewöhnt.

Die Mäuse werden für 24 Stunden nüchtern gesetzt, haben aber beständig Zugang zu Wasser. Am Tage des Versuchs werden die Tiere einzeln aufgestallt, die Käfigdeckel können die mit Milch gefüllten Pipetten aufnehmen. Die Prüfsubstanzen werden oral, intraperitoneal oder subkutan verabreicht. Nach der Applikation werden die Mäuse in ihre Käfige gesetzt und erhalten 30 min. später Zugang zur Milch. Der Milchverbrauch wird alle 30 min. über 7 Stunden abgelesen, gleichzeitig werden offensichtliche Verhaltensänderungen der Tiere notiert.

"Antagonisierung CB1-vermittelter Hypothermie"

Der Test wird zur Messen der Potenz von cannabinoiden CB1-Rezeptor (CB1)-Antagonisten verwendet. Dabei wird gemessen, inwieweit die zu prüfenden CB1-Antagonisten in der Lage sind, die durch einen CB1-Agonisten induzierte Hypothermie zu verhindern, bzw. zu antagonisieren.

Es werden weibliche NMRI-Mäuse, 25-35 g schwer, verwendet. Die Mäuse werden mindestens eine Woche an die Haltungsbedingungen gewöhnt.

Zum Zeitpunkt 0 min. werden die Tiere mit dem zu prüfenden CB1-Antagonisten oral, intravenös oder intraperitoneal behandelt. 30 min. später wird den Mäusen der CB1-Agonist CP55.940, 1,25 mg/kg intraperitoneal verabreicht. Dies bewirkt ein Absinken der Körpertemperatur um 5-6 °C innerhalb von 30 min. Die Körpertemperatur wird zum ersten Mal 30 min. vor der Prüfsubstanz-Applikation und dann alle 30 min. nach dieser Applikation, ggf. unmittelbar vor einer Substanzapplikation über 4 Stunden rektal gemessen.

Die Potenz der Prüfsubstanzen wird als prozentuale Verringerung der Fläche unter der Temperatur-Zeit-Kurve angegeben, die zum einen von der durchschnittlichen Basaltemperatur, zum anderen von der Temperatur-Zeit-Kurve, der ausschließlich mit dem CB1-Antagonisten behandelten Tiere gebildet wird.

"Intestinale Motilität bei der Maus"

Die Methode dient zum einen der Untersuchung des Einflusses von Testsubstanzen selbst auf die Dünndarm-Motilität, zum anderen der Untersuchung, inwieweit spezifisch induzierte Effekte auf die Dünndarm-Motilität verhindert bzw. antagonisiert werden können, z.B. die Verzögerung der intestinalen Passage durch den cannabinoiden CB1-Agonist CP55.940.

Es werden weibliche NMRI-Mäuse mit einem gewicht von 25-35 g verwendet. Die Mäuse werden mindestens eine Woche an die Haltungsbedingungen gewöhnt.

Die Mäuse werden für 24 Stunden nüchtern gesetzt, haben aber beständig Zugang zu Wasser. Die Prüfsubstanzen werden oral, intravenös, subkutan aber nicht intraperitoneal verabreicht. Soll ein spezifischer Effekt antagonisiert werden, so wird die Prüfsubstanz 30-120 min. vor dem spezifischen Effektor verabreicht. 30 min. nach dieser Applikation wird eine definierte Menge eines gefärbten, nicht-kalorischen Füllstoffes per Gavage in den Magen eingebracht. Nach weiteren 30 min, der gefärbte Füllstoff hat zu diesem Zeitpunkt in etwa 80% des Dünndarms gefüllt, werden die Tiere getötet und der Dünndarm präpariert. Die intestinale Motilität wird als Passage des gefärbten Füllstoffes im Vergleich zur Gesamtlänge des Dünndarms in Prozent angegeben. Ein Behandlungseffekt wird als Unterschied dieser Passage zur Vehikel-Kontrolle ebenfalls in Prozent angegeben.

## Patentansprüche

**1.** Verbindungen der Formel I,

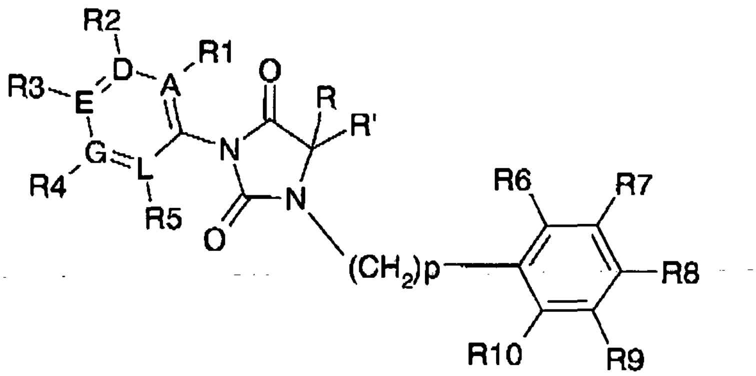

I

worin bedeuten

R, R' Methyl;
oder R und R' bilden gemeinsam einen Cyclohexylring;
n 0, 1, 2;
P 1;
A, D, E, G, L unabhängig voneinander C oder N, wobei bei der Bedeutung N der entsprechende Substituent R1, R2, R3, R4, R5 entfällt;
R1, R2, R5 unabhängig voneinander H, F, Cl, Br, J, CN, $CF_3$, $(C_1-C_4)$-Alkyl, O-$(C_1-C_4)$-Alkyl, Phenyl, -O-Phenyl, $SF_5$, wobei die Alkylreste mit Fluoratomen substituiert sein können und wobei die Phenylreste mit F, Cl, Br, J substituiert sein können;
R3 F, CN;
R4 $CF_3$, $(C_1-C_4)$-Alkyl, O-$(C_1-C_4)$-Alkyl;
R6, R7, R8, R9, R10 unabhängig voneinander H, F, Cl, Br, J, $(C_1-C_4)$-Alkyl, O-$(C_1-C_4)$-Alkyl, wobei die Alkylreste mit Fluoratomen substituiert sein können, NR17-Aryl, wobei der Arylrest substituiert sein kann mit F, Cl, Br, J, $(CH_2)_n$-CO-$NH_2$, $NH_2$, -$SO_2$- $NH_2$, COOH, $(CH_2)_n$-P(O)(OH)[O-$(C_1-C_4)$-Alkyll, $(CH_2)_n$-P(O)$(OH)_2$;

wobei immer mindestens einer der Reste R6, R7, R8, R9 und R10 die Bedeutung NR17-Aryl besitzt;

R17 H, $(C_1-C_4)$-Alkyl;

sowie deren physiologisch verträgliche Salze.

**2.** Verbindungen gemäß Anspruch 1, zur Anwendung als Arzneimittel.

**3.** Arzneimittel enthaltend eine oder mehrere der Verbindungen gemäß Anspruch 1

**4.** Arzneimittel enthaltend eine oder mehrere der Verbindungen gemäß Anspruch 1 und mindestens einen weiteren Wirkstoff.

**5.** Arzneimittel, gemäß Anspruch 4, **dadurch gekennzeichnet, dass** es als weiteren Wirkstoff eine oder mehrere Antidiabetika, hypoglykämischen Wirkstoffe, HMGCoA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, MTP-Inhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, $\alpha$-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1B, Modulatoren des natrium-abhängigen Glukosetransporters 1 oder 2, Modulatoren des GPR40, Inhibitoren der hormon-sensitiven Lipase, Hemmstoffe der Acetyl-CoA Carboxylase,

Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocortiroidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, , CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

**6.** Verwendung der Verbindungen gemäß Anspruch 1 zur Herstellung eines Medikamentes zur Behandlung des metabolischen Syndroms.

**7.** Verwendung der Verbindungen gemäß Anspruch 1 zur Herstellung eines Medikamentes zur Behandlung des Diabetes.

**8.** Verwendung der Verbindungen gemäß Anspruch 1 zur Herstellung eines Medikamentes zur Behandlung von Adipositas.

**9.** Verwendung der Verbindungen gemäß Anspruch 1 zur Herstellung eines Medikamentes zur Gewichtsreduktion.

**10.** Verwendung der Verbindungen gemäß Anspruch 1 zur Herstellung eines Medikamentes zur Behandlung von Nikotinabhängigkeit.

**11.** Verwendung der Verbindungen gemäß Anspruch 1 zur Herstellung eines Medikamentes zur Behandlung von Alkoholabhängigkeit.

**12.** Verwendung der Verbindungen gemäß Anspruch 1 zur Herstellung eines Medikamentes zur Behandlung von ZNS-Erkrankungen.

**13.** Verwendung der Verbindungen gemäß Anspruch 1 zur Herstellung eines Medikamentes zur Behandlung von Schizophrenie.

**14.** Verwendung der Verbindungen gemäß Anspruch 1 zur Herstellung eines Medikamentes zur Behandlung von Alzheimer.

**15.** Verwendung der Verbindungen gemäß Anspruch 1 zur Herstellung eines Medikamentes zur Behandlung des Syndroms der polycystischen Ovarien (PCOS, polycystic ovary syndrome).

**16.** Verfahren zur Herstellung eines Arzneimittels enthaltend eine oder mehrere der Verbindungen gemäß Anspruch 1, **dadurch gekennzeichnet, dass** der Wirkstoff mit einem pharmazeutisch geeigneten Träger vermischt wird und diese Mischung in eine für die Verabreichung geeignete Form gebracht wird.

## Claims

**1.** A compound of the formula I

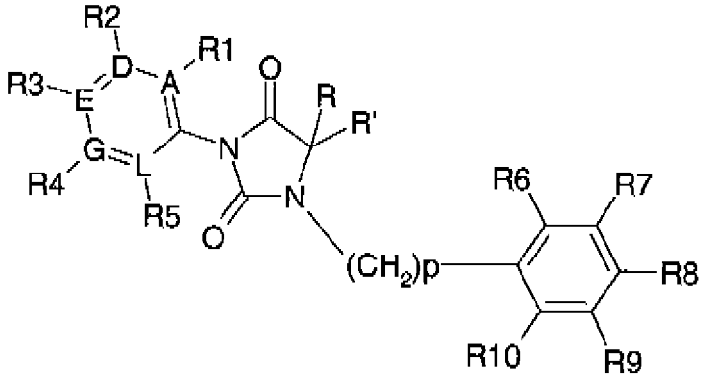

I

in which

R, R' are each methyl;
or R and R' together form a cyclohexyl ring;
n is 0, 1, 2;
P is 1;
A, D, E, G, L are each independently C or N, where there is no corresponding R1, R2, R3, R4, R5 substituent when they are defined as N;
R1, R2, R5 are each independently H, F, Cl, Br, I, CN, $CF_3$, $(C_1\text{-}C_4)$-alkyl, O-$(C_1\text{-}C_4)$ -alkyl, phenyl, -O-phenyl, $SF_5$, where the alkyl radicals may be substituted by fluorine atoms and where the phenyl radicals may be substituted by F, Cl, Br, I;
R3 is F, CN;
R4 is $CF_3$, $(C_1\text{-}C_4)$ -alkyl, O- $(C_1\text{-}C_4)$-alkyl;
R6, R7, R8, R9, R10 are each independently H, F, Cl, Br, I, $(C_1\text{-}C_4)$-alkyl, O-$(C_1\text{-}C_4)$-alkyl, where the alkyl radicals may be substituted by fluorine atoms, NR17-aryl, where the aryl radical may be substituted by F, Cl, Br, I, $(CH_2)_n$-CO-$NH_2$, $NH_2$, -$SO_2$- $NH_2$, COOH, $(CH_2)$n-P(O) (OH) [O-$(C_1\text{-}C_4)$-alkyl], $(CH_2)_n$-P(O) $(OH)_2$;

where at least one of the R6, R7, R8, R9 and R10 radicals is always defined as NR17-aryl;

R17 is H, $(C_1\text{-}C_4)$ -alkyl;

and the physiologically compatible salts thereof.

2. A compound as claimed in claim 1 for use as a medicament.

3. A medicament comprising one or more of the compounds as claimed in claim 1.

4. A medicament comprising one or more of the compounds as claimed in claim 1 and at least one further active ingredient.

5. The medicament as claimed in claim 4, which comprises, as a further active ingredient, one or more antidiabetics, active hypoglycemic ingredients, HMG-CoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, MTP inhibitors, CETP inhibitors, polymeric bile acid adsorbers, LDL receptor inducers, ACAT inhibitors, antioxidants, lipoprotein lipase inhibitors, ATP citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, $\alpha$-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose 1,6-biphosphatase, modulators of glucose transporter 4, inhibitors of glutamine: fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, modulators of GPR40, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, decoupling protein 2 or 3 modulators, leptin agonists, DA agonists (bromocriptin, doprexin), lipase/amylase inhibitors, PPAR modulators, RXR modulators or TR-β-agonists or amphetamines.

6. The use of the compounds as claimed in claim 1 for producing a medicament for the treatment of metabolic syndrome.

7. The use of the compounds as claimed in claim 1 for producing a medicament for the treatment of diabetes.

8. The use of the compounds as claimed in claim 1 for producing a medicament for the treatment of obesity.

9. The use of the compounds as claimed in claim 1 for producing a medicament for weight reduction.

10. The use of the compounds as claimed in claim 1 for producing a medicament for the treatment of nicotine dependence.

11. The use of the compounds as claimed in claim 1 for producing a medicament for the treatment of alcohol dependence.

12. The use of the compounds as claimed in claim 1 for producing a medicament for the treatment of CNS disorders.

13. The use of the compounds as claimed in claim 1 for producing a medicament for the treatment of schizophrenia.

14. The use of the compounds as claimed in claim 1 for producing a medicament for the treatment of Alzheimer's.

15. The use of the compounds as claimed in claim 1 for producing a medicament for the treatment of polycystic ovary syndrome (PCOS).

16. A process for producing a medicament comprising one or more of the compounds as claimed in claim 1, which comprises mixing the active ingredient with a pharmaceutically suitable carrier and bringing this mixture into a form suitable for administration.

## Revendications

1. Composés de formule I,

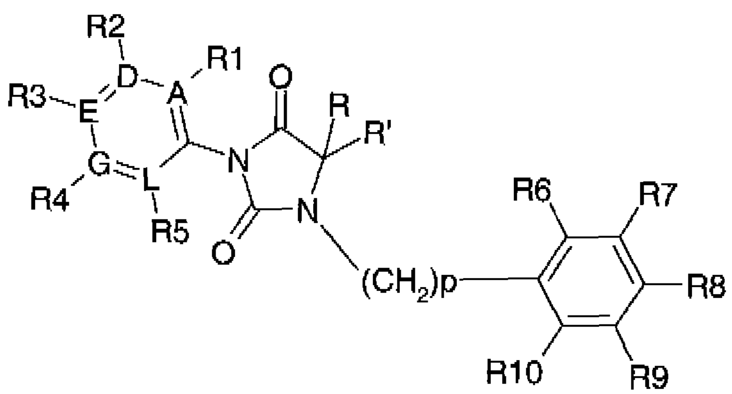

I

où

R, R' représentent un groupement méthyle ;

ou R et R' forment ensemble un cycle cyclohexyle ;
n est égal à 0, 1 ou 2 ;
p est égal à 1 ;
A, D, E, G, L représentent de façon indépendante les uns des autres C ou N, sachant qu'il n'existe pas de substituant R1, R2, R3, R4, R5 correspondant lorsqu'ils représentent N ;
R1, R2, R5 représentent de façon indépendante les uns des autres H, F, Cl, Br, I ou un groupement CN, $CF_3$, alkyle en $C_1$-$C_4$, O-alkyle en $C_1$-$C_4$, phényle, -O-phényle, $SF_5$, où les radicaux alkyle peuvent être substitués par des atomes de fluor et où les radicaux phényle peuvent être substitués par F, Cl, Br, I ;
R3 représente F, CN ;
R4 représente un groupement $CF_3$, alkyle en $C_1$-$C_4$, O-allyle en $C_1$-$C_4$ ;
R6, R7, R8, R9, R10 représentent de façon indépendante les uns des autres H, F, Cl, Br, I ou un groupement alkyle en $C_1$-$C_4$, O-alkyle en $C_1$-$C_4$, où les radicaux alkyle peuvent être substitués par des atomes de fluor, NR17-aryle, où le radical aryle peut être substitué par F, Cl, Br, I, $(CH_2)_n$-CO-$NH_2$, $NH_2$, -$SO_2$-$NH_2$, COOH, $(CH_2)_n$-P(O) (OH) [O-alkyle en $C_1$-$C_4$], $(CH_2)_n$-P(O)$(OH)_2$ ;

où au moins l'un des radicaux R6, R7, R8, R9 et R10 a toujours pour signification NR17-aryle ;

R17 représente H ou un groupement alkyle en $C_1$-$C_4$ ;

et les sels de qualité physiologique de ces composés.

**2.** Composés selon la revendication 1 pour emploi en tant que médicaments.

**3.** Médicament comprenant un ou plusieurs des composés selon la revendication 1.

**4.** Médicament comprenant un ou plusieurs des composés selon la revendication 1 et au moins un principe actif Supplémentaire.

**5.** Médicament selon la revendication 4, **caractérisé en ce qu'**il contient en tant que principe actif supplémentaire un(e) ou plusieur(e)s antidiabétiques, principes hypoglycémiques actifs, inhibiteurs de HMG-COA réductase, inhibiteurs de résorption de cholestérol, agonistes de PPAR gamma, agonistes de PPAR alpha, agonistes de PPAR alpha/gamma, agonistes de PPAR delta, fibrates, inhibiteurs de MTP, inhibiteurs de résorption d'acide biliaire, inhibiteurs de MTP, inhibiteurs de CETP, adsorbants polymères d'acide biliaire, inducteurs de récepteurs aux LDL, inhibiteurs de ACAT, antioxydants, inhibiteurs de lipoprotéine lipase, inhibiteurs de ATP citrate lyase, inhibiteurs de squalène synthétase, antagonistes de lipoprotéine(a), agonistes de récepteurs HM74A, inhibiteurs de lipase, insulines, sulfonylurées, biguanides, meglitinides, thiazolidinediones, inhibiteurs de $\alpha$-glucosidase, principes actifs agissant sur le canal potassique ATP-dépendant des cellules bêta, inhibiteurs de glycogène phosphorylase, antagonistes de récepteurs à la glucagone, agents activant la glucokinase, inhibiteurs de la gluconéogenèse, inhibiteurs de la fructose-1,6-biphosphatase, agents modulant le transporteur de glucose 4, inhibiteurs de glutamine : fructose-6-phosphate amidotransférase, inhibiteurs de dipeptidylpeptidase IV, inhibiteurs de 11-bêta-hydroxystéroïde déshydrogénase 1, inhibiteurs de protéine tyrosine phosphatase 1B, agents modulant le transporteur de glucose sodium-dépendant 1 ou 2, agents modulant GPR40, inhibiteurs de lipase sensible aux hormones, inhibiteurs d'acétyl-CoA carboxylase, inhibiteurs de phosphoénolpyruvate carboxykinase, inhibiteurs de glycogène synthase kinase-3 bêta, inhibiteurs de protéine kinase C bêta, antagonistes de récepteurs à l'endothéline-A, inhibiteurs de I kappaB kinase, agents modulant le récepteur aux glucocorticoïdes, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes de $\beta$3, antagonistes de récepteur CB1, agonistes de MSH (mélanostimuline), agonistes de CCK, inhibiteurs de recapture de sérotonine, composés mixtes sérotoninergiques et noradrénergiques, agonistes de 5HT, agonistes de bombésine, antagonistes de galanine, hormones de croissance, composés libérant l'hormone de croissance, agonistes de TRH, agents modulant la protéine de découplage 2 ou 3, agonistes de leptine, agonistes de DA (bromocriptine, doprexine), inhibiteurs de lipase/amylase, agents modulant PPAR, agents modulant RXR ou agonistes de TR-$\beta$ ou amphétamines.

**6.** Emploi des composés selon la revendication 1 pour la production d'un médicament pour le traitement du syndrome métabolique.

**7.** Emploi des composés selon la revendication 1 pour la production d'un médicament pour le traitement du diabète.

8. Emploi des composés selon la revendication 1 pour la production d'un médicament pour le traitement de l'obésité.

9. Emploi des composés selon la revendication 1 pour la production d'un médicament destiné à la perte de poids.

10. Emploi des composés selon la revendication 1 pour la production d'un médicament pour le traitement de la dépendance à la nicotine.

11. Emploi des composés selon la revendication 1 pour la production d'un médicament pour le traitement de la dépendance à l'alcool.

12. Emploi des composés selon la revendication 1 pour la production d'un médicament pour le traitement des troubles du système nerveux central.

13. Emploi des composés selon la revendication 1 pour la production d'un médicament pour le traitement de la schizophrénie.

14. Emploi des composés selon la revendication 1 pour la, production d'un médicament pour le traitement de la maladie d'Alzheimer.

15. Emploi des composés selon la revendication 1 pour la production d'un médicament pour le traitement du syndrome des ovaires polykystiques (SOPK).

16. Procédé de production d'un médicament comprenant un ou plusieurs des composés selon la revendication 1 **caractérisé en ce que** le principe actif est mélangé avec un vecteur de qualité pharmaceutique et ce mélange est converti en une forme adaptée à l'administration.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 5411981 A
- WO 2004070050 A
- WO 2005049580 A
- WO 2007083017 A
- WO 2005005477 A
- US 6221633 B
- WO 9808871 A
- WO 2005027978 A
- WO 2006037811 A
- WO 2006037810 A
- WO 0104156 A
- WO 0034331 A
- WO 2006124529 A
- WO 2006121860 A
- WO 9726265 A
- WO 9903861 A
- WO 2005042692 A
- WO 2005005453 A
- WO 2005021497 A
- WO 2005021495 A
- WO 2002066464 A
- WO 2005000353 A
- WO 2005044256 A
- WO 2005062824 A
- WO 2005061451 A
- WO 2005061452 A
- WO 2006017257 A
- WO 2005033100 A
- WO 2004097655 A
- WO 2004000805 A
- WO 2004000804 A
- WO 2004000803 A
- WO 2002050068 A
- WO 2002050060 A
- WO 2005047248 A
- WO 2006086562 A
- WO 2006102674 A
- WO 2006116499 A
- WO 2006121861 A
- WO 2006122186 A
- WO 2006122216 A
- WO 2006127893 A
- WO 2006137794 A
- WO 2006137796 A
- WO 2006137782 A
- WO 2006137793 A
- WO 2006137797 A
- WO 2006137795 A
- WO 2006137792 A
- WO 2006138163 A
- WO 2001040207 A
- WO 2002096894 A
- WO 2005097076 A
- US 0011833 W
- US 0011490 W
- DE 10142734
- WO 2006059744 A
- WO 2006084176 A
- WO 2006029699 A
- WO 2007039172 A
- WO 2007039178 A
- WO 2005085226 A
- WO 2005121091 A
- WO 2006010423 A
- WO 2006002342 A
- WO 2006010422 A
- WO 2006012093 A
- WO 2006073973 A
- WO 2006072362 A
- WO 2006097169 A
- WO 2007041494 A
- US 6245744 B
- US 6221897 B
- WO 0061568 A
- DE 102005033099
- DE 102005033100
- WO 200700965556 A
- US 6342512 B
- WO 2005097738 A
- WO 2006072393 A
- WO 2005077907 A
- JP 2007022943 B
- WO 2006045565 A
- WO 2006045564 A
- WO 2006069242 A
- WO 2006124490 A
- WO 2006113150 A
- WO 2007017261 A
- WO 2007017262 A
- WO 2007017265 A
- WO 2007015744 A
- WO 2007027532 A
- WO 2006067531 A
- WO 2006067532 A
- WO 2003097064 A
- WO 2007026761 A
- WO 9741097 A
- FR 258900

- WO 2003084922 A
- WO 2004007455 A
- WO 200507322931 A
- WO 2005067932 A
- WO 2004100875 A
- WO 2005065680 A
- WO 2004063179 A
- WO 2004072031 A
- WO 2004072066 A
- WO 2005080360 A
- WO 2005044801 A
- WO 2006016194 A
- WO 2006058923 A
- WO 2006112549 A
- WO 2006125972 A
- WO 2007017549 A
- WO 2007017649 A
- WO 2007007910 A
- WO 200700704042 A
- WO 2007006760 A
- WO 2007006814 A
- WO 2007007886 A
- WO 2007028135 A
- WO 2007031739 A
- WO 2007041365 A
- WO 2007041366 A
- WO 2007037534 A
- WO 2007043638 A
- WO 2007053345 A
- WO 2007051846 A
- WO 2007051845 A
- WO 2007053765 A
- WO 2007051847 A
- FR 225654
- WO 2006023515 A
- WO 2006104030 A
- WO 2007014619 A
- WO 2004101528 A
- WO 2003074500 A
- WO 2003106456 A
- WO 2004037169 A
- WO 200450658 A
- WO 2005058901 A
- WO 2005012312 A
- WO 2005012308 A
- WO 2006039325 A
- WO 2006058064 A
- EP 2005007821 W
- EP 2005008005 W
- EP 2005008002 W
- EP 2005008004 W
- EP 2005008283 W
- DE 102005012874
- DE 102005012873
- JP 2006160733 B
- WO 2006071752 A
- WO 2006065826 A
- WO 2006078676 A
- WO 2006073167 A
- WO 2006068163 A
- WO 2006090915 A
- WO 2006104356 A
- WO 2006127530 A
- WO 2006111261 A
- WO 2007015767 A
- WO 2007024993 A
- WO 2007029086 A
- WO 20019009094 A
- WO 200343999 A
- WO 2004112782 A
- WO 200344000 A
- WO 200344009 A
- WO 2004112779 A
- WO 2004113310 A
- WO 2004103980 A
- WO 2004112784 A
- WO 2003065983 A
- WO 2003104207 A
- WO 2003104208 A
- WO 2004106294 A
- WO 2004011410 A
- WO 2004033427 A
- WO 2004041264 A
- WO 2004037251 A
- WO 2004056744 A
- WO 2004058730 A
- WO 2004065351 A
- WO 2004089367 A
- WO 2004089380 A
- WO 200408947071 A
- WO 2004089896 A
- WO 2005016877 A
- WO 2005097759 A
- WO 2006010546 A
- WO 2006012227 A
- WO 2006012173 A
- WO 2006017542 A
- WO 2006034804 A
- WO 2006040329 A
- WO 2006051662 A
- WO 2006048750 A
- WO 2006049952 A
- WO 2006048331 A
- WO 2006050908 A
- WO 2006024627 A
- WO 2006066109 A
- WO 2006074244 A
- WO 2006078006 A
- WO 2006106423 A
- WO 2006132436 A
- WO 2006134481 A
- WO 2006134467 A
- WO 2006135795 A
- WO 2006136502 A
- WO 2006138695 A
- WO 2006133926 A

- WO 2007003521 A
- WO 2007007688 A
- US 2007066584 A
- WO 2007047625 A
- WO 2007051811 A
- WO 2007051810 A
- WO 20011983031 A
- WO 200117516 A
- WO 2004506446 A
- WO 2005012295 A
- WO 2005116003 A
- EP 2005005311 W
- EP 2005005321 W
- EP 2005007151 W
- DE 102004060542
- WO 2007009911 A
- WO 2007028145 A
- WO 2004007517 A
- WO 200452903 A
- WO 200452902 A
- EP 2005005959 W
- WO 2005085237 A
- JP 2004359630 B
- WO 2005121161 A
- WO 2006018150 A
- WO 2006035796 A
- WO 2006062224 A
- WO 2006058597 A
- WO 2006073197 A
- WO 2006080577 A
- WO 2006087997 A
- WO 2006108842 A
- WO 2007000445 A
- WO 2007014895 A
- WO 2007013689 A
- WO 2007033002 A
- WO 2004041274 A
- WO 2005061489 A
- WO 2004065380 A
- WO 200700396062 A
- WO 2007003964 A
- WO 2005073199 A
- WO 2006074957 A
- WO 2006087309 A
- WO 2006111321 A
- WO 2007042178 A
- WO 199946262 A
- WO 200372197 A
- WO 2003072197 A
- WO 2005044814 A
- WO 2005108370 A
- JP 2006131559 B
- WO 2007011809 A
- WO 2007011811 A
- WO 2007013691 A
- WO 2004074288 A
- US 2005222220 A
- WO 2005085230 A
- EP 2005005346 W
- WO 2003078403 A
- WO 2004022544 A
- WO 2003106410 A
- WO 2005058908 A
- US 2005038023 A
- WO 2005009997 A
- US 2005026984 A
- WO 2005000836 A
- WO 2004106343 A
- EP 1460075 A
- WO 2004014910 A
- WO 2003076442 A
- WO 2005087727 A
- WO 2004046117 A
- WO 2006072354 A
- WO 2007035355 A
- WO 2001000610 A
- WO 2001030774 A
- WO 2004022553 A
- WO 2005097129 A
- WO 2005090336 A
- WO 2006071609 A
- WO 2006135826 A
- WO 2006001318 A
- WO 2007038942 A
- WO 2007038943 A
- WO 2005080424 A
- WO 2006095166 A
- WO 2006096847 A
- EP 0656354 A
- WO 0015609 A
- WO 200164632 A
- WO 200164633 A
- WO 200164634 A
- WO 02076949 A
- WO 2005080345 A
- WO 2005080328 A
- WO 2005080343 A
- WO 2005075450 A
- WO 2005080357 A
- WO 200170700 A
- WO 200302664748 A
- WO 200302776 A
- WO 2003040107 A
- WO 2003007887 A
- WO 2003027069 A
- US 6509367 B
- WO 200132663 A
- WO 2003086288 A
- WO 2003087037 A
- WO 2004048317 A
- WO 2004058145 A
- WO 2003084930 A
- WO 2003084943 A
- WO 2004058744 A
- WO 2004013120 A
- WO 2004029204 A

- WO 2004035566 A
- WO 2004058249 A
- WO 2004058255 A
- WO 2004058727 A
- WO 2004069838 A
- US 20040214837 A
- US 20040214855 A
- US 20040214856 A
- WO 2004096209 A
- WO 2004096763 A
- WO 2004096794 A
- WO 2005000809 A
- WO 2004099157 A
- US 20040266845 A
- WO 2004110453 A
- WO 2004108728 A
- WO 2004000817 A
- WO 2005000820 A
- US 20050009870 A
- WO 200500974 A
- WO 200411103334 A
- WO 20041103839 A
- WO 2005016286 A
- WO 2005007111 A
- WO 2005007628 A
- US 20050054679 A
- WO 2005027837 A
- WO 2005028456 A
- WO 200506376162 A
- WO 2005061509 A
- WO 2005077897 A
- WO 2006047516 A
- WO 2006060461 A
- WO 2006067428 A
- WO 2006067443 A
- WO 2006087480 A
- WO 2006087476 A
- WO 2006100208 A
- WO 2006106054 A
- WO 2006111849 A
- WO 2006113704 A
- WO 2007009705 A
- WO 2007017124 A
- WO 2007017126 A
- WO 2007018459 A
- WO 2007016460 A
- WO 2007020502 A
- WO 2007026215 A
- WO 2007028849 A
- WO 2007031720 A
- WO 2007031721 A
- WO 2007036945 A
- WO 2007038045 A
- WO 2007039740 A
- US 20070015810 A
- WO 2007046548 A
- WO 2007047737 A
- WO 2007001939 A
- WO 2007044215 A
- WO 0191752 A
- WO 2005060985 A
- WO 2005009950 A
- WO 2004087159 A
- WO 2004078717 A
- WO 2004078716 A
- WO 2004024720 A
- US 20050124652 A
- WO 2005051391 A
- WO 2004112793 A
- WO US20050222014 A
- US 20050176728 A
- US 20050164914 A
- US 20050124636 A
- US 20050130988 A
- US 20040167201 A
- WO 2004005324 A
- WO 2004037797 A
- WO 2005042516 A
- WO 2005040109 A
- WO 2005030797 A
- US 20040224901 A
- WO 200501921 A
- WO 200509184 A
- WO 2005000339 A
- EP 1460069 A
- WO 2005047253 A
- WO 2005047251 A
- WO 2005118573 A
- EP 1538159 A
- WO 2004072076 A
- WO 20040720777 A
- WO 200602165557 A
- WO 2007009894 A
- WO 2007015162 A
- WO 2007041061 A
- WO 2007041052 A
- WO 200196302 A
- WO 200185693 A
- WO 2004085403 A
- WO 2005075458 A
- WO 2006067224 A
- WO 0063208 A
- WO 200064884 A
- WO 2005082893 A
- WO 2006107661 A
- WO 2007003804 A
- WO 2007016496 A
- WO 2007020213 A
- WO 0066585 A
- WO 0183451 A
- JP 2006111553 B
- WO 2002038543 A
- WO 2007048840843 A
- WO 2005085200 A
- WO 2005019240 A
- WO 2004011438 A

- WO 2004012648 A
- WO 2003015769 A
- WO 2004072025 A
- WO 2005070898 A
- WO 2005070925 A
- WO 2004039780 A
- WO 2004092181 A
- WO 2003033476 A
- WO 2002006245 A
- WO 2002089729 A
- WO 2002002744 A
- WO 2003004027 A
- FR 2868780
- WO 2006010446 A
- WO 2006038680 A
- WO 2006044293 A
- WO 2006044174 A
- JP 2006176443 B
- WO 2006018280 A
- WO 2006018279 A
- WO 2006118320 A
- WO 2006130075 A
- WO 2007018248 A
- WO 2007012661 A
- WO 2007029847 A
- WO 2007024004 A
- WO 2007039462 A
- WO 2007042660 A
- WO 2007042668 A
- WO 2007042669 A
- US 2007093508 A
- US 2007093509 A
- WO 2007048802 A
- JP 2007091649 B
- WO 9915525 A
- WO 0244150 A
- WO 2005116034 A
- WO 0071549 A
- WO 0109111 A
- WO 200077010 A
- WO 20007700102 A
- WO 2005019180 A
- WO 2003064423 A
- WO 200242304 A
- WO 2005035533 A
- WO 2005082859 A
- WO 2006077025 A
- WO 2006103511 A
- WO 2005058858 A
- WO 2007054257 A
- WO 0185695 A
- WO 2005030734 A
- EP 0462884 A
- WO 0040569 A
- US 20040224997 A
- WO 2004094618 A
- WO 200058491 A
- WO 2005044250 A
- WO 2005072740 A
- JP 2005206492 B
- WO 2005013907 A
- WO 2006004200 A
- WO 2006019020 A
- WO 2006064189 A
- WO 2006082952 A
- WO 2006120125 A
- WO 2006113919 A
- WO 2006134317 A
- WO 2007016538 A
- WO 2004005277 A
- WO 2007009236 A
- WO 2007044085 A
- WO 2007046867 A
- WO 2007046868 A
- WO 20070501124 A
- WO 20058279 A
- WO 200172692 A
- WO 200194293 A
- WO 2003084915 A
- WO 2004018421 A
- WO 2005092316 A
- WO 2007003419 A
- WO 2007009913 A
- WO 2007039125 A
- US 6992067 B
- US 7205290 B

**In der Beschreibung aufgeführte Nicht-Patentliteratur**


- **PETER M. COLLINS ; ROBERT J. FERRIER.** Monosaccharides. John Wiley & Sons Ltd, 1995, 16-18
- **PETER M. COLLINS ; ROBERT J. FERRIER.** Monosaccharides. John Wiley & Sons Ltd, 1995, 19-21
- **PETER M. COLLINS ; ROBERT J. FERRIER.** Monosaccharides. John Wiley & Sons Ltd, 1995, 126-129
- **PETER M. COLLINS ; ROBERT J. FERRIER.** Monosaccharides. John Wiley & Sons Ltd, 1995, 138-139
- **PETER M. COLLINS ; ROBERT J. FERRIER.** Monosaccharides. John Wiley & Sons Ltd, 1995, 313-314
- *Behavioural Pharmacology,* 2005, vol. 16, 275-296
- **KEN MACKIE.** *Annu. Rev. Pharmacol. Toxicol.,* 2006, vol. 46, 101-122

- **S. C. BLACK.** *Curr. Opin. Investig. Drugs,* 2004, vol. 5, 389-394
- **V. DI MARZIO et al.** *Nat. Rev. Drug Discov.,* 2004, vol. 3, 771-784
- **B. LE FOLL et al.** *J. Pharmacol. Exp. Ther.,* 2005, vol. 312, 875-883
- **L. WALTER et al.** *Br. J. Pharmacol.,* 2004, vol. 141, 775-785
- *Pharmaceutical Research,* 1986, vol. 2 (6), 318
- Roten Liste. 2005
- USP Dictionary of USAN and International Drug Names. 2001
- **D. CHEN et al.** *Proc. Natl. Acad. Sci. USA,* 2007, vol. 104, 943
- **R. D. CARR et al.** *Diabetes,* 2003, vol. 52, 2513-2518
- **J. B. HANSEN et al.** *Current Medicinal Chemistry,* 2004, vol. 11, 1595-1615
- **T. M. TAGMOSE et al.** *J. Med. Chem.,* 2004, vol. 47, 3202-3211
- **M. J. COGHLAN et al.** *J. Med. Chem.,* 2001, vol. 44, 1627-1653
- **J.P.BERGER et al.** *TRENDS in Pharmacological Sciences,* 2005, vol. 28 (5), 244-251
- **A. L. HANDLON.** *Expert Opin. Ther. Patents,* 2005, vol. 15 (11), 1531-1540
- **ASAKAWA, A. et al.** Cocaine-amphetamine-regulated transcript influences energy metabolism, anxiety and gastric emptying in mice. *Hormone and Metabolie Research,* 2001, vol. 33 (9), 554-558
- **LEE, DANIEL W. ; LEINUNG, MATTHEW C. ; ROZHAVSKAYA-ARENA, MARINA ; GRASSO, PATRICIA.** Leptin agonists as a potential approach to the treatment of obesity. *Drugs of the Future,* 2001, vol. 26 (9), 873-881
- **SALVADOR, JAVIER ; GOMEZ-AMBROSI, JAVIER ; FRUHBECK, GEMA.** Perspectives in the therapeutic use of leptin. *Expert Opinion on Pharmacotherapy,* 2001, vol. 2 (10), 1615-1622
- **ZUNFT H J et al.** Carob pulp preparation for treatment of hypercholesterolemia. *ADVANCES IN THERAPY,* September 2001, vol. 18 (5), 230-6
- Roten Liste. 2006
- **S.L.BUCHWALD et al.** *Acc. Chem. Res.,* 1998, vol. 31, 805
- **J.F.HARTWIG et al.** *J. Org. Chem.,* 1999, vol. 64, 5575-5580
- **J.P.WOLFE et al.** *J. Org. Chem.,* 2000, vol. 65, 144-1157
- **M.D.CHARLES et al.** *Org. Lett.,* 2005, vol. 7, 3965-68
- **J.-B.LAN et al.** *SYNLETT,* 2004, 1095-1097
- **D.M.T.CHAN et al.** *Tetrahedron Lett.,* 1998, vol. 39, 2933-2936
- **J. ZHOU ; G. C. FU.** *J. Am. Chem. Soc.,* 2004, vol. 126, 1340-1341
- **F. GONZÄLES-BOBES ; G. C. FU.** *J. Am. Chem. Soc.,* 2006, vol. 128, 5360-5361
- **D. J. WALLACE ; C.-Y. CHEN.** *Tetrahedron Letters,* 2002, vol. 43, 6987-6990
- **T. E. BARDER et al.** *J. Am. Chem. Soc.,* 2005, vol. 127, 4685-4696
- **D. W. OLD et al.** *J. Am. Chem. Soc.,* 1998, vol. 120, 9722
- **T. E. BARDER ; ST. L. BUCHWALD.** *Org. Lett.,* 2004, vol. 6, 2649-2652
- **R. FRLAN ; D. KIKELJ.** *Synthesis,* 2006, vol. 14, 2271-2285
- **A. V. VOROGUSHIN et al.** *J. Am. Chem. Soc.,* 2005, vol. 127, 8146-8149
- **F. Y. KWONG ; ST. L. BUCHWALD.** *Org. Lett.,* 2002, vol. 4, 3517-3520
- **S. M. NOBRE ; A. L. MONTEIRO.** *Tetrahedron Letters,* 2004, vol. 45, 8225-8228
- **S. LANGLE et al.** *Tetrahedron Letters,* 2003, vol. 44, 9255-9258
- **A. R. KATRITZKY et al.** *J. Org. Chem.,* 2005, vol. 70, 9191-9197
- **CHENG, Y.-C. ; PRUSOFF, W.H.** *Biochem. Pharmacol,* 1973, vol. 22, 3099-3108